(19) Europäisches Patentamt
European Patent Office
Office européen des brevets

(11) EP 4 763 185 A1

# (12) EUROPEAN PATENT APPLICATION
published in accordance with Art. 153(4) EPC

(43) Date of publication:
**24.06.2026 Bulletin 2026/26**

(21) Application number: **24854192.2**

(22) Date of filing: **09.08.2024**

(51) International Patent Classification (IPC):
***A61K 8/25*** (2006.01) ***A61K 8/89*** (2006.01)
***A61Q 1/00*** (2006.01) ***A61Q 1/02*** (2006.01)
***A61Q 1/04*** (2006.01) ***A61Q 1/06*** (2006.01)
***A61Q 1/10*** (2006.01) ***A61Q 3/02*** (2006.01)
***A61Q 15/00*** (2006.01) ***A61Q 17/04*** (2006.01)
***A61Q 19/00*** (2006.01) ***C08F 299/04*** (2006.01)
***C08G 63/91*** (2006.01) ***C08G 77/442*** (2006.01)
***C08L 101/16*** (2006.01)

(52) Cooperative Patent Classification (CPC):
**A61K 8/25; A61K 8/89; A61Q 1/00; A61Q 1/02; A61Q 1/04; A61Q 1/06; A61Q 1/10; A61Q 3/02; A61Q 15/00; A61Q 17/04; A61Q 19/00; C08F 299/04; C08G 63/91; C08G 77/442; C08L 101/16**

(86) International application number:
**PCT/JP2024/028740**

(87) International publication number:
**WO 2025/037603 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.08.2023 JP 2023131876**

(71) Applicant: **SHIN-ETSU CHEMICAL CO., LTD.**
**Tokyo 100-0005 (JP)**

(72) Inventors:
- **OKI Takahito**
  **Annaka-shi, Gunma 379-0224 (JP)**
- **TANAKA Mituru**
  **Tokyo 100-0005 (JP)**

(74) Representative: **Angerhausen, Christoph**
**Boehmert & Boehmert**
**Anwaltspartnerschaft mbB**
**Pettenkoferstrasse 22**
**80336 München (DE)**

## (54) COSMETIC

(57) Provided is a cosmetic containing particles that can degrade in the environment after use without remaining as particles (solids) and that, when added to the cosmetic, can provide good softness, high spreadability, and high light diffusibility. The cosmetic contains at least one type of particles (i) and (ii) which are: (i) spherical elastomer particles that have a volume average particle diameter of 1.0 to 100 μm and are crosslinked particles of a copolymer having a polyester structure and a polyether structure; and (ii) elastomer composite particles including the spherical elastomer particles (i) and polyorganosilsesquioxane or silica on the surfaces of the spherical elastomer particles (i).

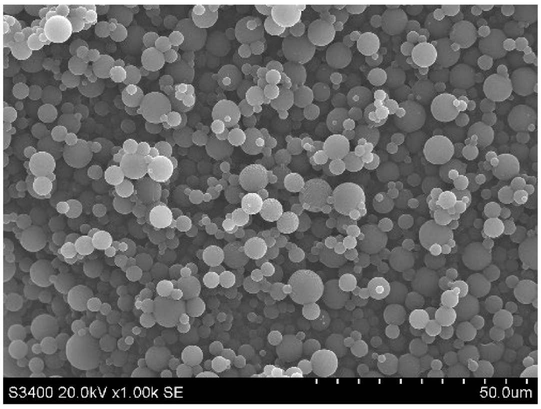


FIG.1



Processed by Luminess, 75001 PARIS (FR)

## Description

### Technical Field

The present invention relates to a cosmetic containing spherical elastomer particles including, as a constitutional unit, a copolymer having a polyester structure and a polyether structure and/or elastomer composite particles including the spherical elastomer particles and polyorganosilsesquioxane or silica on the surfaces of the spherical elastomer particles.

### Background Art

Makeup cosmetics such as foundations are used to conceal skin texture troubles such as wrinkles, pores, and skin texture roughness and color tone troubles such as skin spots and freckles to thereby make the skin appear smooth and beautiful. In recent years, the importance of a non-artificial natural finish (bare skin feel) has been increased. The ability of a cosmetic to provide a natural finish is evaluated based on the absence of unnatural shine (glow), the ability to form a highly uniform cosmetic film, and its degree of transparency.

Many new materials and technologies have been proposed to provide a natural finish while the effects of the makeup cosmetics are maintained. In particular, in one known method for concealing skin texture troubles, various diffuse reflective powders are added to cosmetics.

Patent document 1 proposes a wrinkle-concealing multilayer cosmetic including a combination of a makeup primer containing a tacky material and used for a first layer and a makeup finishing component containing a powder capable of diffusing and reflecting light and used for a second layer. In this wrinkle-concealing multilayer cosmetic, a light diffusion-reflection type composite powder prepared, for example, by coating talc particles with an acrylic-based polymer is used.

However, one drawback of this cosmetic is that, when the cosmetic film stays on the skin for a long time, sebum is mixed with the tacky material, which causes the mixing ratio of the powder to the tacky material to deviate from their appropriate mixing range, so that the effects of the cosmetic deteriorate.

Patent document 2 discloses a fine flaky powder prepared by coating the surface of a flaky material such as natural mica with fine spherical silica particles. The fine spherical particles used for the fine flaky powder have a strong effect of diffuse-scattering light at their surface. Therefore, it is stated that the following technical effects are obtained when the fine flaky powder is used as an ingredient of a cosmetic: "the fine flaky powder can reduce the excessive gloss of mica used as the base and provide a so-called soft focus effect that minimizes the appearance of fine wrinkles etc. Moreover, with the fine flaky powder, cosmetics having improved spreadability, an improved touch, etc. during use can be obtained."

However, because of its very good spreadability, the fine flaky powder has a drawback in that, when a cosmetic containing the fine flaky powder is applied to the skin, creasing tends to occur. Therefore, it is difficult to apply the cosmetic uniformly, and a natural finish cannot be obtained. Moreover, the fine silica particles used as a coating agent decrease the area of contact between the composite powder and the skin, and therefore the adhesion to the skin deteriorates. This causes a drawback in that the composite powder easily falls off the skin due to a physical impact such as rubbing with clothing, thus shortening the duration of the effects of the cosmetic. Another drawback is that, when the content of the fine silica particles in the fine flaky powder is reduced for the purpose of improving the adhesion of the composite powder to the skin, the effects on the skin texture troubles cannot be obtained sufficiently.

Other similar techniques used for the purpose of avoiding the skin texture troubles and proving a natural finish include, for example: a cosmetic containing a pigment having a core-shell structure prepared by coating a flaky extender pigment serving as cores with color pigment-containing titanium dioxide and further coating the coated cores with a light-diffusing powder (Patent document 3); a technique of mixing, into a cosmetic, a composite powder prepared by coating the surface of clay mineral with an inorganic metal hydroxide such as aluminum hydroxide (Patent documents 4 and 5); a cosmetic containing a powder having a refractive index of from 1.6 to 1.7 such as barium sulfate, the powder having a layer formed by coating its surface with a high-refractive-index metal oxide and further having a layer formed by coating the coating layer with one or two or more selected from yellow iron oxide, black iron oxide, and red iron oxide (Patent document 6); and a cosmetic prepared by mixing therewith a silica/zinc oxide composite material obtained by complexing silica sol and zinc oxide (Patent document 7).

These composite powders exhibit the soft focus effect to some extent due to their light diffusing effect. However, these include an inorganic metal oxide or an inorganic metal hydroxide. Therefore, when attention is focused on the textures of the cosmetics, their softness, moistness, and smoothness are not fully satisfactory due to the hardness of the composite powders themselves.

For the purpose of improving the textures of a cosmetic powder, i.e., imparting textures such as silkiness and smoothness, spreadability, adhesiveness, etc., Patent document 8 proposes flaky inorganic particles with surfaces coated with polyurethane, a styrene-butadiene copolymer, a silicone-based elastomer, or a polyolefin-based elastomer. This approach is reported to provide a soft and moist sensory feel.

Particles with surfaces coated with a silicone elastomer adhering thereto and methods for producing such particles have been proposed in other documents.

For example, Patent document 9 proposes a method for pulverizing and mixing silica and a silicone elastomer having a reactive functional group. However, since the silica particles used as base particles are pulverized, particles with the initial shape and the initial particle size maintained cannot be obtained. Specifically, a drawback of this method is that particles having a specific shape such as a granular, plate-like, or rod-like shape cannot be obtained and it is difficult to control the particle size.

Patent document 10 proposes a method including mixing base particles and curable silicone that later becomes a silicone elastomer and then curing the silicone. In the particles obtained, a plurality of base particles are present in each particle. Patent document 11 proposes a method including emulsifying and dispersing a mixture of silica particles used as base particles and curable silicone that later becomes a silicone elastomer in water and then curing the silicone. Patent document 12 proposes a method including emulsifying and dispersing, in water, a mixture of a water dispersion of silica particles and curable silicone that later becomes a silicone elastomer and then curing the silicone. However, with these methods, some of the obtained particles contain a plurality of base particles, and particles containing no base particles are also formed.

In view of this background, Patent document 13 proposes composite particles prepared by adding an acidic material or an alkaline material and a compound selected from alkoxysilanes, silanol group-containing silanes, and partial condensation products thereof to an aqueous dispersion mixture containing a powder and a silicone elastomer dispersed therein and then subjecting the compound to a hydrolysis-condensation reaction to thereby cause the silicone elastomer to adhere to the surface of the powder using a silicon resin serving as a binder. A cosmetic containing these composite particles has textures such as silkiness, softness, and moistness specific to the silicone elastomer and exhibits good spreadability, softness, adherence, and mixability, so that the effect of correcting skin textures can be imparted.

Spherical silicone rubber particles are used in a wide variety of cosmetics and cosmetic materials including makeup cosmetics such as foundations and makeup bases, skincare cosmetics such as creams and milky lotions, and sunscreen cosmetics for the purpose of imparting a sensory feel such as a soft feel or smoothness to the cosmetics, for the purpose of scattering light to create a natural-looking finish, and for the purpose of making pores, wrinkles, etc. less noticeable.

For example, a cosmetic containing polymethylsilsesquioxane particles/powder has been proposed (Patent document 14), and a makeup cosmetic containing spherical silicone rubber particles/powder has been proposed (Patent document 15). Moreover, a cosmetic containing silicone composite particles/powder prepared by coating spherical silicone rubber particles with a polyorganosilsesquioxane resin has been proposed (Patent document 16). The spherical silicone rubber particles and the composite particles prepared by coating spherical silicone rubber particles with a polyorganosilsesquioxane resin can impart a sensory feel such as a soft feel or smoothness to cosmetics, as described above.

Prior Art Documents

Patent documents

Patent document 1: JP-A-H06-128122
Patent document 2: WO-A-92/03119
Patent document 3: JP-A-H08-188723
Patent document 4: JP-A-H09-20609
Patent document 5: JP-A-2002-146238
Patent document 6: JP-A-2003-40737
Patent document 7: JP-B-3702072
Patent document 8: JP-B-3963635
Patent document 9: JP-A-H08-3451
Patent document 10: JP-A-H03-294357
Patent document 11: JP-A-H02-232263
Patent document 12: JP-A-H03-281536
Patent document 13: JP-A-2011-1332
Patent document 14: JP-A-S63-297313
Patent document 15: JP-A-H08-12524
Patent document 16: JP-A-H09-20631

## Summary of Invention

### Technical Problem

As described above, the spherical silicone rubber particles, the polymethylsilsesquioxane particles, and the silicone composite particles prepared by coating spherical silicone rubber particles with a polyorganosilsesquioxane resin are mixed into various cosmetics etc. However, when these particles are released or discharged into natural environments such as soil, inland water, seawater, and oceans, it is feared that these particles remain present in the environments without degradation because they do not have a degradable structure or unit or a structure or unit capable of imparting degradability.

In addition, plastics discharged into the ocean and microplastics which are plastics that have been degraded and reduced in size to the order of millimeters to micrometers have the characteristic of adsorbing harmful substances, pathogens, etc. present in the environment. It is feared that, when marine organisms accidentally ingest these materials, ecosystems may be adversely affected. Therefore, there is a growing movement toward regulating microplastics.

Examples of spherical particles that are not microplastics to be added to various cosmetics include silica and biodegradable cellulose powders. However, their effects of imparting softness, spreadability, and light diffusibility are worse than those of the silicone rubber particles, the polymethylsilsesquioxane particles, and the silicone composite particles prepared by coating spherical silicone rubber particles with a polyorganosilsesquioxane resin.

Against this background, there is a demand for spherical elastomer particles that can degrade in the environment after use, do not remain as particles (solids), and can impart good softness, high spreadability, and high light diffusibility when added to cosmetics.

The present invention has been made in view of the foregoing circumstances, and it is an object to provide a cosmetic containing spherical elastomer particles and/or elastomer composite particles. The spherical elastomer particles contain, as a constitutional unit, a (co)polymer exhibiting high degradability under external stimuli such as light, heat, acids, and bases or through the action of microorganisms, fungi, etc. in natural environments such as soil, inland water, and ocean water/seawater.

### Solution to Problem

The present inventors have conducted extensive studies in order to achieve the foregoing object and found that the problems can be solved by a cosmetic containing spherical elastomer particles that are crosslinked particles of a copolymer having a specific polyester structure and a specific polyether structure and/or elastomer composite particles. Thus, the present invention has been completed.

Accordingly, the present invention provides cosmetic as set forth below:

<1> A cosmetic comprising at least one type of particles (i) and (ii) defined as:

(i) spherical elastomer particles that have a volume average particle diameter of 1.0 to 100 μm and are crosslinked particles of a copolymer having a polyester structure and a polyether structure; and
(ii) elastomer composite particles containing

the spherical elastomer particles (i) and
polyorganosilsesquioxane or silica present on surfaces of the spherical elastomer particles (i).

<2> The cosmetic according to <1>, wherein the copolymer is a polyester-polyether copolymer having at least two radical-polymerizable unsaturated groups per molecule.

<3> The cosmetic according to <2>, wherein the copolymer is a polyester-polyether copolymer represented by general formula (1) or (2) defined as:

[Chem. 1]

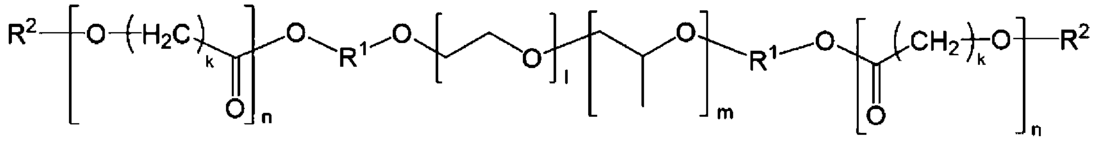

- (1)

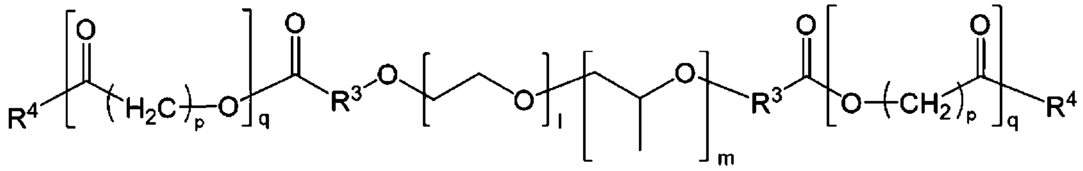

- (2)

wherein, in the general formula (1), each $R^1$ independently represents a divalent hydrocarbon group having 1 to 10 carbon atoms; each $R^2$ independently represents a radical-polymerizable functional group-containing organic group represented by general formula (3a), (3b), or (3c) as defined below; each k is independently a number satisfying $1 \leq k \leq 10$; 1 is a number satisfying $1 \leq 1 \leq 1{,}000$; m is a number satisfying $1 \leq m \leq 1{,}000$; and each n is independently a number satisfying $1 \leq n \leq 100$, and
wherein, in the general formula (2), each $R^3$ independently represents a divalent hydrocarbon group having 1 to 10 carbon atoms; each $R^4$ independently represents a radical-polymerizable functional group-containing organic group represented by general formula (4a) or (4b) as defined below; each p is independently a number satisfying $1 \leq p \leq 10$; 1 is a number satisfying $1 \leq 1 \leq 1{,}000$; m is a number satisfying $1 \leq m \leq 1{,}000$; and each q is independently a number satisfying $1 \leq q \leq 100$,

[Chemical formula 2]

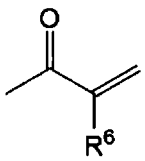

- (3a)

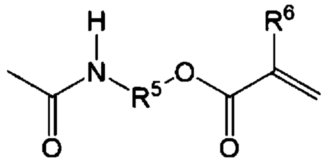

- (3b)

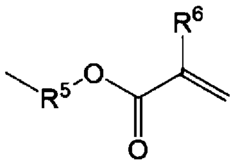

- (3c)

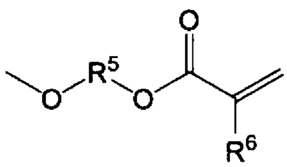

- (4a)

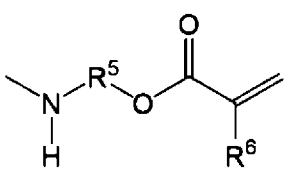

- (4b)

wherein, in the general formulas (3a), (3b), (3c), (4a), and (4b), each $R^5$ independently represents a divalent hydrocarbon group having 1 to 8 carbon atoms; and each $R^6$ independently represents a hydrogen atom or a

hydrocarbon group having 1 to 3 carbon atoms.

<4> The cosmetic according to <2>, wherein the copolymer is a polyester-polyether copolymer represented by general formula (5) defined as:

[Chemical formula 3]

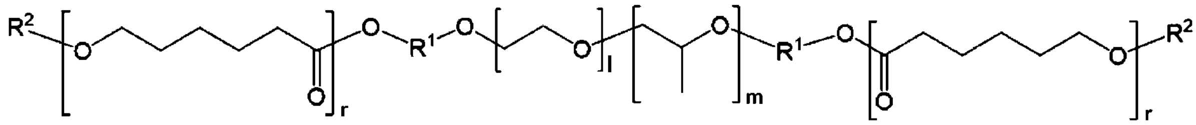

- (5)

wherein, in the general formula (5), each $R^1$ independently represents a divalent hydrocarbon group having 1 to 10 carbon atoms; 1 is a number satisfying 1 ≤ 1 ≤ 1,000; m is a number satisfying 1 ≤ m ≤ 1,000; each r is independently a number satisfying 1 ≤ r ≤ 100, and each $R^2$ independently represents a radical-polymerizable functional group-containing organic group represented by general formula (3a), (3b), or (3c) defined as

[Chemical formula 4]

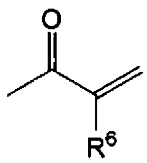

- (3a)

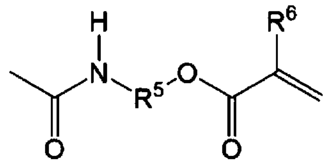

- (3b)

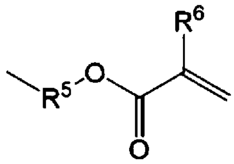

- (3c)

wherein, in the general formulas (3a), (3b), and (3c), each $R^5$ independently represents a divalent hydrocarbon group having 1 to 8 carbon atoms, and each $R^6$ independently represents a hydrogen atom or a hydrocarbon group having 1 to 3 carbon atoms.

<5> The cosmetic according to any one of <1> to<4>, wherein the spherical elastomer particles have a rubber hardness of 10 to 80 as measured by a type A durometer specified in JIS K 6253.

<6> The cosmetic according to any one of <1> to<5>, wherein the cosmetic is a skincare cosmetic.

<7> The cosmetic according to any one of <1> to<5>, wherein the cosmetic is a makeup cosmetic.

<8> The cosmetic according to any one of <1> to<5>, wherein the cosmetic is a suncare cosmetic.

Advantageous Effects of Invention

The cosmetic of the present invention containing spherical elastomer particles and/or elastomer composite particles can provide a cosmetic that exhibits good spreadability, softness, adherence, and mixability and that has an enhanced skin texture correcting effect.

The elastomer composite particles prepared by causing polyorganosilsesquioxane or silica to adhere to the surfaces of the spherical elastomer particles are excellent in spreadability, usability, adhesiveness, dispersibility, a skin texture correcting effect, water resistance, and sebum resistance. The cosmetic of the present invention using these composite particles can provide a cosmetic that can spread smoothly, is soft, can be applied uniformly, is excellent in dispersibility and sebum resistance, and has excellent staying power. Moreover, the elastomer composite particles can be mixed into any cosmetic, and the resulting cosmetic exhibits very high stability without deterioration under varying temperatures and over time.
The spherical elastomer particles and/or the elastomer composite particles have a polyester structure with a degradable unit skeleton in the particles. The crosslinked structure of the particles is cleaved in the presence of water, and therefore the particles have degradability. In particular, particles having, as the polyester structure in the particles, a poly-ε-caprolactone structure, which is a microbe recognition skeleton, are expected to have environmental degradability and biodegradability.
Therefore, the cosmetic of the present invention containing the spherical elastomer particles and/or the elastomer composite particles is expected to be used/utilized as an environmental load-reducing material.

## Brief Description of Drawings

[FIG. 1] FIG. 1 is an electron microscope photograph of spherical elastomer particles obtained in preparation example 1.
[FIG. 2] FIG. 2 is an electron microscope photograph of spherical elastomer particles obtained in preparation example 3.
[FIG. 3] FIG. 3 is an electron microscope photograph (magnification: 1,000X) of elastomer composite particles (silica-coated spherical elastomer particles) obtained in preparation/production example 3.
[FIG. 4] FIG. 4 is an electron microscope photograph (magnification: 7,500X) of the elastomer composite particles (silica-coated spherical elastomer particles) obtained in the preparation/production example 3.

## Description of Embodiments

The cosmetic of the present invention contains

(i) spherical elastomer particles that have a volume average particle diameter of 1.0 to 100 μm and are crosslinked particles of a copolymer having a polyester structure and a polyether structure and/or
(ii) elastomer composite particles including the spherical elastomer particles and polyorganosilsesquioxane or silica present on the surfaces of the spherical elastomer particles.

The present invention will next be described in detail in the following order:

I. spherical elastomer particles and a method for producing the spherical elastomer particles;
II. elastomer composite particles and a method for producing the elastomer composite particles;
III. a method for producing a cosmetic using the spherical elastomer particles and the elastomer composite particles; and
IV. a cosmetic containing the spherical elastomer particles and the elastomer composite particles.

## I. Spherical elastomer particles and method for producing spherical elastomer particles

The spherical elastomer particles added to the cosmetic of the present invention are a polymer (crosslinked particles) of a copolymer having a polyester structure and a polyether structure.
Preferably, the spherical elastomer particles have a spherical shape.
The term "spherical" as used herein not only refers to a perfect spherical particle shape but also encompasses deformed elliptical shapes with an average aspect ratio (the length of the maximum major axis / the length of the minimum minor axis) in the range of typically 1 to 4, preferably 1 to 2, more preferably 1.0 to 1.6, and still more preferably 1.0 to 1.4.
As will be shown in a production method described later, when a method in which a copolymer having a polyester structure and a polyether structure is emulsified, suspended, and dispersed using a surfactant or a suspending agent to crosslink the copolymer is used, the shape of the particles obtained is spherical.
The shape of the spherical elastomer particles can be checked by observation using an optical microscope or an electron microscope. The aspect ratio of the particles is a value obtained by measuring the maximum major axes and

minimum minor axes of randomly selected 50 particles in a microscope photograph and averaging the measurements.

In the present invention, the volume average particle diameter of the spherical elastomer particles is in the range of 1.0 to 100 μm and is preferably in the range of 1.0 to 50 μm and more preferably in the range of 2.0 to 20 μm.

If the volume average particle diameter is larger than 100 μm, the flowability of the particles decreases, and the cohesiveness increases. Therefore, smoothness and light diffusibility cannot be imparted to the particles sufficiently. If the volume average particle diameter is smaller than 1.0 μm, the dry feel, smoothness, etc. of the particles deteriorate. This may result in a grainy feel, and light diffusibility deteriorates.

The volume average particle diameter of the spherical elastomer particles is a value measured as follows. Before the measurement of the volume average particle diameter, the diameters of randomly selected 50 particles in a microscope photograph of the spherical elastomer particles are measured, and whether the average is more than or equal to 1 μm is determined. Various surfactants are used to redisperse the spherical elastomer particles in water to prepare dispersions. When the result of the determination described above is more than or equal to 1 μm, the volume average particle diameter is a value measured by an electric resistance method. When the result of the determination is less than 1 μm, the volume average particle diameter is a value measured by a laser diffraction/scattering method.

Preferably, the rubber (elastomer) that is a component of the spherical elastomer particles exhibits no tackiness or stickiness. The rubber hardness of the rubber (elastomer) that is a component of the spherical elastomer particles may be measured using a type A durometer specified in JIS K6253, and this rubber hardness is preferably in the range of 10 to 80 and more preferably in the range of 20 to 70. Moreover, the rubber hardness of the rubber (elastomer) that is a component of the spherical elastomer particles may be measured using an ASKER rubber hardness tester type C specified in The Society of Rubber Industry, Japan Standard (SRIS), and this rubber hardness is preferably in the range of 10 to 90, more preferably in the range of 20 to 90, and still more preferably in the range of 40 to 90.

If the rubber hardness value obtained by any of the above methods is less than 10, the cohesiveness of the particles is high, and the dispersibility deteriorates. If the rubber hardness measured using the type A durometer is larger than 80, the soft feel deteriorates, which is undesirable.

The spherical elastomer particles are preferably polymer particles of a polyester-polyether copolymer having at least two radical-polymerizable unsaturated groups per molecule, i.e., crosslinked particles of the polyester-polyether copolymer having at least two radical-polymerizable unsaturated groups per molecule.

The polyester-polyether copolymer having at least two radical-polymerizable unsaturated groups per molecule is preferably a copolymer represented by general formula (1) or (2) defined as:

[Chem. 5]

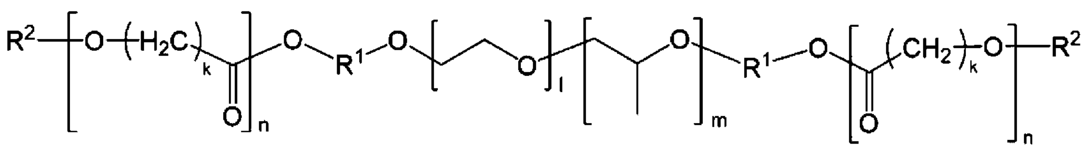

- (1)

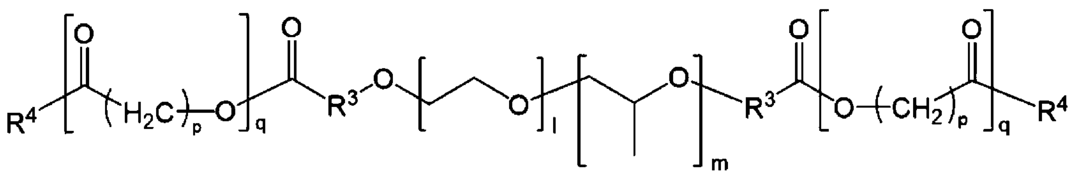

- (2)

wherein, in the general formula (1), each $R^1$ independently represents a divalent hydrocarbon group having 1 to 10 carbon atoms; each $R^2$ independently represents a radical-polymerizable functional group-containing organic group represented by general formula (3a), (3b), or (3c) shown below; each k is independently a number satisfying $1 \leq k \leq 10$; 1 is a number satisfying $1 \leq 1 \leq 1,000$; m is a number satisfying $1 \leq m \leq 1,000$; and each n is independently a number satisfying $1 \leq n \leq 100$, and

wherein, in the general formula (2), each $R^3$ independently represents a divalent hydrocarbon group having 1 to 10 carbon atoms; each p is independently a number satisfying $1 \leq p \leq 10$; 1 is a number satisfying $1 \leq 1 \leq 1,000$; m is a number satisfying $1 \leq m \leq 1,000$; each q is independently a number satisfying $1 \leq q \leq 100$; and each $R^4$ independently represents a radical-polymerizable functional group-containing organic group represented by general formula (4a) or (4b) defined as

[Chem. 6]

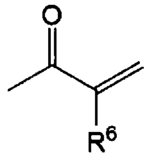

- (3a)

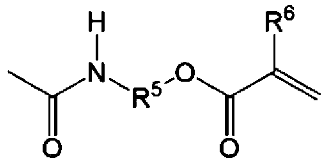

- (3b)

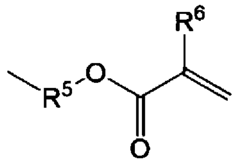

- (3c)

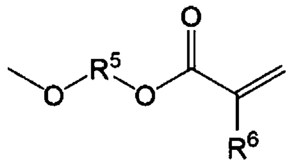

- (4a)

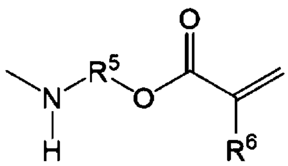

- (4b)

wherein, in the general formulas (3a), (3b), (3c), (4a), and (4b), each $R^5$ independently represents a divalent hydrocarbon group having 1 to 8 carbon atoms, and each $R^6$ independently represents a hydrogen atom or a hydrocarbon group having 1 to 3 carbon atoms.

Examples of $R^1$ include alkylene groups such as a methylene group, an ethylene group, a propylene group, a trimethylene group, a tetramethylene group, a pentamethylene group, a hexamethylene group, a heptamethylene group, and an octamethylene group. $R^1$ is preferably a methylene group, an ethylene group, a trimethylene group, or a tetramethylene group.

Examples of $R^3$ include alkylene groups such as a methylene group, an ethylene group, a propylene group, a trimethylene group, a tetramethylene group, a pentamethylene group, a hexamethylene group, a heptamethylene group, and an octamethylene group. $R^3$ is preferably a methylene group, an ethylene group, a trimethylene group, or a tetramethylene group.

$R^2$ represents a radical-polymerizable functional group-containing organic group represented by the general formula (3a), (3b), or (3c), and $R^4$ represents a radical-polymerizable functional group-containing organic group represented by the general formula (4a) or (4b).

Examples of $R^5$ in the general formulas (3b), (3c), (4a), and (4b) include alkylene groups such as a methylene group, an ethylene group, a propylene group, a trimethylene group, a tetramethylene group, a pentamethylene group, a hexamethylene group, a heptamethylene group, and an octamethylene group. $R^5$ is preferably a methylene group, an ethylene group, a trimethylene group, or a tetramethylene group.

Examples of $R^6$ in the general formulas (3a), (3b), (3c), (4a), and (4b) include a hydrogen atom and alkyl groups having 1 to 3 carbon atoms such as a methyl group, an ethyl group, and a propyl group. $R^6$ is preferably a hydrogen atom or a methyl group.

The radical-polymerizable functional group-containing organic groups represented by the general formulas (3a), (3b), (3c), (4a), and (4b) are residues derived from polymerizable monomers. Specific examples of the polymerizable monomers include hydroxy group-containing (meth)acrylates, isocyanate group-containing (meth)acrylates, and (meth) acryloyl chlorides.

Examples of the hydroxy group-containing (meth)acrylate include: C2-C8 hydroxyalkyl esters of (meth)acrylic

acid such as hydroxyethyl (meth)acrylate and hydroxypropyl (meth)acrylate; and carboxy (meth)acrylates such as carboxyethyl (meth)acrylate, (meth)acryloyloxyethyl succinate, and (meth)acryloyloxyethyl phthalate.

Examples of the isocyanate group-containing (meth)acrylate include isocyanatoethyl (meth)acrylate, isocyanatopropyl (meth)acrylate, isocyanatobutyl (meth)acrylate, and isocyanatohexyl (meth)acrylate.

Examples of the (meth)acryloyl chloride include acryloyl chloride, methacryloyl chloride, acryloyl bromide, and methacryloyl bromide.

The polyester structure (unit) in the polyester-polyether copolymer having at least two radical-polymerizable unsaturated groups per molecule is preferably degradable aliphatic polyester or aliphatic polyester known to exhibit high degradability.

Examples of the aliphatic polyester include poly-ε-caprolactone, poly-β-propiolactone, γ-butyrolactone, polylactic acid, polyhydroxybutyrate, polyglycolic acid, polyethylene adipate, polyhydroxybutyric acid, polyethylene succinate, and polybutylene succinate. From the viewpoint of degradability and ease of handling, those having a poly-ε-caprolactone structure are preferred.

In the general formula (1), each k is independently a number satisfying $1 \leq k \leq 10$ and preferably a number satisfying $1 \leq k \leq 6$.

In the general formula (2), each p is independently a number satisfying $1 \leq p \leq 10$ and preferably a number satisfying $1 \leq p \leq 6$.

In the general formula (1), each n is independently a number satisfying $1 \leq n \leq 100$, preferably a number satisfying $1 \leq n \leq 50$, and more preferably a number satisfying $2 \leq n \leq 20$.

In the general formula (2), each q is independently a number satisfying $1 \leq q \leq 100$, preferably a number satisfying $1 \leq q \leq 50$, and more preferably a number satisfying $2 \leq q \leq 20$.

If n and q are larger than their upper limits, the crystallinity becomes high due to intra-/inter-molecular interactions of the polymer caused by ester structures (units). In this case, when the flowability of the copolymer is excessively low, the handleability in the step of preparing an emulsion (suspension) composition described later may be influenced.

In the general formulas (1) and (2), 1 is a number satisfying $1 \leq 1 \leq 1{,}000$ and preferably a number satisfying $2 \leq 1 \leq 100$.

In the general formulas (1) and (2), m is a number satisfying $1 \leq m \leq 1{,}000$ and preferably a number satisfying $10 \leq m \leq 500$.

If the value of 1 is larger than the upper limit, the crystallinity becomes high due to intra-/inter-molecular interactions of the polymer caused by ethylene oxide structures (units), and this may cause a reduction in the flowability of the copolymer. In this case, as in the above case, the handleability in the step of preparing an emulsion (suspension) composition described later may be influenced, which is undesirable.

From the viewpoint of the handleability of raw materials and the ease of production, the polyester-polyether copolymer having at least two radical-polymerizable unsaturated groups per molecule is more preferably a copolymer represented by general formula (5) or (6) defined as:

[Chem. 7]

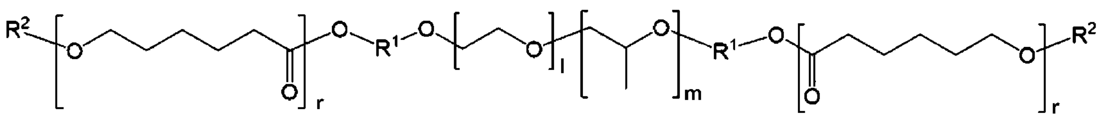

- (5)

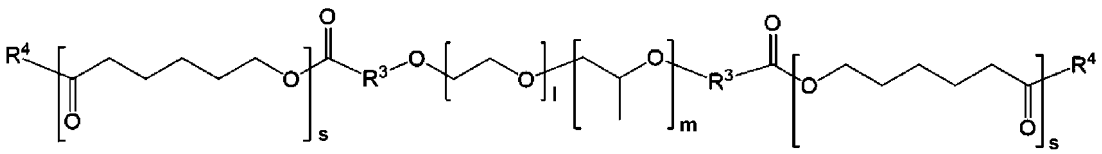

- (6)

wherein, in the general formula (5), each $R^1$ independently represents a divalent hydrocarbon group having 1 to 10 carbon atoms; each $R^2$ independently represents a radical-polymerizable functional group-containing organic group represented by the general formula (3a), (3b), or (3c); 1 is a number satisfying $1 \leq 1 \leq 1{,}000$, m is a number satisfying $1 \leq m \leq 1{,}000$; and each r is independently a number satisfying $1 \leq r \leq 100$,

wherein, in the general formula (6), each $R^3$ independently represents a divalent hydrocarbon group having 1 to 10 carbon atoms; each $R^4$ independently represents a radical-polymerizable functional group-containing organic group represented by the general formula (4a) or (4b); 1 is a number satisfying $1 \leq 1 \leq 1{,}000$; m is a number satisfying $1 \leq m \leq 1{,}000$; and each s is independently a number satisfying $1 \leq s \leq 100$.

[Method for producing polyester-polyether copolymer]

Before explaining the method for producing spherical elastomer particles, a method for producing the polyester-polyether copolymer that constitutes the spherical elastomer particles will be described.

An example of the method for producing the polyester-polyether copolymer is a method in which a polyether containing active hydrogen such as a polyether having a hydroxy group, a carboxy-modified polyether, or an amino-modified polyether is used as a starting material, and cyclic-ε-caprolactone, γ-butyrolactone, or the like is then subjected to ring-opening polymerization to produce a poly-ε-caprolactone-modified polyether or a poly-γ-butyrolactone-modified polyether, followed by introducing a polymerizable monomer having a radical-polymerizable unsaturated group via an ester bond, an ether bond, a urethane bond, an amide bond, etc.

From the viewpoint of reactivity during production, it is preferable that the terminal structure of each polyether is a structure in which a reactive functional group is bonded to a primary carbon atom.

Examples of the reaction conditions for the above production method include, but are not limited to, the following reaction conditions.

For example, 3.0 to 4.0 equivalents (functional group equivalent ratio) of cyclic-ε-caprolactone is added to 1.0 equivalent of a polyether or an active hydrogen-containing polyether such as a carboxy-modified polyether, and the mixture is allowed to react at 120°C for 4 to 6 hours in the presence of a well-known ring-opening polymerization catalyst to thereby obtain a poly-ε-caprolactone-modified polyether.

Next, for example, 1.0 to 1.25 moles (functional group equivalent ratio) of (meth)acryloyl chloride such as acryloyl chloride or isocyanate group-containing (meth)acrylate such as isocyanatoethyl (meth)acrylate is added relative to 1.0 mole of hydroxy groups in the obtained poly-ε-caprolactone-modified polyether. A reaction catalyst is optionally added to the mixture, and the resulting mixture is allowed to react at 40 to 100°C for 4 hours or longer.

After the reaction, alcohol etc. is added to the crude product for quenching, and the resulting product is subjected to filtration, washing with water, and/or adsorption treatment to remove by-products. Finally, the solvent is removed by evaporation to thereby obtain an acrylic-modified poly-ε-caprolactone polyether (polyester-polyether copolymer).

Examples of the polyester-polyether copolymer when a polyether having a hydroxy group is used as a starting material include copolymers represented by formulas (7a) and (7b) (only the structure on one side is shown because of their symmetric structure).

[Chem. 8]

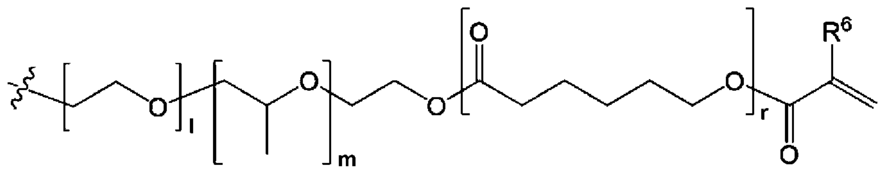

- (7a)

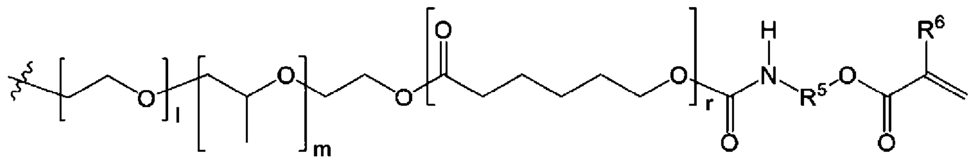

- (7b)

In the formulas (7a) and (7b), $R^5$ is a divalent hydrocarbon group having 1 to 8 carbon atoms, and $R^6$ is a hydrogen atom or a hydrocarbon group having 1 to 3 carbon atoms.

1, m, and r satisfy $1 \leq 1 \leq 1{,}000$, $1 \leq m \leq 1{,}000$, and $1 \leq r \leq 30$ and preferably satisfy $2 \leq 1 \leq 100$, $10 \leq m \leq 500$, and $2 \leq r \leq 10$.

Examples of the polyester-polyether copolymer when a carboxy-modified polyether is used as a starting material include copolymers represented by formulas (8a) and (8b) (only the structure on one side is shown because of their symmetric structure).

[Chem. 9]

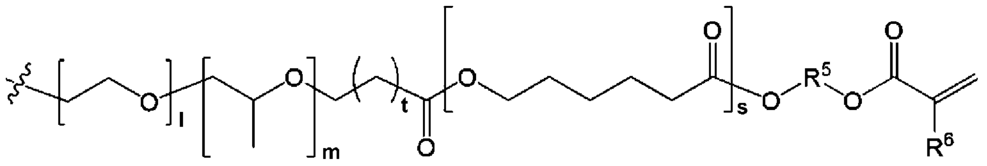

- (8a)

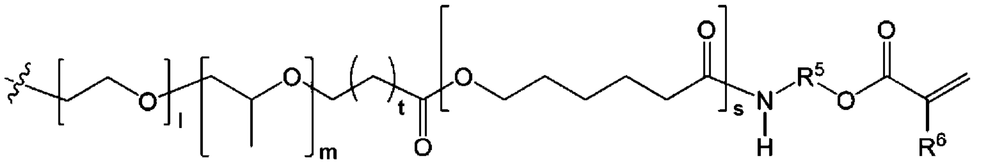

- (8b)

In the formulas (8a) and (8b), $R^5$ is a divalent hydrocarbon group having 1 to 8 carbon atoms, and $R^6$ is a hydrogen atom or a hydrocarbon group having 1 to 3 carbon atoms.

l, m, s, and t satisfy $1 \leq 1 \leq 1{,}000$, $1 \leq m \leq 1{,}000$, $1 \leq s \leq 30$, and $0 \leq t \leq 10$ and preferably satisfy $2 \leq 1 \leq 100$, $10 \leq m \leq 500$, $2 \leq s \leq 10$, and $1 \leq t \leq 10$.

No particular limitation is imposed on the catalyst used for ring-opening polymerization of cyclic-ε-caprolactone, and a well-known catalyst may be used.

Specific examples of the catalyst include: organotitanium-based compounds such as tetramethoxytitanium, tetraethoxytitanium, tetra-n-propoxytitanium, and tetra-n-butoxytitanium; organotin compounds such as di-n-butyltin laurate, diisobutyltin oxide, and dibutyltin diacetate; acetates of magnesium, calcium, zinc, etc.; antimony oxide; stannous halides; and perchloric acid.

The amount of the ring-opening polymerization catalyst added may be in the range of 1 to 10,000 ppm and is preferably 10 to 1,000 ppm based on the amount of the ε-caprolactone monomer (cyclic-ε-caprolactone).

To introduce the polymerizable monomer having a radical-polymerizable unsaturated group into the poly-ε-caprolactone-modified polyether, any of various catalysts may be used according to the reactive moiety (reactive functional group) of the polymerizable monomer and the skeleton to be formed, and a well-known catalyst may be used.

When the reaction of the polymerizable monomer and the poly-ε-caprolactone-modified polyether is an esterification reaction, a catalyst may be used, and examples of the (esterification) catalyst used for the esterification reaction include: Lewis acid catalysts such as alcoholates, carboxylates, and chelate compounds of titanium, zirconium, tin, aluminum and zinc, as well as boron trifluoride, and boron trifluoride etherate; acid catalysts such as hydrochloric acid, sulfuric acid, hydrogen bromide, acetic acid, trifluoroacetic acid, methanesulfonic acid, and p-toluenesulfonic acid; and amine-based catalysts such as pentamethyldiethylenetriamine (PMDETA), trimethyltriazacyclononane (TACN), triethylamine (TEA), 4-(N,N-dimethylamino)pyridine (DMAP), 1,4-diazabicyclo(2,2,2)octane (DABCO), and tetramethylethylenediamine (TMEDA). In particular, amine-based catalysts are preferred from an economical point of view and the viewpoint of the stability of the product obtained through the esterification reaction.

When an amine-based catalyst is used, a dehydration-condensing agent may be added to improve the reaction efficiency. A well-known dehydration-condensing agent may be used, and examples thereof include, but are not limited to, 1,1'-carbonyldiimidazole (CDI), N,N'-dicyclohexylcarbodiimide (DCC), N,N'-diisopropylcarbodiimide (DIC), 1-ethyl-3-(3-dimethylaminopropyl)-carbodiimide (EDC), 1-ethyl-3-(3-dimethylaminopropyl)-carbodiimide hydrochloride (EDC-HCl), and 1-[bis(dimethylamino)methylene]-1H-1,2,3-triazolo[4,5-b]pyridinium 3-oxide hexafluorophosphate (HATU).

When the reaction of the polymerizable monomer and the poly-ε-caprolactone-modified polyether is a urethanization reaction, a catalyst may be used. Examples of the catalyst used for the urethanization reaction include: amines such as triethylamine, triethylenediamine, pentamethylenediethylenetriamine, N,N-dimethylethanolamine, 1,4-diazabicyclo(2,2,2)octane (DABCO), pyridine, and N,N,N',N'-tetramethyl-1,3-propanediamine (TMPDA); organotin compounds such as di-n-butyltin laurate, diisobutyltin oxide, dibutyltin diacetate, dibutyltin dilaurate (DBTL), and dioctyltin dineodecanoate; organotitanium compounds such as tetramethoxytitanium, tetraethoxytitanium, tetra-n-propoxytitanium, tetrabutoxytitanium, tetrakis(octyloxy)titanium, titanium acetylacetonate, titanium tetraacetylacetonate, a titanium dodecylbenzene sulfonate compound, a titanium phosphate complex, titanium triethanolaminate, and titanium diisopropoxy bis(ethylacetoacetate); organozirconium compounds such as n-propyl zirconate, n-butyl zirconate, zirconium tetraacetylacetonate, zirconium dibutoxy bis(ethylacetoacetate), and a zirconium octylate compound; and organoiron compounds such as tris(2,4-pentanedionato)iron(III).

The amount of the catalyst added when the polymerizable monomer having a radical-polymerizable unsaturated group is introduced may be in the range of 1 to 10,000 ppm and is preferably 10 to 1,000 ppm based on the total amount of the polymerizable monomer.

In the reaction described above, a polymerization inhibitor or an antioxidant may be used for the purpose of

inhibiting the polymerization reaction of (meth)acrylate groups during the reaction.

Examples of the polymerization inhibitor and the antioxidant include, but are not limited to, hydroquinone, p-methoxyphenol, 2,6-di-tert-butyl-p-cresol, 2,4-dimethyl-6-t-butylphenol, p-benzoquinone, dibutylhydroxytoluene, 2,5-dihydroxy-p-benzoquinone, and mequinol.

When a carboxy-modified polyether is used as a starting material, the polyester-polyether copolymer can be produced by a production method in which the carboxy-modified polyether and poly-ε-caprolactone-modified (meth) acrylate represented by formula (9) below are subjected to an esterification reaction.

Commercial examples of the poly-ε-caprolactone-modified (meth)acrylate include PLACCEL FA2D, PLACCEL FA10L, PLACCEL FN2D, and PLACCEL FM4 (manufactured by Daicel Corporation).

[Chem. 10]

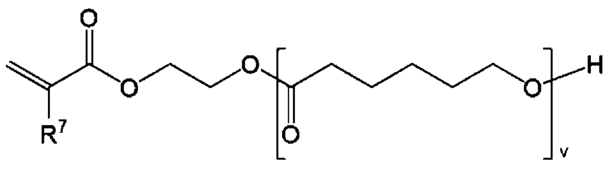

- (9)

In the general formula (9), $R^7$ is a hydrogen atom or a monovalent hydrocarbon group having 1 to 3 carbon atoms and preferably a hydrogen atom or a methyl group. v satisfies $1 \leq v \leq 50$ and preferably satisfies $1 \leq v \leq 30$.

Examples of the method for producing the polyester-polyether copolymer include, but are not limited to, the following method.

1.0 to 1.25 moles (functional group equivalent ratio) of the poly-ε-caprolactone-modified (meth)acrylate (formula (9)) and 0.1 to 5.0 moles of an esterification catalyst are added relative to 1.0 moles of carboxy groups in the carboxy-modified polyether and mixed therewith, and the mixture is stirred at 15 to 150°C for 10 to 30 minutes. Then 1.0 to 1.25 moles of a dehydration-condensing agent is optionally added, and the resulting mixture is allowed to react at 15 to 150°C for 4 to 20 hours.

After the reaction, the crude product is subjected to filtration, washing with water, and/or adsorption treatment to remove by-products. Finally, the solvent is removed by evaporation, and an acrylic-modified polyester (poly-ε-caprolactone)-polyether copolymer can thereby be obtained.

The obtained polyester-polyether copolymer having at least two radical-polymerizable unsaturated groups per molecule is preferably liquid, and its weight average molecular weight (Mw) measured by gel permeation chromatography (GPC) is preferably in the range of 100 to 100,000 and is more preferably 500 to 50,000.

If the weight average molecular weight is less than the lower limit, the crosslinking density of the elastomer in the obtained spherical elastomer/elastomer composite particles is high, and degradability may deteriorate, which is undesirable. If the weight average molecular weight is larger than the upper limit, the viscosity of the copolymer is high, and it may be difficult to prepare the spherical elastomer particles.

In the filtration step, a dilution operation using a hydrophobic organic solvent may be performed for the purpose of adjusting the viscosity of the crude reaction product.

No particular limitation is imposed on the hydrophobic organic solvent used. From the viewpoint of the ability to dissolve the polyester-polyether copolymer and the affinity therefor, toluene, hexane, ethyl acetate, etc. are preferred.

The adsorption step is performed for the purpose of removal of hydrochlorides unremovable by washing with water, dehydration, decolorization, and deodorization.

A well-known adsorbent may be used, and a combination of a plurality of adsorbents may be used. Preferred examples of the adsorbent include desiccants such as magnesium sulfate and sodium sulfate, activated carbon, silica gel, and the KYOWAAD series (manufactured by Kyowa Chemical Industry Co., Ltd.).

[Method for producing spherical elastomer particles]

Next, a method for producing spherical elastomer particles using the polyester-polyether copolymer obtained by the method described above will be described.

The spherical elastomer particles can be produced using a well-known method via a dispersion state of the spherical elastomer particles.

The spherical elastomer particles can be produced, for example, by a method including the following steps i) to iii) in which:

the step i) is a step of stirring, emulsifying, and suspending components (A), (B), (C), and (D) shown below to prepare

an emulsion (suspension) composition,

(A) a copolymer having a polymerizable group and having a polyester structure and a polyether structure,
(B) an oil phase component or an aqueous phase component in which the component (A) is insoluble or poorly soluble,
(C) a surfactant or a suspending agent, and
(D) a polymerization initiator;

the step ii) is a step of subjecting the component (A) in the emulsion (suspension) composition obtained through the step i) to (radical) polymerization under an external stimulus such as heat, light, or UV light to thereby obtain a spherical elastomer particle dispersion; and
the step iii) is a step of removing the component (B) present as a continuous phase from the spherical elastomer particle dispersion obtained through the step ii) by washing and drying to thereby obtain spherical elastomer particles.

When the spherical elastomer particles are obtained by the above-described production method of preparing the emulsion (suspension) composition and then performing the (radical) polymerization reaction, the particles obtained have a spherical shape.

Step i)

In the step i), the components (A), (B), (C), and (D) explained below are stirred, emulsified, and suspended to prepare an emulsion (suspension) composition.
The components used in the step i) are as explained below.
The component (A) is a copolymer having a polymerizable group and having a polyester structure and a polyether structure and is preferably a polyester-polyether copolymer having a radical-polymerizable unsaturated group.
The constitutional units of the spherical elastomer particles are derived from the copolymer having a polyester structure and a polyether structure, and the above-described polyester-polyether copolymer having at least two radical-polymerizable unsaturated groups per molecule can be preferably used.
Specific examples of the component (A) include the polyester-polyether copolymers represented by the general formulas (1) and (2) described above. The component (A) is more preferably the polyester-polyether copolymer represented by the general formula (5) or (6) described above.
The content of the component (A) in the emulsion (suspension) composition prepared in the step i) is preferably 1.0 to 80 parts by mass based on 100 parts by mass of the composition. If the amount of the component (A) is less than the lower limit, the production efficiency may deteriorate. If the amount of the component (A) is more than the upper limit, the degree of emulsification (suspension) is insufficient. In this case, it may be difficult to obtain the dispersion of the spherical elastomer particles, which is undesirable.
The component (B) is a component that forms a continuous phase in the emulsion (suspension) composition and is an oil phase component or an aqueous phase component in which the component (A) is insoluble or poorly soluble.
When the component (B) is an aqueous phase component, specific examples of the water contained in the aqueous phase component include distilled water, ion exchanged water, pure water, and ultrapure water.
An additive may be optionally added to the aqueous phase component, so long as the function of the continuous phase is not impaired. Specific examples of the additive include, but are not limited to, a preservative, salts, a pH modifier, a chelating agent, vitamins, amino acids, a moisturizing agent, and an antioxidant. To adjust the composition of the aqueous phase component such that the component (A) is insoluble or poorly soluble, the content of water in the aqueous phase component is preferably 90 to 100% by mass.
When the component (B) is an oil phase component, examples of the oil phase component include, but are not limited to, silicone oils, hydrocarbon oils, higher fatty acids, ester oils, and liquid fats and oils. One of them may be used alone, or an appropriate combination of two or more may be used.
Examples of the silicone oil include dimethylpolysiloxane, methylhydrogenpolysiloxane, methylphenylpolysiloxane, octamethylsiloxane, decamethyltetrasiloxane, decamethylcyclopentasiloxane, hexamethylcyclotrisiloxane, and octamethylcyclotetrasiloxane.
Examples of the hydrocarbon oil include liquid paraffin, $\alpha$-olefin oligomers, isododecane, isohexadecane, squalane, ozokerite, squalene, ceresin, paraffin, isoparaffin, paraffin wax, polyethylene wax, polyethylene-polypropylene wax, pristane, polyisobutylene, Vaseline, and microcrystalline wax.
Examples of the higher fatty acid include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.
Examples of the ester oil include isopropyl myristate, cetyl octanoate, octyldodecyl myristate, isopropyl

palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, isononyl isononanoate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerin di-2-heptylundecanoate, trimethylolpropane tri-2-ethylhexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of the liquid fats and oils include avocado oil, camellia oil, turtle oil, macadamia nut oil, corn oil, mink oil, olive oil, rape oil, egg yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, Chinese tung oil, Japanese tung oil, jojoba oil, germ oil, and triglycerin.

The kinematic viscosity of the component (B) at 25°C is preferably 100,000 $mm^2/s$ or less and more preferably 10,000 $mm^2/s$ or less. If the kinematic viscosity is larger than the upper limit, it is difficult to emulsify (suspend) the components in the step i), and obtaining an emulsion (suspension) composition with a narrow particle size distribution and spherical elastomer particles may also be difficult.

When the component (C) used is a surfactant, no particular limitation is imposed on the type of surfactant, and a well-known nonionic surfactant, a well-known anionic surfactant, a well-known cationic surfactant, or a well-known amphoteric surfactant may be used. One surfactant may be used alone, or an appropriate combination of two or more surfactants may be used.

Examples of the nonionic surfactant include polyoxyethylene alkyl ethers, polyoxyethylene polyoxypropylene alkyl ethers, polyoxyethylene alkyl phenyl ethers, polyethylene glycol fatty acid esters, sorbitan fatty acid esters, polyoxyethylene sorbitan fatty acid esters, polyoxyethylene sorbit fatty acid esters, glycerin fatty acid esters, polyoxyethylene glycerin fatty acid esters, polyglycerin fatty acid esters, propylene glycol fatty acid esters, polyoxyethylene castor oils, polyoxyethylene hydrogenated castor oils, polyoxyethylene hydrogenated castor oil fatty acid esters, polyoxyethylene alkyl amines, polyoxyethylene fatty acid amides, polyoxyethylene-modified organopolysiloxanes, and polyoxyethylene polyoxypropylene-modified organopolysiloxanes.

Examples of the polyoxyethylene-modified organopolysiloxane include linear type polyether-modified silicones (product names: KF-6011, KF-6011P, and KF-6043 manufactured by Shin-Etsu Chemical Co., Ltd.), linear type alkyl co-modified polyether-modified silicones (product name: KF-6048 manufactured by Shin-Etsu Chemical Co., Ltd.), branched type polyether-modified silicones (product names: KF-6028 and KF-6028P manufactured by Shin-Etsu Chemical Co., Ltd.), and branched type alkyl co-modified polyether-modified silicones (product name: KF-6038 manufactured by Shin-Etsu Chemical Co., Ltd.).

Examples of the anionic surfactant include alkyl sulfate salts such as sodium lauryl sulfate, polyoxyethylene alkyl ether sulfate salts, polyoxyethylene alkyl phenyl ether sulfate salts, alkylbenzenesulfonates, polyoxyethylene alkyl phenyl ether sulfonates, alkyl diphenyl ether disulfonates, alkane sulfonates, N-acyl taurates, dialkyl sulfosuccinates, monoalkyl sulfosuccinates, polyoxyethylene alkyl ether sulfosuccinates, fatty acid salts, polyoxyethylene alkyl ether carboxylates, N-acylamino acid salts, monoalkyl phosphate salts, dialkyl phosphate salts, and polyoxyethylene alkyl ether phosphate salts.

Examples of the cationic surfactant include alkyltrimethylammonium salts, dialkyldimethylammonium salts, polyoxyethylene alkyldimethylammonium salts, dipolyoxyethylene alkylmethylammonium salts, tripolyoxyethylene alkylammonium salts, alkylbenzyldimethylammonium salts, alkylpyridinium salts, monoalkylamine salts, and monoalkylamidoamine salts.

Examples of the amphoteric surfactant include alkyldimethylamine oxides, alkyldimethylcarboxybetaines, alkylamidopropyl dimethylcarboxybetaines, alkylhydroxysulfobetaines, and alkylcarboxymethylhydroxyethylimidazoliniuim betaines.

The surfactant is preferably a nonionic surfactant or an anionic surfactant because the component (A) can be emulsified (suspended) using a small amount and fine spherical elastomer particles can be obtained.

When the component (B) used is water or an aqueous phase component containing water, the surfactant (C) has an HLB value, which is a parameter indicating the balance between hydrophilicity and hydrophobicity, of preferably 6.0 to 18.0 and more preferably 9.0 to 18.0. When the component (B) used is an oil phase component, the surfactant (C) has an HLB value of preferably 2.0 to 13.0 and more preferably 2.0 to 9.0.

The amount of the surfactant (C) added is preferably 0.01 to 25 parts by mass and more preferably 0.05 to 15 parts by mass based on 100 parts by mass of the emulsion (suspension) composition. If the amount of the surfactant added is less than the lower limit, emulsification may be insufficient, or fine spherical elastomer particles may not be obtained, which is undesirable. If the amount added is larger than the upper limit, the viscosity of the composition as a whole

increases significantly. In this case, fine spherical elastomer particles may not be obtained, and the degree of dispersion of the particles may be insufficient, which is undesirable.

When the component (C) used is a suspending agent, no particular limitation is imposed on the type of suspending agent. Examples of the suspending agent include: well-known water-soluble macromolecular compounds such as natural macromolecular compounds, semi-synthetic macromolecular compounds, and synthetic macromolecular compounds; and macromolecular compounds used as thickeners. One of them may be used alone, or an appropriate combination of two or more may be used.

Examples of the natural macromolecular compound include xanthan gum, cellulose, tamarind gum, tamarind seed gum, locust bean gum, gellan gum, HM pectin, carrageenan, guar gum, linseed gum, gum arabic, pullulan, agarose, agaropectin, alginic acid, karaya gum, succinoglycan, starch, dextrin, gelatin, and casein.

Examples of the semi-synthetic macromolecular compound include methyl cellulose, ethyl cellulose, hydroxyethyl cellulose, hydroxypropyl cellulose, hydroxypropyl methyl cellulose, carboxymethyl cellulose, cationic xanthan gum, LM pectin (acid treated/alkali treated), cationic guar gum, alginates, soluble starch, and cellulose nanofibers.

Examples of the synthetic macromolecular compound include polyvinyl alcohol, polyvinyl methyl ether, polyvinylpyrrolidone, carboxyvinyl polymers, polyacrylic acids, sodium polyacrylate, ammonium polyacrylate, polyacrylamide, polyethylene glycol, polypropylene glycol, and polyethylene-polypropylene glycol.

The suspending agent is preferably xanthan gum, tamarind gum, carrageenan, guar gum, gum arabic, methyl cellulose, ethyl cellulose, hydroxyethyl cellulose, hydroxypropyl cellulose, hydroxypropyl methyl cellulose, carboxymethyl cellulose, cationic xanthan gum, cationic guar gum, polyvinyl alcohol, or polyvinylpyrrolidone because the component (A) can be suspended (emulsified) using a small amount of the suspending agent and fine spherical elastomer particles can be obtained.

The amount of the suspending agent added is preferably 0.01 to 25 parts by mass and more preferably 0.05 to 15 parts by mass based on 100 parts by mass of the emulsion (suspension) composition. If the amount of the suspending agent added is less than the lower limit, emulsification may be insufficient, or fine spherical elastomer particles may not be obtained, which is undesirable. If the amount of the suspending agent added is larger than the upper limit, the viscosity of the composition increases significantly. In this case, fine spherical elastomer particles may not be obtained, and the degree of dispersion of the particles may be insufficient, which is undesirable.

The component (D) used as a polymerization initiator may be a well-known radical polymerization initiator. By applying an external stimulus such as heat, light irradiation, or UV irradiation in the presence of the polymerization initiator to generate radicals, the reaction and curing (crosslinking) proceed.

Specific examples of the polymerization initiator (D) include peroxides, azo-based initiators, photo-initiators, and redox-based initiators obtained by combining an oxidizing agent and a reducing agent.

Examples of the peroxide include benzoyl peroxide, 2,4-dichlorobenzoyl peroxide, o-methylbenzoyl peroxide, p-methylbenzoyl peroxide, 2,4-dicumyl peroxide, 2,5-dimethyl-2,5-di(t-butylperoxy)hexane, di-t-butyl peroxide, t-butylperbenzoate, and hydrogen peroxide.

Perchlorates such as potassium perchlorate and sodium perchlorate may also be used.

Examples of the azo-based initiator include 2,2'-azobis-isobutyronitrile, 2,2'-azobis-(2-methylbutyronitrile), 2,2'-azobis-(2,4-dimethylvaleronitrile), dimethyl 2,2'-azobis-(2-methylpropionate), dimethyl 2,2'-azobis-isobutyrate, t-butylperoxy-2-ethylhexanoate, and 2,2-azobis-(2-aminodipropane)dihydrochloride.

Examples of the photo-initiator include 2,2-diethoxyacetophenone, 2,2-dimethoxy-1,2-diphenylethan-1-one, 1-hydroxy-cyclohexyl phenyl ketone, 2-hydroxy-2-methyl-1-phenylpropan-1-one, 2-hydroxy-1-{4-[4-(2-hydroxy-2-methylpropionyl)benzyl]phenyl}-2-methylpropan-1-one, phenylglyoxylic acid methyl ester, 2-methyl-1-[4-(methylthio)phenyl]-2-morpholinopropan-1-one, 2-benzyl-2-dimethylamino-1-(4-morpholinophenyl)-1-butanone, bis(2,4,6-trimethylbenzoyl)-phenylphosphine oxide, and (2,4,6-trimethylbenzoyl)-diphenylphosphine oxide.

Benzoin alkyl ethers such as benzoin methyl ether, benzoin ethyl ether, benzoin isopropyl ether, and benzoin isobutyl ether may also be used.

Examples of the redox-based initiator include a combination of ferrous sulfate/sodium pyrophosphate/glucose/hydroperoxide and a combination of ferrous sulfate/ethylenediaminetetraacetic acid disodium salt/Rongalite/hydroperoxide.

The redox-based initiator may be used in combination with an azo-based initiator or a photo-initiator.

In terms of the stability during the (radical) polymerization reaction of the emulsion (suspension) composition and ease of handling, the polymerization initiator is preferably a peroxide, an azo-based initiator, or a photo-initiator used for a heating method or a light irradiation method.

The amount of the polymerization initiator added is preferably in the range of 0.01 to 5.0 parts by mass based on 100 parts by mass of the component (A).

If the amount of the polymerization initiator added is less than the lower limit, curing (crosslinking) may be insufficient. If the amount of the polymerization initiator added is larger than the upper limit, reaction residues (contaminations) may be mixed, and this may cause odor, bleeding, etc., which is undesirable.

Additional additives

In the spherical elastomer particle production method, when the emulsion (suspension) composition is prepared in the step i), various different additives may be optionally added in addition to the components (A), (B), (C), and (D).

Examples of such additive include a thickener, a pH modifier, a preservative, an antioxidant, and a polymerization inhibitor. For each of them, one type may be used alone, or an appropriate combination of two or more types may be used. They may be used in appropriate amounts so long as the effects of the present invention are not impaired.

No particular limitation is imposed on the order of addition/mixing of the components in the step i). For example, the component (A) and the component (D) may be mixed in advance, and then the component (B) and the component (C) may be added thereto to prepare an emulsion (suspension) composition. Alternatively, the component (A), the component (B), and the component (C) may be used to prepare an emulsion (suspension) composition, and then the component (D) may be added thereto.

Alternatively, after the preparation of an emulsion (suspension) composition using the component (A), the component (B), the component (C), and the component (D), the component (B) may be further added for dilution before undergoing the step ii) to achieve the desired concentration.

When the component (A), i.e., the polyester-polyether copolymer having radical-polymerizable unsaturated groups, has temperature-dependent characteristics such as a cloudy point (i.e., when the component (A) exhibits a rapid decrease in solubility at a specific temperature during heating and causes phase separation), the emulsion (suspension) composition is prepared in the step i) at a temperature adjusted according to the temperature characteristics of the component (A). For example, stirring, emulsification, and suspension may be performed at an increased temperature under heating.

As for the temperature conditions at an increased temperature under heating, the temperature is, for example, 100°C or lower, preferably 30 to 90°C, and still more preferably in the range of 40 to 70°C. When the continuous phase is an aqueous phase component, if the increased temperature during the heating is higher than the upper limit, evaporation of water and bumping may occur, which is undesirable.

To prepare the emulsion (suspension) composition in the step i), a well-known emulsification/dispersion machine may be used. Typical examples of the emulsification/dispersion machine include a high-speed rotary shear-type agitator such as a homogenizing mixer, a high-speed centrifugal radiation-type agitator such as a homogenizing disper, a combi mix emulsification/dispersion machine which is a combination of a homogenizing mixer and a homogenizing disper, a mixing/emulsifying/agitating machine (agi-homo mixer) which is a combination of a homogenizing mixer or a homogenizing disper with an anchor mixer, a high-pressure injection-type emulsification/dispersion machine such as a homogenizer, a colloid mill, an ultrasonic emulsifying machine, and a propeller stirrer.

Step ii)

The step ii) is a step of subjecting the component (A) in the emulsion (suspension) composition prepared in the step i) to a reaction and curing (crosslinking) through (radical) polymerization to thereby obtain a spherical elastomer particle dispersion.

In the step ii), the polymerization reaction conditions can be appropriately determined according to the type of polymerization initiator (D).

When, for example, a peroxide or an azo-based initiator is used, a heating method in which the reaction is allowed to proceed at a temperature of 30 to 80°C for 10 to 24 hours is used. When a redox-based initiator is used, a redox method in which the reaction is allowed to proceed at a temperature of 30 to 70°C for 2 to 24 hours is used. When a photo-initiator is used, a light irradiation method in which the reaction is allowed to proceed under light irradiation is used. A well-known light source may be used for light or UV irradiation, and a well-known wavelength range may be used.

Step iii)

The step iii) is a step of removing the component (B) present as a continuous phase from the spherical elastomer particle dispersion obtained in the step ii) by washing and drying to thereby obtain spherical elastomer particles.

Specific examples of the method for the step iii) when the component (B) present as a continuous phase (dispersion medium) is an aqueous phase component in the step iii) include: a method of concentrating the dispersion by a method such as thermal dehydration, (pressure) filtration, centrifugal separation, and decantation, and then optionally adding pure water etc. to perform washing with water, and finally heat-drying the resulting product at normal pressure or under reduced pressure; a method of spraying the dispersion into a heated airflow to perform heat-drying (spray drying); a method of using a circulating heating medium to perform heat drying; and a freeze-drying method of freezing the dispersion and then reducing the pressure to remove the dispersion medium. By using any of the above methods, the spherical elastomer particles may be obtained.

When the spherical elastomer particles obtained by removing the dispersion medium by washing and drying are aggregated, the aggregated particles may be pulverized using a mortar, a ball mill, a jet mill, etc.

When the component (B) present as a continuous phase (dispersion medium) is an oil phase component in the step iii), specific examples of the method for performing the step iii) include a method in which a hydrophobic organic solvent is added to the spherical elastomer particle dispersion, and the mixture is stirred for a predetermined time and then subjected to pressure filtration to thereby remove the component (B) by washing and perform solvent replacement. By repeating this washing procedure a plurality of times, the component (B) can be removed sufficiently, and the solvent replacement can be completed. Examples of the hydrophobic organic solvent used for this procedure include toluene and hexane.

Finally, a method involving heat-drying at normal pressure or under reduced pressure, a method of spraying the dispersion into a heated airflow to perform heat-drying (spray drying), a method of using a circulating heating medium to perform heat drying, or a freeze-drying method of freezing the dispersion and then reducing the pressure to remove the dispersion medium may be performed to thereby obtain the spherical elastomer particles.

II. Elastomer composite particles and method for producing elastomer composite particles

The elastomer composite particles added to the cosmetic of the present invention include: the spherical elastomer particles that are crosslinked particles of the copolymer having polyester and polyether structures; and polyorganosilsesquioxane or silica on the surfaces of the spherical elastomer particles. Preferably, the elastomer composite particles include fine spherical polyorganosilsesquioxane particles or fine spherical silica particles adhering onto the spherical elastomer particles so as to cover their surfaces.

Preferably, the elastomer composite particles have a spherical shape.

The shape of the elastomer composite particles can be examined using an optical microscope or an electron microscope in the same manner as that used for the spherical elastomer particles.

In the present invention, the volume average particle diameter of the elastomer composite particles is in the range of 1.0 to 100 μm, preferably in the range of 1.0 to 50 μm, and more preferably in the range of 2.0 to 20 μm.

If the volume average particle diameter is larger than 100 μm, the flowability of the particles deteriorates, and the cohesiveness increases. In this case, sufficient smoothness and light diffusibility cannot be imparted to the particles. If the volume average particle diameter is smaller than 1.0 μm, the dry feel, smoothness, etc. of the particles deteriorate. This may result in a grainy feel, and light diffusibility deteriorates.

The volume average particle diameter of the elastomer composite particles can be measured by an electric resistance method or a laser diffraction/scattering method, as described above.

[Polyorganosilsesquioxane]

In the present invention, the polyorganosilsesquioxane present on the surfaces of the spherical elastomer particles is a resin-like solid material in which units represented by $R^7SiO_{3/2}$ are crosslinked to form a three-dimensional network.

$R^7$ in the above formula is an unsubstituted or substituted monovalent hydrocarbon group having 1 to 20 carbon atoms. Examples of $R^7$ include: alkyl groups such as a methyl group, an ethyl group, a propyl group, a butyl group, a pentyl group, a hexyl group, a heptyl group, an octyl group, a decyl group, an undecyl group, a dodecyl group, a tetradecyl group, a pentadecyl group, a hexadecyl group, a heptadecyl group, an octadecyl group, a nonadecyl group, and an icosyl group; alkenyl groups such as a vinyl group and an allyl group; aryl groups such as a phenyl group, a tolyl group, and a naphthyl group; aralkyl groups such as a benzyl group and a phenethyl group; cycloalkyl groups such as a cyclopentyl group, a cyclohexyl group, and a cycloheptyl group; and hydrocarbon groups obtained by substituting some or all of hydrogen atoms bonded to carbon atoms in any of the above groups with atoms such as halogen atoms (fluorine atoms, chlorine atoms, bromine atoms, or iodine atoms) and/or substituents such as amino groups, acryloyloxy groups, methacryloyloxy groups, epoxy groups, glycidoxy groups, mercapto groups, or carboxyl groups.

To allow the polyorganosilsesquioxane to adhere to the surfaces of the spherical elastomer particles using a method described later, it is preferable that at least 50% by mole of the $R^7$s are methyl groups, vinyl groups, or phenyl groups, and it is more preferable that at least 80% by mole of the $R^7$s are methyl groups, vinyl groups, or phenyl groups. It is still more preferable that at least 90% by mole of the $R^7$s are methyl groups, vinyl groups, or phenyl groups.

The polyorganosilsesquioxane may include not only the $R^7SiO_{3/2}$ units but also at least one type of units selected from $R^7{}_2SiO_{2/2}$ units, $R^7{}_3SiO_{1/2}$ units, and $SiO_{4/2}$ units, so long as the characteristics of the elastomer composite particles to be obtained such as non-cohesiveness and dispersiveness, textures such as a dry feel and a smooth feel, and a soft feel are not impaired.

In the above polyorganosilsesquioxane, the content of the $R^7SiO_{3/2}$ units with respect to the total amount of siloxane units is preferably 70 to 100% by mole and more preferably 80 to 100% by mole.

[Silica]

In the present invention, silica present on the surfaces of the spherical elastomer particles is an inorganic solid material in which units represented by $R^8SiO_{4/2}$ are crosslinked to form a three-dimensional network.
$R^8$ in the above formula is an unsubstituted or substituted monovalent hydrocarbon group having 1 to 6 carbon atoms. Examples of $R^8$ include a methyl group, an ethyl group, a propyl group, a butyl group, a pentyl group, and a hexyl group.
Silica is obtained by a hydrolysis-polycondensation reaction of tetraalkoxysilane and is composed mainly of $SiO_2$ units.
Silica may not only contain structures composed only of $SiO_2$ units but also contain alkoxy groups derived from tetraalkoxysilane used as the raw material and silanol groups not subjected to the condensation reaction.
In the present invention, the shape of the polyorganosilsesquioxane or silica is preferably spherical.
The particle diameters of the polyorganosilsesquioxane and silica are each preferably 10 to 500 nm and more preferably 20 to 200 nm.
If the particle diameters of the polyorganosilsesquioxane and silica are each smaller than 10 nm, the ability of the elastomer composite particles to be obtained to scatter light may deteriorate. If the particle diameters of the polyorganosilsesquioxane and silica are each larger than 500 nm, the elastomer composite particles to be obtained lack a soft feel, and their ability to scatter light may deteriorate.
The polyorganosilsesquioxane or silica may adhere to only part of the surfaces of the spherical elastomer particles or may adhere to the entire particle surfaces, i.e., may coat the entire particle surfaces. It is preferable that the entire surfaces of the spherical elastomer particles are coated, with substantially no regions remaining uncoated.
The particle diameters and shapes of the polyorganosilsesquioxane and silica and their adhesion densities on the surfaces of the spherical elastomer particles can be determined by observing the surfaces of the obtained elastomer composite particles using an electron microscope. The diameter of each of the polyorganosilsesquioxane and silica refers to a value obtained by randomly selecting 50 particles from an electron microscope photograph that depicts the surfaces of the obtained elastomer composite particles, measuring their particle diameters, and averaging their values to determine the averaged particle diameter.
In the elastomer composite particles, the amount of the polyorganosilsesquioxane or silica adhering to the surfaces of the spherical elastomer particles is preferably 0.2 to 200 parts by mass and more preferably 0.5 to 50 parts by mass based on 100 parts by mass of the spherical elastomer particles.
If the amount of the polyorganosilsesquioxane or silica is less than the lower limit, the cohesiveness is high, and the dispersibility is low. Moreover, the ability to scatter light may deteriorate, and the elastomer composite particles may lack a dry feel. If the amount of the polyorganosiloxane or silica is more than the upper limit, the elastomer composite particles may lack their soft feel.

[Method for producing elastomer composite particles]

The elastomer composite particles can be produced, for example, by a method including the step iv) of:
iv) adding component (I) shown below to a liquid phase containing components (E), (F), (G), and (H) defined as:

(E) spherical elastomer particles,
(F) an alkaline material,
(G) at least one selected from a cationic surfactant and a cationic water-soluble macromolecular compound,
(H) water, and
(I) trialkoxysilane or tetraalkoxysilane,
to subject the component (I) to a hydrolysis-polymerization reaction.

Step iv)

In the step iv), the component (I) described below is added to the liquid phase containing the components (E), (F), (G), and (H) described below to subject the component (I) to a hydrolysis-polymerization reaction.
The components used in the step iv) are as follows.
The component (E) is spherical elastomer particles, and the spherical elastomer particles obtained through the steps i) to ii) or i) to iii) described above are used as the component (E).
When the component (B) used in the step i) is an aqueous phase component, the water dispersion of the spherical elastomer particles obtained through the steps i) and ii) without performing the step iii) may be used as a mixture of the components (E) and (H) in the step iv).
The amount of the spherical elastomer particles (E) is preferably in the range of 1.0 to 150 parts by mass and more

preferably in the range of 3.0 to 70 parts by mass based on 100 parts by mass of water (H) in the liquid phase containing (E) to (H). If the amount of the component (E) is less than the lower limit, the production efficiency of the target elastomer composite particles may deteriorate. If the amount of the component (E) is larger than the upper limit, it is difficult to coat the surfaces of the spherical elastomer particles with polyorganosilsesquioxane or silica, and aggregation and fusion of the particles may occur, which is undesirable.

The alkaline material (F) functions as a catalyst for the hydrolysis-polycondensation reaction of trialkoxysilane or tetraalkoxysilane. One alkaline material may be used alone, or an appropriate combination of two or more alkaline materials may be used.

No particular limitation is imposed on the alkaline material. Examples of the alkaline material that can be used include: alkali metal hydroxides such as potassium hydroxide, sodium hydroxide, and lithium hydroxide; alkaline-earth metal hydroxides such as calcium hydroxide and barium hydroxide; alkali metal carbonates such as potassium carbonate and sodium carbonate; ammonia; tetraalkylammonium hydroxides such as tetramethylammonium hydroxide and tetraethylammonium hydroxide; and amines such as monomethylamine, monoethylamine, monopropylamine, monobutylamine, monopentylamine, dimethylamine, diethylamine, trimethylamine, triethanolamine, and ethylenediamine. One of these alkaline materials may be used alone, or an appropriate combination of two or more may be used.

The alkaline material is most preferably ammonia because it can be easily removed by volatilization from the obtained elastomer composite particle powder. The ammonia used may be any of various commercial aqueous ammonia solutions with different concentrations.

The amount of the alkaline material (F) added is adjusted such that the pH of the liquid phase containing the components (E) to (H) at 25°C is preferably 9.0 to 13.0 and more preferably 10.0 to 12.0. By adding the alkaline material (F) in such an amount that the pH is 9.0 to 13.0, the hydrolysis-polycondensation reaction of trialkoxysilane or tetraalkoxysilane proceeds sufficiently, and the surfaces of the spherical elastomer particles are coated sufficiently with the polyorganosilsesquioxane or silica.

The cationic surfactant and the cationic water-soluble macromolecular compound both used as the component (G) have the function of facilitating the condensation reaction of the hydrolyzed trialkoxysilane or tetraalkoxysilane to generate the resin or silica. They also have the function of allowing the generated resin or silica to adhere to the surfaces of the spherical elastomer particles.

One cationic surfactant or one cationic water-soluble macromolecular compound may be used alone, or an appropriate combination of two or more may be used.

Examples of the cationic surfactant include the same cationic surfactants as those exemplified for the component (C).

Examples of the cationic water-soluble macromolecular compound include polymers of dimethyldiallylammonium chloride, polymers of vinylimidazoline, polymers of methylvinylimidazolium chloride, polymers of ethyl acrylate trimethylammonium chloride, polymers of ethyl methacrylate trimethylammonium chloride, polymers of acrylamidopropyltrimethylammonium chloride, polymers of methacrylamidopropyltrimethylammonium chloride, epichlorohydrin/dimethylamine polymers, polymers of ethyleneimine, quaternized products of ethyleneimine polymers, polymers of allylamine hydrochloride, polylysine, cationic starch, cationic cellulose, chitosan, their copolymers with a monomer having a nonionic group or an anionic group, and derivatives thereof.

The component (G) is preferably an alkyltrimethylammonium salt of a cationic surfactant and more preferably lauryltrimethylammonium salt or cetyltrimethylammonium salt.

The amount of the cationic surfactant and cationic water-soluble macromolecular compound added is preferably 0.01 to 2.0 parts by mass and more preferably in the range of 0.1 to 1.0 parts by mass based on 100 parts by mass of water in the liquid phase containing (E) to (H). If the amount of the component (G) added is less than the lower limit, some of the polyorganosilsesquioxane or silica generated may fail to cover the surfaces of the spherical elastomer particles. Similarly, if the amount of the component (G) added is larger than the upper limit, some of the polyorganosilsesquioxane or silica generated may fail to cover the surfaces of the spherical elastomer particles.

No particular limitation is imposed on the water (H). For example, purified water, ion exchanged water, pure water, etc. may be used. When the component (B) is an aqueous phase component and the water dispersion of the spherical elastomer particles obtained through the step ii) without performing the step iii) is used for the step iv), the water in the water dispersion and water optionally added are included in the water (H).

No particular limitation is imposed on the trialkoxysilane and tetraalkoxysilane both used as the component (I), and a well-known material may be used. From the viewpoint of reactivity, methyltrimethoxysilane, methyltriethoxysilane, tetramethoxysilane, and tetraethoxysilane are preferred, and methyltrimethoxysilane and tetramethoxysilane are more preferred

In the tetraalkoxysilane used, part or all of the alkoxy groups may be hydrolyzed, or part of the alkoxy groups may be condensed.

The amount of the trialkoxysilane added is such that the amount of polyorganosilsesquioxane is preferably 1.0 to 50 parts by mass and more preferably 2 to 25 parts by mass based on 100 parts by mass of the spherical elastomer

particles (E).
The amount of the tetraalkoxysilane added is such that the amount of silica is preferably 0.5 to 200 parts by mass and more preferably 1.0 to 50 parts by mass based on 100 parts by mass of the spherical elastomer particles (E).

Hydrolysis-polycondensation reaction

The trialkoxysilane or tetraalkoxysilane (I) is added to the liquid phase containing (E) to (H), and the mixture is subjected to a hydrolysis-polycondensation reaction.
Specifically, the spherical elastomer particles (E) are dispersed in the water (H), and the alkaline material (F) and at least one selected from the cationic surfactant and the cationic water-soluble macromolecular compound (G) are dissolved in the water dispersion. Then the trialkoxysilane or tetraalkoxysilane (I) is added to the water dispersion, and the mixture is subjected to hydrolysis and polycondensation. As a result of the hydrolysis and polycondensation, polyorganosilsesquioxane or silica, which is a condensation product of the trialkoxysilane or tetraalkoxysilane, adheres to the surfaces of the spherical elastomer particles, and the surfaces of the spherical elastomer particles are coated with the polyorganosilsesquioxane or silica.
Preferably, the trialkoxysilane or tetraalkoxysilane is added under stirring using a conventional stirrer such as propeller blades or flat blades. The trialkoxysilane or tetraalkoxysilane may be added all at once, but preferably added gradually over time. The addition time is preferably in the range of 1 minute to 6 hours and more preferably 10 minutes to 3 hours.
The temperature of the liquid phase in this case is preferably 0 to 60°C and more preferably in the range of 0 to 40°C. When the temperature is within the above range, the polyorganosilsesquioxane or silica can adhere effectively to the surfaces of the spherical elastomer particles in the liquid phase to coat the surfaces.
After the addition of the trialkoxysilane or tetraalkoxysilane, the stirring is continued until the hydrolysis-polycondensation reaction is completed. To complete the hydrolysis-polycondensation reaction, the reaction may be performed at room temperature or may be performed at an increased temperature of about 40 to 100°C under heating, and an alkaline material may be optionally added.

Dehydration step - pulverization step

After the hydrolysis-polycondensation reaction in the step iv), water in the resulting water dispersion of the elastomer composite particles is optionally removed. To remove water, the water dispersion after the reaction is heated at normal pressure or under reduced pressure.
Specifically, for example, a method of heat-drying at normal pressure or under reduced pressure, a method of spraying the dispersion into a heated airflow to perform heat-drying (spray drying), a method of using a circulating heating medium to perform heat drying, or a freeze-drying method of freezing the dispersion and then reducing the pressure to remove the dispersion medium may be performed to thereby obtain the elastomer composite particles.
As a pretreatment step prior to the above procedure, the dispersion may be concentrated by thermal dehydration, filtration, centrifugal separation, decantation, etc. The water dispersion may be optionally washed with water or alcohol.
When the powder of the elastomer composite particles obtained by removing water from the water dispersion after the reaction is an aggregated powder, the aggregated powder may be pulverized using a pulverizer such as a jet mill, ball mill, or a hammer mill.

III. Method for producing cosmetic using spherical elastomer particles and elastomer composite particles

The cosmetic of the present invention can be produced using at least one type of particles selected from the spherical elastomer particles (i) and the elastomer composite particles (ii) produced by the methods described above.
More specifically, the spherical elastomer particles and/or the elastomer composite particles are used, and various cosmetic components described later are further used. These components may be mixed and subjected to emulsification etc. to thereby produce the desired cosmetic.

IV. Cosmetic containing spherical elastomer particles and elastomer composite particles

The cosmetic of the present invention is characterized by containing at least one type of particles selected from the spherical elastomer particles (i) and the elastomer composite particles (ii).
No particular limitation is imposed on the ratio of the elastomer composite particles added, and the ratio is appropriately selected according to the type of formulation.
The present invention is applicable to various cosmetics and is particularly preferably applied to cosmetics externally applied to the skin such as skincare cosmetics, makeup cosmetics, antiperspirant cosmetics, and suncare

cosmetics, cosmetics externally applied to the hair such as haircare cosmetics, and nailcare cosmetics.

No limitation is imposed on the amount of the spherical elastomer particles and elastomer composite particles in the present invention added to the cosmetic of the present invention.

Examples of the skincare cosmetic include beauty lotions, milky lotions, creams, cleansing creams, face packs, oil liquids, massage lotions, cosmetic liquids, cosmetic oils, detergents, deodorants, hand creams, lip creams, and wrinkle concealers. In the skincare cosmetic, the ratio of the total mass of the spherical elastomer particles (i) and the elastomer composite particles (ii) to the total mass of its components is, for example, preferably 0.1 to 99% by mass and more preferably 1 to 70% by mass.

Examples of the makeup cosmetic include makeup bases, foundations, concealers, face powders, blushes, eye colors, eyeshadows, mascaras, eye liners, eye brows, and lipsticks. In the makeup cosmetic, the ratio of the total mass of the spherical elastomer particles (i) and the elastomer composite particles (ii) to the total mass of its components is, for example, preferably 0.1 to 100% by mass and more preferably 1 to 90% by mass.

Examples of the antiperspirant cosmetic include antiperspirants of the roll-on type, the cream type, the solution type, and the stick type. In the antiperspirant cosmetic, the ratio of the total mass of the spherical elastomer particles (i) and the elastomer composite particles (ii) to the total mass of its components is, for example, preferably 0.1 to 80% by mass and more preferably 1 to 70% by mass.

Examples of the suncare cosmetic include sunscreen oils, sunscreen milky lotions, and sunscreen creams. In the suncare cosmetic, the ratio of the total mass of the spherical elastomer particles (i) and the elastomer composite particles (ii) to the total mass of its components is, for example, preferably 0.1 to 90% by mass and more preferably 0.1 to 80% by mass.

Examples of the haircare cosmetic include shampoos, hair rinses, hair treatments, and hair styling products. In the haircare cosmetic, the ratio of the total mass of the spherical elastomer particles (i) and the elastomer composite particles (ii) to the total mass of its components is, for example, preferably 0.1 to 80% by mass and more preferably 1 to 70% by mass.

The cosmetic of the present invention may be in the form of a powder, an oily liquid, a water-in-oil emulsion, an oil-in-water emulsion, a nonaqueous emulsion, or a multiple emulsion such as a W/O/W emulsion or an O/W/O emulsion. The cosmetic of the present invention may be in a physical state selected from various physical states such as a liquid, a milky lotion, a cream, a solid, a paste, a gel, a powder, a pressed state, a multilayer state, a mousse, a spray, a stick, and a pencil.

The cosmetic of the present invention may contain various components used for ordinary cosmetics so long as the effects of the present invention are not impaired. For example, the cosmetic may contain (1) an oily agent, (2) a water-based component, (3) a surfactant, (4) a powder other than the spherical elastomer particles and the elastomer composite particles (5) a composition of a crosslinked organopolysiloxane and an oily agent in a liquid state at room temperature, (6) a film-forming agent, (7) an ultraviolet absorbing-scattering agent, and (8) additional additives. For each of them, one type may be used alone, or an appropriate combination of two or more types may be used.

(1) Oily agent

The oily agent may be volatile or nonvolatile and may be any of a solid, a semi-solid, and a liquid at room temperature (25°C). Examples of the oily agent include silicone oils, silicone waxes, natural animal and vegetable oils and fats, semi-synthetic oils and fats, hydrocarbon oils, higher alcohols, fatty acids, ester oils, fluorine-based oily agents, and ultraviolet absorbers.

Silicone oil

Examples of the silicone oil include: alkyl-modified silicones such as Dimethicone (INCI), Trisiloxane (INCI), Methyl Trimethicone (INCI), Ethyl Trisiloxane (INCI), Ethyl Methicone (INCI), and Hexyl Dimethicone (INCI); long-chain alkyl-modified silicones such as Caprylyl Methicone (INCI); low to high viscosity linear or branched organopolysiloxanes such as Phenyl Trimethicone (INCI), Diphenyl Dimethicone (INCI), Diphenylsiloxy Phenyl Trimethicone (INCI), Tetraphenyl Dimethyl Disiloxane (INCI), and methylhydrogenpolysiloxane; cyclic organopolysiloxanes such as Cyclotetrasiloxane (INCI), Cyclopentasiloxane (INCI), and Cyclohexasiloxane (INCI); amino-modified organopolysiloxanes such as Amodimethicone (INCI) and Aminopropyl Dimethicone (INCI); pyrrolidone-modified organopolysiloxanes such as PCA Dimethicone (INCI); pyrrolidone carboxylic acid-modified organopolysiloxane; silicone rubbers such as high-polymerization degree gum-like dimethylpolysiloxanes, gum-like amino-modified organopolysiloxanes, and gum-like dimethylsiloxane-methylphenylsiloxane copolymers; low-viscosity organopolysiloxane solutions of silicone gum or rubber; amino acid-modified silicones; fluorine-modified silicones; silicone resins; and dissolution products of silicone resins.

Commercial examples of the silicone oil include KF-96L-1cs, KF-96L-1.5cs, KF-96L-2cs, KF-96A-6cs, KF-4422, KF-4418, KF-54, KF-54HV, KF-56A, and KF-995 manufactured by Shin-Etsu Chemical Co., Ltd.

Solid oily component

In the present invention, to prepare the cosmetic in a solid form, it is preferable to add an oily component that is solid at 25°C.

The oily component that is solid at 25°C has a melting point of preferably 40°C or higher and more preferably 60 to 110°C. Examples of such an oily component include waxes, hydrocarbons, esters, higher alcohols, and higher fatty acids. No particular limitation is imposed on the oily component so long as it can be added to ordinary cosmetics. Specific examples of the oily component include: vegetable waxes such as carnauba wax (INCI: Copernicia Cerifera (Carnauba) Wax), sugarcane wax, candelilla wax (INCI: Euphorbia Cerifera (Candelilla) Wax), purified candelilla wax, rice wax, Japan tallow, jojoba wax, kapok wax, rice bran wax, myrica cerifera fruit wax, shea butter, cacao butter, Japan wax (INCI: Rhus succedanea Fruit Wax), montan wax (INCI: Montan Wax), and hydrogenated castor oil isostearate; animal waxes such as beeswax, beef tallow, beef bone fat, lard (INCI: Lard), horse fat (INCI: Horse Fat), mutton tallow, lanolin (INCI: Lanolin), insect wax, shellac wax, and spermaceti; semisynthetic waxes such as lanolin esters, lanolin fatty acid esters, and beeswax acid esters; hydrogenated oils such as hydrogenated castor oil and hydrogenated coconut oil; hydrocarbon-based waxes such as solid paraffin, polyethylene, ceresin, ozokerite, and microcrystalline wax; wax esters such as synthetic beeswax; amino acid stearyl alcohols such as dioctyldodecyl lauroyl glutamate, dioctyldodecyl lauroyl glutamate, and dioctyldodecyl lauroyl glutamate; fatty acids such as stearic acid and behenic acid; silicone waxes such as acrylic silicone resins that are acrylic-silicone graft or block copolymers (acrylic-silicone graft copolymers: KP-561P, KP-562P, etc., manufactured by Shin-Etsu Chemical Co., Ltd.); and derivatives thereof. One or two or more selected from these are preferred.

Natural animal and vegetable oily agents and semi-synthetic oily agents

Examples of the natural animal and vegetable oily agents and semi-synthetic oily agents include: natural vegetable oils such as avocado oil (:label name (INCI: Persea Gratissima (Avocado) Oil)), linseed oil (:label name (INCI: Linum Usitatissimum (Linseed) Seed Oil)), almond oil (:label name (INCI: Prunus Amygdalus Dulcis (Sweet Almond) Oil)), perilla oil (label name), olive oil (:label name (INCI: Olea Europaea (Olive) Fruit Oil)), torreya californica oil (:label name (INCI: Torreya Californica (California Nutmeg) Oil)), citronella oil (:label name (INCI: Cymbopogon Nardus (Citronella) Oil)), torreya nucifera seed oil (:label name (INCI: Torreya Nucifera Seed Oil)), apricot kernel oil (:label name (INCI: Kyounin Yu)), wheat germ oil (:label name (INCI: Triticum Vulgare (Wheat) Germ Oil)), sesame oil (:label name (INCI: Sesamum Indicum (Sesame) Seed Oil)), wheat germ oil (:label name (INCI: Triticum Vulgare (Wheat) Germ Oil)), rice germ oil (:label name (INCI: Oryza Sativa (Rice) Germ Oil)), rice bran oil (:label name (INCI: Oryza Sativa (Rice) Bran Oil)), sasanqua oil (:label name (INCI: Camellia Kissi Seed Oil)), safflower oil (:label name (INCI: Carthamus Tinctorius (Safflower) Seed Oil)), soybean oil (:label name (INCI: Glycine Soja (Soybean)Oil)), tea seed oil (:label name (INCI: Camellia Sinensis Seed Oil)), camellia oil (:label name (INCI: Camellia Japonica Seed Oil)), evening primrose oil (:label name (INCI: Oenothera Biennis (Evening Primrose) Oil)), rape oil (label name), germ oils such as corn germ oil (:label name (INCI: Zea Mays (Corn) Germ Oil)) and wheat germ oil (:label name (INCI: Triticum Vulgare (Wheat) Germ Oil)), persic oil (label name ), palm oil (:label name (INCI: Elaeis Guineensis (Palm) Oil)), palm kernel oil (:label name (INCI: Elaeis Guineensis (Palm) Kernel Oil)), castor oil (:label name (INCI: Ricinus Communis (Castor) Seed Oil)), sunflower oil (:label name (INCI: Helianthus Annuus (Sunflower) Seed Oil)), grape seed oil (:label name (INCI: Vitis Vinifera (Grape) Seed Oil)), jojoba seed oil (:label name (INCI: Simmondsia Chinensis (Jojoba) Seed Oil)), macadamia seed oil (:label name (INCI: Macadamia Ternifolia Seed Oil)), meadowfoam oil (:label name (INCI: Limnanthes Alba (Meadowfoam) Seed Oil)), cottonseed oil (:label name (INCI: Gossypium Herbaceum (Cotton) Seed Oil)), coconut oil (:label name (INCI: Cocos Nucifera (Coconut) Oil)), and peanut oil (:label name (INCI: Arachis Hypogaea (Peanut) Oil)); natural animal oils such as shark liver oil (:label name (INCI: Shark Liver Oil)), cod liver oil (:label name (INCI: Cod Liver Oil)), fish liver oil (:label name (INCI: Fish Liver Oil)), turtle oil (:label name (INCI: Turtle Oil)), mink oil (:label name (INCI: Mink Oil)), and egg yolk oil (:label name (INCI: Egg Oil)); and semi-synthetic oils and fats such as hydrogenated coconut oil (:label name (INCI: Hydrogenated Coconut Oil)) and lanolin oil (:label name (INCI: Lanolin Oil)).

Hydrocarbon oil

Examples of the hydrocarbon oil include straight and branched hydrocarbon oils, and the hydrocarbon oil may be a volatile hydrocarbon oil or a nonvolatile hydrocarbon oil. Specific examples of the hydrocarbon oil include Olefin Oligomers (INCI), isoparaffins such as (C13, 14) Isoparaffin (INCI), Isododecane (INCI), Undecane (INCI), Dodecane (INCI), Isohexadecane (INCI), hydrogenated polyisobutene (:label name (INCI: Hydrogenated Polyisobutene)), Squalane (INCI), Mineral Oil (INCI), and alkanes such as Coconut Alkanes (INCI) and (C13-15) Alkane (INCI).

Higher alcohols

Examples of the higher alcohols include: linear saturated alcohols having 6 or more carbon atoms such as Lauryl Alcohol (INCI), Hexyldecanol (INCI), Oleyl Alcohol (INCI), Isostearyl Alcohol (INCI), Octyldodecanol (INCI), Decyltetradecanol (INCI), Myristyl Alcohol (INCI), Cetyl Alcohol (INCI), Stearyl Alcohol (INCI), and Behenyl Alcohol (INCI); and Batyl Alcohol (INCI). Other examples include sterols such as Cholesterol (INCI), sitosterol (:label name (INCI: Beta-Sitosterol)), Phytosterols (INCI), and Lanosterol (INCI).

Ester oil

Examples of the ester oil include diisobutyl adipate (:label name (INCI: Diisobutyl Adipate)), dihexyldecyl adipate (label name), diheptylundecyl adipate (:label name (INCI: Diheptylundecyl Adipate)), n-alkylglycol monoisostearates such as isostearyl isostearate (:label name (INCI: Isostearyl Isostearate)), isocetyl isostearate (:label name (INCI: Isocetyl Isostearate)), trimethylolpropane triisostearate (:label name (INCI: Trimethylolpropane Triisostearate)), glycol diethylhexanoate (:label name (INCI: Glycol Diethylhexanoate)), cetyl ethylhexanoate (:label name (INCI: Cetyl Ethylhexanoate)), trimethylolpropane triethylhexanoate (:label name (INCI: Trimethylolpropane Triethylhexanoate)), pentaerythrityl tetraethylhexanoate (:label name (INCI: Pentaerythrityl Tetraethylhexanoate)), cetyl octanoate (:label name (INCI: Cetyl Ethylhexanoate)), octyldodecyl esters such as octyldodecyl stearoyl stearate (:label name (INCI: Octyldodecyl Stearoyl Stearate)), oleyl oleate (:label name (INCI: Oleyl Oleate)), octyldodecyl oleate (:label name (INCI: Octyldodecyl Oleate)), decyl oleate (:label name (INCI: Decyl Oleate)), neopentyl glycol dioctanoate (:label name (INCI: Neopentyl Glycol Diethylhexanoate)), neopentyl glycol dicaprate (:label name (INCI: Neopentyl Glycol Dicaprate)), diisostearyl malate (:label name (INCI: Diisostearyl Malate)), triethyl citrate (:label name (INCI: Triethyl Citrate)), diethylhexyl succinate (:label name (INCI: Diethylhexyl Succinate)), amyl acetate (:label name (INCI: Amyl Acetate)), ethyl acetate (:label name (INCI: Ethyl Acetate)), butyl acetate (:label name (INCI: Butyl Acetate)), isocetyl stearate (:label name (INCI: Isocetyl Stearate)), butyl stearate (:label name (INCI: Butyl Stearate)), diisopropyl sebacate (:label name (INCI: Diisopropyl Sebacate)), diethylhexyl sebacate (:label name (INCI: Diethylhexyl Sebacate)), cetyl lactate (:label name (INCI: Cetyl Lactate)), myristyl lactate (:label name (INCI: Myristyl Lactate)), isononyl isononanoate (:label name (INCI: Isononyl Isononanoate)), isotridecyl isononanoate (:label name (INCI: Isotridecyl Isononanoate)), palmitates such as isopropyl palmitate (:label name (INCI: Isopropyl Palmitate)), ethylhexyl palmitate (:label name (INCI: Ethylhexyl Isopalmitate)), and hexyldecyl palmitate (:label name (INCI: Isocetyl Palmitate, Hexyldecyl Palmitate)), cholesteryl (:label name (INCI: Cholesteryl Hydroxystearate)), myristates such as isopropyl myristate (:label name (INCI: Isopropyl Myristate)), octyldodecyl myristate (label name (INCI: Octyldodecyl Myristate)), and myristyl myristate (:label name (INCI: Myristyl Myristate)), ethylhexyl laurate (:label name (INCI: Ethylhexyl Laurate)), hexyl laurate (:label name (INCI: Hexyl Laurate)), dioctyldodecyl lauroyl glutamate (:label name (INCI: Dioctyldodecyl Lauroyl Glutamate)), isopropyl lauroyl sarcosinate (:label name (INCI: Isopropyl Lauroyl Sarcosinate)), and coco-alkyl (caprylate/caprate) (:label name (INCI: Coco-Caprylate/Caprate)).

The ester oils also include glyceride oils, and examples of the glyceride oils include Triethylhexanoin (INCI), (caprylic/capric) triglyceride (:label name (INCI: Caprylic/Capric Triglyceride)), Cocoglyceride (INCI), (caprylic/capric/-succinic) triglyceride (:label name (INCI: Caprylic/Capric/Succinic Triglyceride)), and (caprylic/capric) glycerides (:label name (INCI: Caprylic/Capric Glycerides)).

Fluorine-based oily agent

Examples of the fluorine-based oily agent include Perfluorodecalin (INCI), Perfluorononyl Dimethicone (INCI), and Perfluoromethylcyclopentane (INCI).

Ultraviolet absorber

Examples of the ultraviolet absorber include oxybenzone-1 (:label name (INCI: Benzophenone-1)), oxybenzone-2 (:label name (INCI: Benzophenone-2)), oxybenzone-3 (:label name (INCI: Benzophenone-3)), oxybenzone-4 (:label name (INCI: Benzophenone-4)), oxybenzone-5 (:label name (INCI: Benzophenone-5)), oxybenzone-6 (:label name (INCI: Benzophenone-6)), oxybenzone-9 (:label name (INCI: Benzophenone-9)), Homosalate (INCI), Octocrylene (INCI), t-butyl methoxydibenzoylmethane (:label name (INCI: Butyl Methoxydibenzoylmethane)), ethylhexyl salicylate (:label name (INCI: Ethylhexyl Salicylate)), diethylamino hydroxybenzoyl hexyl benzoate (:label name (INCI: Diethylamino Hydroxybenzoyl Hexyl Benzoate)), Polysilicone-15 (INCI), ethylhexyl dimethoxybenzylidene dioxoimidazolidine propionate (:label name (INCI: Ethylhexyl Dimethoxybenzylidene Dioxoimidazolidine Propionate)), terephthalylidene dicamphor sulfonic acid (:label name (INCI: Terephthalylidene Dicamphor Sulfonic Acid)), Ethylhexyl Triazone (INCI), isopentyl trimethoxycinnamate trisiloxane (:label name (INCI: Isopentyl Trimethoxycinnamate Trisiloxane)), Drometrizole Trisiloxane (INCI), ethylhexyl dimethyl PABA (:label name (INCI: Ethylhexyl Dimethyl PABA)), isopropyl methoxycinnamate

(:label name (INCI: Isopropyl Methoxycinnamate)), ethylhexyl methoxycinnamate (:label name (INCI: Ethylhexyl Methoxycinnamate)), Bis-Ethylhexyloxyphenol Methoxyphenyl Triazine (INCI), phenylbenzimidazole sulfonic acid (:label name (INCI: Phenylbenzimidazole Sulfonic Acid)), Methylene Bis-Benzotriazolyl Tetramethylbutylphenol (INCI), glyceryl ethylhexanoate dimethoxycinnamate (:label name (INCI: Glyceryl Ethylhexanoate Dimethoxycinnamate)), Glyceryl PABA (INCI), diisopropyl methyl cinnamate (:label name (INCI: Diisopropyl Methyl Cinnamate)), Cinoxate (INCI), and ethylhexyl dimethoxybenzylidene dioxoimidazolidine propionate (:label name (INCI: Ethylhexyl Dimethoxybenzylidene Dioxoimidazolidine Propionate)). A UVA absorber (such as diethylamino hydroxybenzoyl hexyl benzoate) and a UVB absorber (such as ethylhexyl methoxycinnamate) can be used in combination. They may be combined freely.

(2) Water-based component

No particular limitation is imposed on the water-based component, so long as it can be added to ordinary cosmetics. Specific examples of the water-based component include water, lower alcohols having preferably 2 to 5 carbon atoms such as ethanol (:label name (INCI: Alcohol)) and isopropanol (:label name (INCI: Isopropyl Alcohol)), and sugar alcohols such as Sorbitol (INCI), Maltose (INCI), and Xylitol (INCI). Other examples include: polyhydric alcohols such as BG (:label name (INCI: Butylene Glycol)), PG (:label name (INCI: Propylene Glycol)), DPG (:label name (INCI: Dipropylene Glycol)), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; and moisturizing agents such as Glucose (INCI), Glyceryl Glucoside (INCI), Betaine (INCI), sodium chondroitin sulfate (:label name (INCI: Sodium Chondroitin Sulfate)), PCA-Na (:label name (INCI: Sodium PCA)), Methyl Gluceth-10 (INCI), Methyl Gluceth-20 (INCI), hyaluronic acid, egg yolk lecithin, soybean lecithin, phosphatidyl choline, phosphatidyl ethanolamine, phosphatidyl serine, phosphatidyl glycerol, phosphatidyl inositol, and sphingophospholipid.

(3) Surfactant

No particular limitation is imposed on the surfactant, and any surfactant from among nonionic, anionic, cationic, and amphoteric surfactants may be used, so long as it is used for ordinary cosmetics. Among these surfactants, one or two or more selected from non-crosslinked and crosslinked silicone surfactants are preferred because a stable cosmetic can be obtained. In any case, the amount of the surfactant added is preferably 0.1 to 20% by mass based on the total mass of the cosmetic. When the amount of the surfactant is 0.1% or more, its dispersing and emulsifying function is sufficient. When the amount is 20% by mass or less, the cosmetic exhibits no stickiness upon application, which is desirable.

No particular limitation is imposed on the HLB value of the surfactant. For the purpose of maintaining the water resistance of the cosmetic, the HLB value of the surfactant is preferably 2.0 to 14.5.

The non-crosslinked silicone surfactant is a surfactant in which some of the methyl groups on the linear or branched silicone main chain are replaced with hydrophilic groups such as polyethylene glycol or polyglycerin. Specifically, the non-crosslinked silicone surfactant is preferably a linear or branched polyoxyethylene-modified organopolysiloxane, a linear or branched polyoxyethylene polyoxypropylene-modified organopolysiloxane, a linear or branched polyoxyethylene/alkyl co-modified organopolysiloxane, a linear or branched polyoxyethylene polyoxypropylene/alkyl co-modified organopolysiloxane, a linear or branched polyglycerin-modified organopolysiloxane, a linear or branched polyglycerin/alkyl co-modified organopolysiloxane, or a linear or branched pyrrolidone-modified organopolysiloxane.

Examples of the non-crosslinked silicone surfactant include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), Lauryl Polyglyceryl-3 Polydimethylsiloxyethyl Dimethicone (INCI), and Bis-Butyldimethicone Polyglyceryl-3 (INCI).

Commercial examples thereof include KF-6011, KF-6011P, KF-6012, KF-6015, KF-6017, KF-6043, KF-6028, KF-6038, KF-6048, KF-6100, KF-6104, KF-6105, KF-6106, and KF-6115 manufactured by Shin-Etsu Chemical Co., Ltd.

Examples of the crosslinked silicone surfactant include: crosslinked polyether-modified silicones such as (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), and (PEG-15/Lauryl Polydimethylsiloxyethyl Dimethicone) Crosspolymer (INCI); and crosslinked polyglycerin-modified silicones such as (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI).

When the crosslinked silicone surfactant is used, it is preferable that the crosslinked silicone surfactant in a composition of the surfactant and an oily agent in a liquid state at room temperature swells with the liquid oily agent so as to contain the liquid oil in an amount more than or equal to its own weight.

The liquid oily agent used may be any of the liquid silicone oils, hydrocarbon oils, ester oils, natural animal and

vegetable oils and fats, semi-synthetic oils, and fluorine-based oils exemplified for the optional oily agent (1). Examples of the liquid oily agent include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (:label name (INCI: Isotridecyl Isononanoate)), and Squalane (INCI).

Commercial examples of the crosslinked silicone surfactant capable of swelling with a liquid oily agent include KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z manufactured by Shin-Etsu Chemical Co., Ltd.

(4) Powder other than spherical elastomer particles and elastomer composite particles

A powder other than the spherical elastomer particles and the elastomer composite particles may be added. Examples of such powder include a color pigment, an inorganic powder, a metal powder, an organic powder, and an inorganic/organic composite powder. Specific examples are as follows.

Color pigment

No particular limitation is imposed on the color pigment, and any ordinary color pigment used for the purpose of coloring cosmetics can be used. Examples of the color pigment include red iron oxide (:label name (INCI: Iron Oxides)), yellow iron oxide (:label name (INCI: Iron Oxides)), white titanium oxide (:label name (INCI: Titanium Dioxide)), black iron oxide (:label name (INCI: Iron Oxides)), ultramarines (:label name (INCI: Ultramarines)), iron blue (:label name (INCI: Ferric Ferrocyanide, Ferric Ammonium Ferrocyanide)), manganese violet (:label name (INCI: Manganese Violet)), cobalt titanate (:label name (INCI: Cobalt Titanium Oxide)), chromium hydroxide (:label name (INCI: Chromium Hydroxide Green)), chromium oxide (:label name (INCI: Chromium Oxide Greens)), (Al/cobalt) oxide (:label name (INCI: Cobalt Aluminum Oxide)), cobalt titanate (:label name (INCI: Cobalt Titanium Oxide)), sintered product of (titanium/titanium oxide) (:label name (INCI: Titanium/Titanium Dioxide)), (Li/cobalt) titanate (:label name (INCI: Lithium Cobalt Titanate)), cobalt titanate (:label name (INCI: Cobalt Titanium Oxide)), sintered product of (iron oxide/titanium oxide) (:label name ), composites doped with different metals such as iron oxide-doped titanium oxide (:label name (INCI: Iron Oxides, Titanium Dioxide)), titanium nitride (:label name (INCI: Titanium Nitride)), ferrous hydroxide (:label name (INCI: Iron Hydroxide)), inorganic brown pigments such as $\gamma$-iron oxide, inorganic yellow pigments such as yellow ocher, colored pigments such as lakes prepared from tar-based dyes and lakes prepared from natural dyes. Any of the above pigments may be used.

The pigment may have any of various shapes such as spherical, substantially spherical, rod-like, spindle-like, petal-like, strip-like, and irregular shapes. No particular limitation is imposed on the geometrical form of the pigment, so long as it can impart color to the cosmetic.

Inorganic powder

Examples of the inorganic powder include fine particles of zirconium oxide (:label name (INCI: Zirconium Dioxide)), zinc oxide (:label name (INCI: Zinc Oxide)), cerium oxide (:label name (INCI: Cerium Oxide)), magnesium oxide (:label name (INCI: Magnesium Oxide)), barium sulfate (:label name (INCI: Barium Sulfate)), calcium sulfate (:label name (INCI: Calcium Carbonate)), magnesium sulfate (:label name (INCI: Magnesium Sulfate)), calcium carbonate (:label name (INCI: Calcium Carbonate)), magnesium carbonate (:label name (INCI: Magnesium Carbonate)), Talc (INCI), Mica, (INCI), Kaolin (INCI), synthetic fluorphlogopite (:label name (INCI: Synthetic Fluorphlogopite), synthetic ferrous golden mica (label name), biotite (:label name (INCI: Biotite), potassium silicate (:label name (INCI: Potassium Silicate)), Silica (INCI), aluminum silicate (:label name (INCI: Aluminum Silicate)), magnesium silicate (:label name (INCI: Magnesium Silicate)), (aluminum/magnesium) silicate (:label name (INCI: Magnesium Aluminum Silicate)), calcium silicate (:label name (INCI: Calcium Silicate)), (aluminum/calcium/sodium) silicate (:label name (INCI: Aluminum Calcium Sodium Silicate)), (lithium/-magnesium/sodium) silicate (:label name (INCI: Lithium Magnesium Sodium Silicate)), (sodium/magnesium) silicate (:label name (INCI: Sodium Magnesium Silicate)), (calcium/aluminum) borosilicate (:label name (INCI: Calcium Aluminum Borosilicate)), (calcium/sodium) borosilicate (:label name (INCI: Calcium Sodium Borosilicate)), Hydroxyapatite (INCI), Bentonite (INCI), Montmorillonite (INCI), Hectorite (INCI), Zeolite (INCI), Alumina (INCI), aluminum hydroxide (:label name (INCI: Aluminum Hydroxide)), boron nitride (:label name (INCI: Boron Nitride)), and glass (:label name (INCI: Glass)).

Examples of an inorganic pearl coloring pigment include: pearly agents such as Mica (INCI) coated with titanium oxide (:label name (INCI: Titanium Dioxide)) and synthetic fluorphlogopite (:label name (INCI: Synthetic Fluorphlogopite) coated with titanium oxide (:label name (INCI: Titanium Dioxide)); and pearl pigments such as bismuth oxychloride (:label name (INCI: Bismuth Oxychloride)), bismuth oxychloride (:label name (INCI: Bismuth Oxychloride)) coated with titanium oxide (:label name (INCI: Titanium Dioxide)), Talc (INCI) coated with titanium oxide (:label name (INCI: Titanium Dioxide)), fish scale flakes (label name), and coloring mica coated with titanium oxide (:label name (INCI: Titanium Dioxide)). No

particular limitation is imposed on the inorganic pearl coloring pigment, and the inorganic pearl coloring pigment may be untreated or may be subjected to well-known surface treatment commonly used for cosmetics.

Metal powder

Examples of the metal powder include fine metal powders of aluminum (:label name (INCI: Aluminum, Aluminum Powder)), copper (:label name (INCI: Copper Powder)), silver (:label name (INCI: Silver Powder)), and gold (:label name (INCI: Gold)).

Organic powder

Examples of the organic powder include powders of silicone, polyamide, polyacrylic acid-acrylic acid esters, polyester, Polyethylene (INCI), Polypropylene (INCI), Polystyrene (INCI), styrene-acrylic acid copolymers, divinylbenzene-styrene copolymers, polyurethane, vinyl resins, urea resins, melamine resins, benzoguanamine, polymethylbenzoguanamine, tetrafluoroethylene, polymethyl methacrylate, Cellulose (INCI), Silk (INCI), nylon (label name), phenolic resins, epoxy resins, and polycarbonate.

In particular, examples of the silicone include silicone resin particles, Polymethylsilsesquioxane (INCI), silicone rubber powder, silicone resin-coated silicone rubber powder, (Vinyl Dimethicone/Methicone Silsesquioxane) Crosspolymer (INCI), (Diphenyl Dimethicone/Vinyl Diphenyl Dimethicone/Silsesquioxane) Crosspolymer (INCI), Polysilicone-1 Crosspolymer (INCI), and Polysilicone-22 (INCI).

Commercial examples of the silicone powder include KMP-590, KMP-591, KMP-592, KMP-597, KMP-598, KSP-100, KSP-101, KSP-102, KSP-105, KSP-300, KSP-411, KSP-441, KM-9729, and KM-440 manufactured by Shin-Etsu Chemical Co., Ltd.

Metal soap or the like can also be used, and specific examples thereof include powders of zinc stearate (:label name (INCI: Zinc Stearate)), aluminum stearate (:label name (INCI: Aluminum Stearate)), calcium stearate (:label name (INCI: Calcium Stearate)), magnesium stearate (:label name (INCI: Magnesium Stearate)), zinc myristate (:label name (INCI: Zinc Myristate)), magnesium myristate (:label name (INCI: Magnesium Myristate)), (zinc/sodium) cetyl phosphate (:label name (INCI: Sodium Zinc Cetyl Phosphate)), and potassium cetyl phosphate (:label name (INCI: Potassium Cetyl Phosphate)).

Organic pigments etc. may also be used, and specific examples thereof include: tar dyes such as Red No. 3, Red No. 104(1) (:label name (INCI: Red 28, Red 28 Lake)), Red No. 106, Red No. 201 (:label name (INCI: Red 6)), Red No. 202 (:label name (INCI: Red 7)), Red No. 204, Red No. 205, Red No. 220 (:label name (INCI: Red 34)), Red No. 226 (:label name (INCI: Red 30)), Red No. 227 (:label name (INCI: Red 33, RED 33 Lake )), Red No. 228 (:label name (INCI: Red 36)), Red No. 230(1) (:label name (INCI: Red 22, Red 22 Lake)), Red No. 230(2)(label name), Red No. 401 (label name), Red No. 505 (label name), Yellow No. 4 (:label name (INCI: Yellow 5)), Yellow No. 5 (:label name (INCI: Yellow 6, Yellow 6 Lake)), Yellow No. 202(1) (:label name (INCI: Yellow 8)), Yellow No. 203 (:label name (INCI: Yellow 10, Yellow 10 Lake)), Yellow No. 204 (:label name (INCI: Yellow 11)), Yellow No. 401, Blue No. 1 (:label name (INCI: Blue 1, Blue 1 Lake)), Blue No. 2, Blue No. 201, Blue No. 205 (:label name (INCI: Blue 4)), Blue No. 404 (label name ), Green No. 3 (:label name (INCI: Green 3, Green 3 Lake)), Green No. 201 (:label name (INCI: Green 5)), Green No. 202 (:label name (INCI: Green 6)), Green No. 204 (:label name (INCI: Green 8)), Green No. 205 (label name ), Orange No. 201 (:label name (INCI: Orange 5)), Orange No. 203 (:label name (INCI: Pigment Orange 5)), Orange No. 204 (label name ), Orange No. 205 (:label name (INCI: Orange 4, Orange 4 Lake)), Orange No. 206 (:label name (INCI: Orange 10)), and Orange No. 207 (:label name (INCI: Orange 11)); and natural dyes such as Cochineal (INCI), laccaic acid (:label name (INCI: Laccaic Acid)), safflower red (:label name (INCI: Carthamus Tinctorius (Safflower) Flower Extract)), Lithospermum officinale root extract (:label name (INCI: Lithospermum Officinale Root Extract)), gardenia yellow (label name), and gardenia blue (:label name (INCI: Hydrolyzed Gardenia Florida Extract)).

Inorganic/organic composite powder

Examples of the inorganic/organic composite powder include composite powders prepared by coating the surfaces of inorganic powders with organic powders by a well-known method.

The powder used may be particles with treated surfaces. Preferably, from the viewpoint of the water resistance of the cosmetic, the surface treatment agent used can impart hydrophobicity. No particular limitation is imposed on the treatment agent for imparting hydrophobicity, and examples thereof include: silicone treatment agents, waxes, paraffins, organofluorine compounds such as perfluoroalkyls and phosphates, surfactants, amino acids such as N-acyl glutamic acid, and metal soaps such as aluminum stearate and magnesium myristate.

The surface treatment agent is more preferably a silicone treatment agent, and examples thereof include silanes and silylating agents such as Triethoxycaprylylsilane (INCI), Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethyl Hexyl Dimethicone (INCI), and an (acrylates/tridecyl acrylate/triethoxysilylpropyl methacrylate/dimethicone methacrylate) copolymer (:label name (INCI: Acrylates/Tridecyl Acrylate/Triethoxysilylpropyl Methacrylate/Dimethicone Methacrylate Copolymer).

Specific examples of the silicone treatment agent include AES-3083, KF-99P, KF-9901, KF-9908, KF-9909, KP-574, and KP-541 manufactured by Shin-Etsu Chemical Co., Ltd.

One of the above surface-hydrophobizing treatment agents may be used alone, or a combination of two or more maybe used. Specific examples of the surface-treated color pigment include the KTP-09 series manufactured by Shin-Etsu Chemical Co., Ltd., more particularly KTP-09W, KTP-09R, KTP-09Y, KTP-09B, etc.

(5) Composition of crosslinked organopolysiloxane and oily agent in liquid state at room temperature

In the composition of a crosslinked organopolysiloxane and an oily agent in a liquid state at room temperature, it is preferable that the crosslinked organopolysiloxane swells with the liquid oily agent so as to contain the liquid oil in an amount more than or equal to its own weight.

The liquid oily agent used may be any of the liquid silicone oils, hydrocarbon oils, ester oils, natural animal and vegetable oils and fats, semi-synthetic oils, and fluorine-based oils exemplified for the optional oily agent (1). Examples of the oily agent include Cyclopentasiloxane (INCI), Dimethicone (INCI), Mineral Oil (INCI), Isododecane (INCI), Isohexadecane (INCI), Triethylhexanoin (INCI), isotridecyl isononanoate (:label name (INCI: Isotridecyl Isononanoate)), and Squalane (INCI).

Unlike the crosslinked silicone surfactant used as the component (3) described above, the component (5) is a compound having no polyether or polyglycerin structure in its molecule, and specific examples include (Dimethicone/Vinyl Dimethicone) Crosspolymer (INCI), (Dimethicone/Phenyl Vinyl Dimethicone) Crosspolymer (INCI), (Vinyl Dimethicone/-Lauryl Dimethicone) Crosspolymer (INCI), and (Lauryl Polydimethylsiloxyethyl Dimethicone/Bis-Vinyldimethicone) Crosspolymer (INCI).

Commercial examples of the composition including the crosslinked organopolysiloxane and the oily agent in a liquid state at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z manufactured by Shin-Etsu Chemical Co., Ltd.

(6) Film-forming agent

The film-forming agent is added mainly for the purpose of further extending the effects of the cosmetic. No particular limitation is imposed on the film-forming agent, but the film-forming agent is preferably a silicone-based compound, from the viewpoint of imparting water repellency. Specific examples of the film-forming agent that can be used include trimethylsiloxysilicate, an acrylic-silicone film-forming agent, silicone-modified norbornene, silicone-modified pullulan, and silicone-modified polyvinyl alcohol.

Examples of the film-forming agent of the silicone-based composition include trimethylsiloxysilicate (:label name (INCI: Trimethylsiloxysilicate), (Acrylates/Dimethicone) Copolymer (INCI), Norbornene/Tris(Trimethylsiloxy)silylnorbornene Copolymer (INCI), and tri(trimethylsiloxy)silylpropyl carbamoyl pullulan (:label name (INCI: Trimethylsiloxysilylcarbamoyl Pullulan).

The film-forming agent may be dissolved in an oily agent in a liquid state at room temperature in advance and then added to the cosmetic. The liquid oily agent used may be any of the liquid silicone oils, hydrocarbon oils, ester oils, natural animal and vegetable oils and fats, semi-synthetic oils, and fluorine-based oils exemplified for the optional oily agent (1).

Commercial examples of the silicone film-forming agent include KF-7312J, KP-545, KP-549, KP-543, NBN-30-ID, TSPL-30-ID, and TSPL-30-D5 manufactured by Shin-Etsu Chemical Co., Ltd.

(7) Ultraviolet absorbing-scattering agent

Examples of the ultraviolet absorbing-scattering agent include particles capable of absorbing and/or scattering ultraviolet rays such as fine titanium oxide particles, ion-containing fine titanium oxide particles, fine zinc oxide particles, fine cerium oxide particles, and composites thereof. A dispersion prepared by dispersing any of these ultraviolet-absorbing/scattering particles in an oily agent in advance may be used.

The oily agent used may be any of the liquid silicone oils, hydrocarbon oils, ester oils, natural animal and vegetable oils and fats, semi-synthetic oils, and fluorine-based oils exemplified for the optional oily agent (1).

Specific examples of the dispersion prepared by dispersing any of these ultraviolet-absorbing/scattering particles in an oily agent in advance include the SPD series manufactured by Shin-Etsu Chemical Co., Ltd., more particularly SPD-T5, SPD-T5L, SPD-Z5, SPD-Z5L, SPD-T6, SPD-Z6, SPD-T7, and SPD-Z7L.

(8) Additional additives

Examples of the additional additives include an oil-soluble gelling agent, a preservative/bactericide, an antiperspirant, a fragrance, a salt, an antioxidant, a pH modifier, a chelating agent, a refrigerant, an anti-inflammatory agent, skin care components (such as a whitening agent, a cell activating agent, a rough skin improving agent, a blood circulation promoting agent, a skin astringent, and an antiseborrheic agent), vitamins, amino acids, nucleic acids, hormones, and an inclusion compound.

Oil-soluble gelling agent

Examples of the oil-soluble gelling agent include: metal soaps such as aluminum stearate, magnesium stearate, and zinc myristate; amino acid derivatives such as lauroyl glutamic acid (:label name (INCI: Lauroyl Glutamic Acid)) and $\alpha,\gamma$-di-n-butylamine; dextrin fatty acid esters such as dextrin palmitate (:label name (INCI: Dextrin Palmitate)), dextrin isostearate (:label name (INCI: Dextrin Isostearate)), dextrin myristate (:label name (INCI: Dextrin Myristate)), stearoyl inulin (:label name (INCI: Stearoyl Inulin)), and dextrin (palmitate/ethylhexanoate) (:label name (INCI: Dextrin Palmitate/Ethylhexanoate)); sucrose fatty acid esters such as sucrose palmitate and sucrose stearate; fructooligosaccharide fatty acid esters such as fructooligosaccharide stearate and fructooligosaccharide 2-ethylhexanoate; benzylidene derivatives of sorbitol such as monobenzylidene sorbitol and dibenzylidene sorbitol; Disteardimonium Hectorite (INCI), Stearalkonium Hectorite (INCI), and organic modified clay minerals of hectorite; and Stearalkonium Bentonite (INCI).

Preservative/bactericide

Examples of the preservative/bactericide include paraoxybenzoic acid alkyl esters, benzoic acid, sodium benzoate, sorbic acid, potassium sorbate, phenoxyethanol, imidazolidinyl urea, salicylic acid, isopropylmethylphenol, carbolic acid, parachlorometacresol, hexachlorophene, benzalkonium chloride, chlorhexidine chloride, trichlorocarbanilide, iodopropynyl butylcarbamate, polylysine, photosensitizers, silver, and plant extracts.

Antiperspirant

Examples of the antiperspirant include aluminum hydroxyhalides such as aluminum hydroxychloride, aluminum halides such as aluminum chloride, an aluminum salt of allantoin, tannic acid, persimmon tannin, (aluminum/potassium) sulfate, zinc oxide, zinc parephenolsulfonate, burnt alum, (aluminum/zirconium) tetrachlorohydrate, and (aluminum/zirconium) trichlorohydrex glycine.

In particular, aluminum hydroxyhalides, aluminum halides, complexes and mixtures of these compounds with zirconyl oxyhalides and zirconyl hydroxyhalides (such as (aluminum/zirconium) tetrachlorohydrate and (aluminum/zirconium) trichlorohydrex glycine) are preferred as components exhibiting a high effect.

Fragrance

Examples of the fragrance include natural fragrances and synthetic fragrances. Examples of the natural fragrances include: vegetable fragrances separated from flowers, leaves, stems, and pericarps; and animal fragrances such as musk and civet. Examples of the synthetic fragrances include: hydrocarbons such as monoterpene; alcohols such as aliphatic alcohols and aromatic alcohols; aldehydes such as terpene aldehydes and aromatic aldehydes; ketones such as alicyclic ketones; esters such as terpene-based esters; lactones; phenols; oxides; nitrogen-containing compounds; and acetals.

Salts

Examples of the salt include inorganic salts, organic acid salts, amine salts, and amino acid salts. Examples of the inorganic salts include sodium, potassium, magnesium, calcium, aluminum, zirconium, and zinc salts of inorganic acids such as hydrochloric acid, sulfuric acid, carbonic acid, and nitric acid. Examples of the organic acid salts include salts of organic acids such as acetic acid, dehydroacetic acid, citric acid, malic acid, succinic acid, ascorbic acid, and stearic acid. Examples of the amine salts and amino acid salts include salts of amines such as triethanolamine and salts of amino acids such as glutamic acid. In addition, salts of hyaluronic acid, chondroitin sulfuric acid, etc., aluminum-zirconium-glycine complexes, etc. can be used, and acid-alkali neutralization salts used in cosmetic formulations can also be used.

Antioxidant

No particular limitation is imposed on the antioxidant. Examples of the antioxidant include carotenoid, ascorbic acid and salts thereof, ascorbyl stearate, tocophenol, tocophenol acetate, tocopherol, p-t-butylphenol, butylated hydroxyanisole, dibutylhydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. One antioxidant may be used alone, or a combination of two or more antioxidants may be used.

pH modifier

Examples of the pH modifier include lactic acid, citric acid, glycolic acid, succinic acid, tartaric acid, dl-malic acid, potassium carbonate, sodium hydrogencarbonate, and ammonium hydrogencarbonate.

Chelating agent

Examples of the chelating agent include alanine, sodium edetate, sodium polyphosphate, sodium metaphosphate, and phosphoric acid.

Refrigerant

Examples of the refrigerant include L-menthol, camphor, and menthyl lactate.

Anti-inflammatory agent

Examples of the anti-inflammatory agent include allantoin, glycyrrhizic acid and salts thereof, glycyrrhetinic acid, stearyl glycyrrhetinate, tranexamic acid, and azulene.

Skin care component

Examples of the skin care component include: whitening agents such as arbutin, glutathione, and Saxifraga stolonifera extract; cell activating agents such as royal jelly, photosensitizers, cholesterol derivatives, and calf blood extract; rough skin improving agents; blood circulation promoting agents such as vanillylamide nonylate, benzyl nicotinate, β-butoxyethyl nicotinate, capsaicin, zingerone, cantharides tincture, ichthammol, caffeine, tannic acid, α-borneol, tocopheryl nicotinate, inositol hexanicotinate, cyclandelate, cinnarizine, tolazoline, acetylcholine, verapamil, cepharanthine, and γ-oryzanol; skin astringents such as zinc oxide and tannic acid; and antiseborrheic agents such as sulfur and thianthol.

Vitamins

Examples of the vitamins include: vitamin A compounds such as vitamin A oil, retinol, retinol acetate, and retinol palmitate; vitamin B2 compounds such as riboflavin, riboflavin butyrate, and flavin adenine nucleotide; vitamin B6 compounds such as pyridoxine hydrochloride, pyridoxine dioctanoate, and pyridoxine tripalmitate; vitamin B compounds such as vitamin B12 and derivatives thereof, vitamin B15 and derivatives thereof; vitamin C compounds such as L-ascorbic acid, L-ascorbyl dipalmitate, L-ascorbic acid-2-sodium sulfate, and dipotassium L-ascorbyl phosphate; vitamin D compounds such as ergocalciferol and cholecalciferol; vitamin E compounds such as α-tocopherol, β-tocopherol, γ-tocopherol, dl-α-tocopherol acetate, dl-α-tocopheryl nicotinate, and dl-α-tocopherol succinate; nicotinic acids such as nicotinic acid, benzyl nicotinate, and nicotinamide; vitamin H; vitamin P; pantothenic acids such as calcium pantothenate, D-pantothenyl alcohol, pantothenyl ethyl ether, and acetyl pantothenyl ethyl ether; and biotin.

Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

Nucleic acid

Examples of the nucleic acid include deoxyribonucleic acid.

Hormone

Examples of the hormone include estradiol and ethenyl estradiol.

Inclusion compound

Examples of the inclusion compound include cyclodextrin.

Examples

The present invention will next be described in more detail by way of working examples and comparative examples. However, the present invention is not limited to the following working examples.

In the following examples, "%" and "parts" as used herein for denoting concentrations and contents refer to "% by mass" and "parts by mass," respectively, unless otherwise specified. The kinematic viscosity is a value measured at 25°C. In the following examples, the volume average particle diameters and aspect ratios of spherical elastomer particles and elastomer composite particles are values measured by the methods described above.

The hardness of a cured rubber product (elastomer) is a value measured according to The Society of Rubber Industry, Japan Standard (SRIS).

The molecular weight of a copolymer having a polyester structure and a polyether structure (a polyester-polyether copolymer having at least two radical-polymerizable unsaturated groups per molecule) is a weight average molecular weight (Mw.) measured by GPC using polystyrene as a reference material under the following conditions.

[Measurement conditions]

Eluent: Tetrahydrofuran (THF)
Flow rate: 0.60 mL/min.
Detector: Differential refractometer (RI)
Columns: TSK Guardcolumn SuperH-H,

TSKgel SuperHM-N,
TSKgel SuperH2500 (all manufactured by TOSOH Corporation)

Column temperature: 40°C
Sample injection volume: 50 μL (THF solution with a concentration of 0.5% by mass)

[Method for measuring and evaluating biodegradability]

The biodegradability was measured by a method using activated sludge as a microbial (degradation) source according to OECD Guidelines for the Testing of Chemicals, No. 301F, July 17, 1992, "Ready Biodegradability: MANOMETRIC RESPIROMETRY TEST" and evaluated based on the degree of biodegradation. The activated sludge used was activated sludge from a municipal sewage treatment plant, and the concentration of suspended materials was 2,400 mg/L. Sodium benzoate was used as a reference (control) material.

To measure the degree of biodegradation, the consumption of oxygen (Biochemical Oxygen Demand (BOD)) in a closed system in an incubator was measured using a BOD meter, and the degree of biodegradation was computed using the following formula.

$$\text{Degree of biodegradation (\%)} = \text{BOD} - \text{B} / \text{TOD} \times 100$$

BOD: Biochemical oxygen demand of test suspension or operation control (measured value: mg)
B: Average biochemical oxygen demand of plant-derived blank (measured value: mg)
TOD: Theoretical oxygen demand necessary to completely oxidize test substance or sodium benzoate (calculated value: mg)

[Synthesis example 1: Acrylic-modified poly-ε-caprolactone/polyether copolymer 1]

A 2 L separable flask equipped with a stirrer, a thermometer, a condenser tube, and a dropping funnel was charged with 500 g of end-primarized EO (ethylene oxide)/PO (propylene oxide) polyether (molecular weight: about 2,000, OH group equivalent: 0.09-0.10 mol/100 g), 183.6 g of ε-caprolactone (molecular weight: 114.1), and 350 g of dehydrated toluene, and the mixture was heated to 90°C in a nitrogen flow. After the target temperature was reached, 0.68 g of tetra-n-butoxytitanium (molecular weight 340.0) serving as a catalyst was added, and the resulting mixture was aged at 120°C for 4 to 6 hours.

Next, the poly-ε-caprolactone/polyether copolymer obtained through the above procedure was cooled to near room temperature, and then 60.7 g of triethylamine (molecular weight 101.2), 100 g of dehydrated toluene, and 0.22 g of dibutylhydroxytoluene (BHT) (molecular weight 220.4) serving as a polymerization inhibitor were added. The mixture was stirred for a predetermined time to achieve uniform dissolution, and 49.7 g of acryloyl chloride (molecular weight 90.5) was added dropwise using a dropping funnel. Once heat generation was detected, the mixture was aged at 60°C for 4 hours.

The obtained crude product was subjected to pressure filtration, to washing operation with an aqueous sodium chloride solution using a separatory funnel, and to centrifugation etc. Then magnesium sulfate, silica gel, activated carbon, etc. were added, and the resulting mixture was subjected to powder processing by shaking to remove impurities by adsorption. The powders were removed by pressure filtration, and 0.22 g of dibutylhydroxytoluene (BHT) was again added. Then the solvent was removed by evaporation under the conditions of 60 to 70°C and 50 mmHg or lower to thereby obtain an acrylic-modified poly-ε-caprolactone/polyether copolymer 1 (the following formula (11), weight average molecular weight: 2,880).

[Chem. 11]

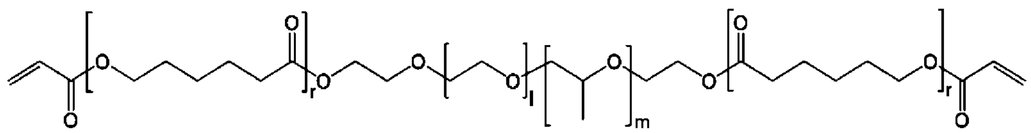

- (11)

(1 ≈ 12 to 14, m ≈ 22 to 26, r ≈ 3 to 4)

[Synthesis example 2: Acrylic-modified poly-ε-caprolactone/polyether copolymer 2]

A 1 L separable flask equipped with a stirrer, a thermometer, a condenser tube, and a dropping funnel was charged with 300 g of the same end-primarized EO/PO polyether as that in the synthesis example 1, 110.2 g of ε-caprolactone, and 200 g of dehydrated toluene, and the mixture was heated to 90°C in a nitrogen flow. After the target temperature was reached, 0.41 g of tetra-n-butoxytitanium serving as a catalyst was added, and the resulting mixture was aged at 120°C for 4 to 6 hours.

Next, the poly-ε-caprolactone/polyether copolymer obtained through the above procedure was cooled to near room temperature, and then 100 g of dehydrated toluene, 0.96 g of dioctyltin dineodecanoate (molecular weight: 687.7) serving as a catalyst, and 0.12 g of dibutylhydroxytoluene (BHT) were added. The mixture was stirred for a predetermined time to achieve uniform dissolution, and 44.4 g of 2-isocyanatoethyl acrylate (molecular weight: 141.1) was added dropwise using a dropping funnel. Once heat generation was detected, the mixture was aged at 60°C for 4 hours.

The obtained crude product was cooled to 40°C or lower, and then 0.8 g of ethanol was added and reacted with unreacted (remaining) isocyanate groups to perform quenching treatment. Then magnesium sulfate, silica gel, activated carbon, etc. were added, and the resulting mixture was subjected to powder processing by shaking to remove impurities by adsorption. The powders were removed by pressure filtration, and 0.12 g of dibutylhydroxytoluene (BHT) was again added, and the solvent was removed by evaporation under the conditions of 60 to 70°C and 50 mmHg or lower to thereby obtain an acrylic-modified poly-ε-caprolactone/polyether copolymer 2 (the following formula (12), weight average molecular weight: 3,120).

[Chem. 12]

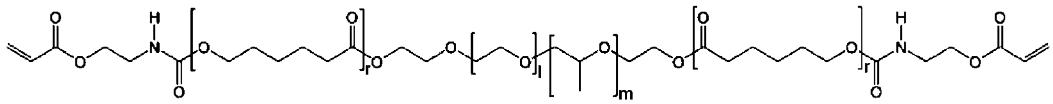

- (12)

(1 ≈ 12 to 14, m ≈ 22 to 26, r ≈ 3 to 4)

[Synthesis example 3: Acrylic-modified poly-ε-caprolactone/polyether copolymer 3]

A 1 L separable flask equipped with a stirrer, a thermometer, a condenser tube, and a dropping funnel was charged with 300 g of end-primarized EO/PO polyether (molecular weight: about 2,600, OH group equivalent: 0.09 to 0.10 mol/100 g), 115.2 g of ε-caprolactone, and 200 g of dehydrated toluene, and the mixture was heated to 90°C in a nitrogen flow. After the target temperature was reached, 0.42 g of tetra-n-butoxytitanium serving as a catalyst was added, and the resulting mixture was aged at 120°C for 4 to 6 hours.

Next, the poly-ε-caprolactone/polyether copolymer obtained through the above procedure was cooled to near room temperature, and then 70 g of dehydrated toluene, 0.18 g of tris(2,4-pentanedionato)iron(III) (also known as iron(III) acetylacetonate, molecular weight: 353.2), and 0.13 g of dibutylhydroxytoluene (BHT) were added. The mixture was stirred for a predetermined time to achieve uniform dissolution, and 46.4 g of 2-isocyanatoethyl acrylate was added dropwise using a dropping funnel. Once heat generation was detected, the mixture was aged at 60°C for 4 hours.

The obtained crude product was subjected to post-treatment and purification treatment using the same method as described in the synthesis example 2 to thereby obtain an acrylic-modified poly-ε-caprolactone/polyether copolymer 3. The acrylic-modified poly-ε-caprolactone/polyether copolymer 3 had the structure shown in the formula (12), wherein, in the formula (12), 1 ≈ 18 to 21, m ≈ 34 to 38, and r ≈ 3 to 4, and the weight average molecular weight was 4,870.

[Synthesis example 4: Acrylic-modified poly-ε-caprolactone/polyether copolymer 4]

A 1 L separable flask equipped with a stirrer, a thermometer, a condenser tube, and a dropping funnel was charged with 300 g of the same end-primarized EO/PO polyether as that used in the synthesis example 3, 115.2 g of ε-caprolactone, and 200 g of dehydrated toluene, and the mixture was heated to 90°C in a nitrogen flow. After the target temperature was reached, 0.42 g of tetra-n-butoxytitanium serving as a catalyst was added, and the resulting mixture was aged at 120°C for 4 to 6 hours.

Next, the poly-ε-caprolactone/polyether copolymer obtained through the above procedure was cooled to near room temperature, and 70 g of dehydrated toluene, 2.77 g of zirconium tetraacetylacetonate (molecular weight: 487.7) serving as a catalyst, and 0.13 g of dibutylhydroxytoluene (BHT) were added. The mixture was stirred for a predetermined time to achieve uniform dissolution, and 46.4 g of 2-isocyanatoethyl acrylate was added dropwise using a dropping funnel. Once heat generation was detected, the mixture was aged at 60°C for 4 hours.

The obtained crude product was subjected to post-treatment and purification treatment using the same method as described in the synthesis example 2 to thereby obtain an acrylic-modified poly-ε-caprolactone/polyether copolymer 4. The acrylic-modified poly-ε-caprolactone/polyether copolymer 4 had the structure shown in the formula (12), wherein, in the formula (12), 1 ≈ 18 to 21, m ≈ 34 to 38, and r ≈ 3 to 4, and the weight average molecular weight was 4,990.

[Synthesis example 5: Acrylic-modified poly-ε-caprolactone/polyether copolymer 5]

A 1 L separable flask equipped with a stirrer, a thermometer, a condenser tube, and a dropping funnel was charged with 300 g of end-primarized EO/PO polyether (molecular weight: about 3,200, OH group equivalent: 0.062 mol/100 g), 106.3 g of ε-caprolactone, and 200 g of dehydrated toluene, and the mixture was heated to 90°C in a nitrogen flow. After the target temperature was reached, 0.41 g of tetra-n-butoxytitanium serving as a catalyst was added, and the resulting mixture was aged at 120°C for 4 to 6 hours.

Next, the poly-ε-caprolactone/polyether copolymer obtained through the above procedure was cooled to near room temperature, and then 70 g of dehydrated toluene, 1.01 g of dioctyltin dineodecanoate serving as a catalyst, and 0.13 g of dibutylhydroxytoluene (BHT) were added. The mixture was stirred for a predetermined time to achieve uniform dissolution, and 28.4 g of 2-isocyanatoethyl acrylate was added dropwise using a dropping funnel. Once heat generation was detected, the mixture was aged at 60°C for 4 hours.

The obtained crude product was subjected to post-treatment and purification treatment using the same method as described in the synthesis example 2 to thereby obtain an acrylic-modified poly-ε-caprolactone/polyether copolymer 5 (formula (13), weight average molecular weight: 7,940).

[Chem. 13]

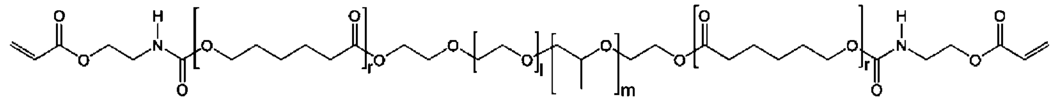

- (13)

(1 ≈ 24 to 26, m ≈ 35 to 37, r ≈ 4 to 5)

[Synthesis example 6: Acrylic-modified poly-ε-caprolactone/polyether copolymer 6]

A 1 L separable flask equipped with a stirrer, a thermometer, a condenser tube, and a dropping funnel was charged with 300 g of end-primarized EO/PO polyether (molecular weight: about 1,100, OH group equivalent: 0.182 mol/100 g), 311.6 g of ε-caprolactone, and 250 g of dehydrated toluene, and the mixture was heated to 90°C in a nitrogen flow. After the target temperature was reached, 0.61 g of tetra-n-butoxytitanium serving as a catalyst was added, and the resulting mixture was aged at 120°C for 4 to 6 hours.

Next, the poly-ε-caprolactone/polyether copolymer obtained through the above procedure was cooled to near room temperature, and then 100 g of dehydrated toluene, 2.77 g of zirconium tetraacetylacetonate (molecular weight: 487.7) serving as a catalyst, and 0.21 g of dibutylhydroxytoluene (BHT) were added. The mixture was stirred for a predetermined time to achieve uniform dissolution, and 80.84 g of 2-isocyanatoethyl acrylate (molecular weight: 141.1) was added dropwise using a dropping funnel. Once heat generation was detected, the mixture was aged at 60°C for 4 hours.

The obtained crude product was subjected to post-treatment and purification treatment using the same method as described in the synthesis example 2 to thereby obtain an acrylic-modified poly-ε-caprolactone/polyether copolymer 6 (formula (14), weight average molecular weight: 2,530).

[Chem. 14]

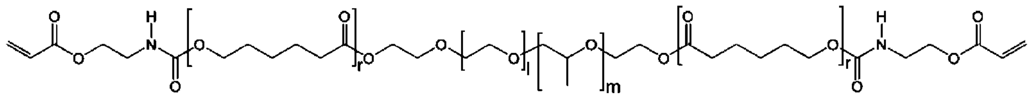

- (14)

(1 ≈ 6 to 8, m ≈ 11 to 13, r ≈ 5 to 6)

[Synthesis example 7: Acrylic-modified poly-ε-caprolactone/polyether copolymer 7]

A 1 L separable flask equipped with a stirrer, a thermometer, a condenser tube, and a dropping funnel was charged with 300 g of end-primarized EO/PO polyether (molecular weight: about 7,200, OH group equivalent: 0.028 mol/100 g), 31.4 g of ε-caprolactone, and 200 g of dehydrated toluene, and the mixture was heated to 90°C in a nitrogen flow. After the target temperature was reached, 0.33 g of tetra-n-butoxytitanium was added, and the resulting mixture was aged at 120°C for 4 to 6 hours.

Next, the poly-ε-caprolactone/polyether copolymer obtained through the above procedure was cooled to near room temperature, and then 50 g of dehydrated toluene, 1.37 g of zirconium tetraacetylacetonate (molecular weight: 487.7) serving as a catalyst, and 0.11 g of dibutylhydroxytoluene (BHT) were added. The mixture was stirred for a predetermined time to achieve uniform dissolution, and 12.3 g of 2-isocyanatoethyl acrylate (molecular weight: 141.1) was added dropwise using a dropping funnel. Once heat generation was detected, the mixture was aged at 60°C for 4 hours.

The obtained crude product was subjected to post-treatment and purification treatment using the same method as described in the synthesis example 2 to thereby obtain an acrylic-modified poly-ε-caprolactone/polyether copolymer 7 (formula (15), weight average molecular weight: 8,250).

[Chem. 15]

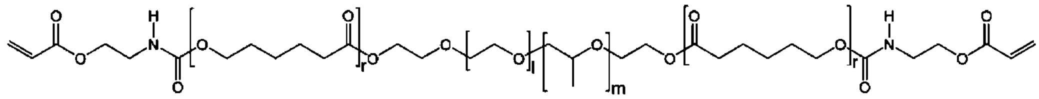

- (15)

(1 ≈ 24 to 32, m ≈ 44 to 50, r ≈ 3 to 4)

[Evaluation of biodegradability]

The biodegradability of the acrylic-modified poly-ε-caprolactone/polyether copolymer 1 was evaluated according to the evaluation method described above. The measurement was performed at an incubation temperature of 22±1°C for 60 days. The degree of biodegradation of the acrylic-modified poly-ε-caprolactone/polyether copolymer 1 was 62% on

average at day 28 and 73% on average at day 60. Since the degree of biodegradation was better than the evaluation criterion at day 28, i.e., a degree of biodegradation of 60%, the acrylic-modified poly-ε-caprolactone/polyether copolymer 1 was determined as a "readily biodegradable material."

Therefore, even when spherical elastomer particles produced as crosslinked particles of the acrylic-modified poly-ε-caprolactone/polyether copolymer 1 and the elastomer composite particles flow into the oceans through inland water etc. after use, it is assumed that they will eventually degrade without remaining in the environment as particles.

The biodegradability of each of the acrylic-modified poly-ε-caprolactone/polyether copolymers 2 to 4 was evaluated. Specifically, the measurement was performed at an incubation temperature of 22±1°C for 60 days. The degree of biodegradation of each of the acrylic-modified poly-ε-caprolactone/polyether copolymers 2 to 4 was 40% on average at day 28 and 65% on average at day 60. Since the degree of biodegradation was better than the evaluation criterion at day 60, i.e., a degree of biodegradation of 60%, the acrylic-modified poly-ε-caprolactone/polyether copolymers were determined as "inherently biodegradable materials."

Therefore, even when spherical elastomer particles produced as crosslinked particles of each of the acrylic-modified poly-ε-caprolactone/polyether copolymers 2 to 4, the composite elastomer particles thereof, spherical elastomer particles produced as crosslinked particles of each of the acrylic-modified poly-ε-caprolactone/polyether copolymers 5 to 7 that have the same repeating unit structures as those of the acrylic-modified poly-ε-caprolactone/polyether copolymers 2 to 4 but differ in the number of repeating units, and the composite elastomer particles thereof flow into the oceans through inland water etc. after use, it is assumed that they will eventually degrade without remaining in the environment as particles.

[Preparation example 1: Spherical elastomer particles 1]

A 1 L container of an agi-homo mixer was charged with 250 g of a 2.6% aqueous solution of hydroxypropyl methyl cellulose (product name: METOLOSE 60SH-4,000 manufactured by Shin-Etsu Chemical Co., Ltd.), and the aqueous solution was stirred and mixed under heating at 55 to 60°C while a homogenizing mixer and an anchor mixer were operated.

At the same time, a disposable cup was charged with 100 g of the acrylic-modified poly-ε-caprolactone/polyether copolymer 4 described in the synthesis example 4 and 1.0 g of dimethyl 2,2'-azobis-(2-methyl propionate) (molecular weight: 230.3), and the components were premixed using a dispersing mixer and preheated to 60 to 65°C.

Next, the premixed acrylic-modified poly-ε-caprolactone/polyether copolymer was added to the 1 L agi-homo mixer containing the heated aqueous hydroxypropyl methyl cellulose solution, and the resulting mixture was stirred under heating at 55 to 60°C at a homo mixer rotation speed of 4,500 rpm for 10 minutes or longer for emulsification and suspension, and an O/W type emulsion (suspension) composition was thereby obtained.

Then the obtained emulsion (suspension) composition was stirred and aged at 70°C for 8 hours using a paddle-type stirring impeller at a rotation speed of 200 rpm. Then 50 g of pure water was added, and the resulting mixture was further aged at 80°C at the same rotation speed for 6 hours to thereby obtain a dispersion of spherical elastomer particles 1.

The shapes of the spherical elastomer particles 1 in the obtained dispersion were observed under an optical microscope and were found to be spherical. Their volume average particle diameter was measured by an electric resistance method using a particle size distribution analyzer (Multisizer 3 manufactured by Beckman Coulter, Inc.) and was found to be 5 μm.

The obtained dispersion of the spherical elastomer particles 1 was filtered using a mesh (#100) to check the presence of aggregates. When aggregates were found, they were removed, and then pure water present as a continuous phase was removed by solid-liquid separation using pressure filtration. This washing-separation procedure using pure water was repeated three times, and the water was thereby removed. When the solid-liquid separation in the pressure filtration step was not successful, centrifugation may be optionally performed.

Finally, the concentrate of the resulting spherical elastomer particles 1 was left to stand and dried for 8 hours or longer to obtain the target spherical elastomer particles 1 as a white to light yellow powder.

The obtained powder of the spherical elastomer particles 1 was observed under an electron microscope (scanning electron microscope S-4700 manufactured by Hitachi High-Technologies Corporation), and the spherical elastomer particles were found to be spherical particles with a diameter of about 5 μm. The aspect ratio of the obtained spherical elastomer particles 1 was 1.0.

The obtained spherical elastomer particles 1 were re-dispersed in water using polyoxyethylene lauryl ether serving as a surfactant, and measurement using an electric resistance method was performed on the dispersion to evaluate the dispersion. The volume average particle diameter of the spherical elastomer particles 1 was found to be 5 μm.

The hardness of the elastomer (rubber) in the spherical elastomer particles 1 was measured as follows.

30 g of the synthesized acrylic-modified poly-ε-caprolactone/polyether copolymer 4 and 0.24 g of 2'-azobis-(2,4-dimethylvaleronitrile) (molecular weight: 248.4) were mixed and stirred and then poured into an aluminum-made petri dish such that the thickness of the mixture was 10 mm. The petri dish was left to stand at 70°C in an air thermostatic chamber for 1 to 2 hours, and a flat rubber with no stickiness (no tackiness) was thereby obtained. The rubber hardness of the obtained

flat rubber was measured. The hardness was 72 as measured by an Asker C hardness meter and 55 as measured by an Asker A hardness meter.

[Preparation example 2: Spherical elastomer particles 2]

Spherical elastomer particles 2 were produced using the same procedure as in the preparation example 1 except that 250 g of the 2.6% aqueous hydroxypropyl methyl cellulose solution was replaced with 173 g of a 3.8% aqueous hydroxypropyl methyl cellulose solution. The volume average particle diameter of the obtained spherical elastomer particles was 2 μm.

[Preparation example 3: Spherical elastomer particles 3]

Spherical elastomer particles 3 were produced using the same procedure as in the preparation example 1 except that the hydroxypropyl methyl cellulose (product name: METOLOSE 60SH-4,000 manufactured by Shin-Etsu Chemical Co., Ltd.) was replaced with hydroxypropyl methyl cellulose (product name: METOLOSE 60SH-50 manufactured by Shin-Etsu Chemical Co., Ltd.). The volume average particle diameter of the obtained spherical elastomer particles was 11 μm.

[Preparation example 4: Spherical elastomer particles 4]

Spherical elastomer particles 4 were produced using the same procedure as in the preparation example 1 except that 250 g of the 2.6% aqueous solution of the hydroxypropyl methyl cellulose (product name: METOLOSE 60SH-4,000 manufactured by Shin-Etsu Chemical Co., Ltd.) was replaced with 250 g of a 5.2% aqueous xanthan gum solution. The volume average particle diameter of the obtained spherical elastomer particles was 20 μm.

[Preparation example 5: Spherical elastomer particles 5]

Spherical elastomer particles 5 were produced using the same procedure as in the preparation example 1 except that the acrylic-modified poly-ε-caprolactone/polyether copolymer 4 in the preparation example 1 was replaced with the acrylic-modified poly-ε-caprolactone/polyether copolymer 6. The volume average particle diameter of the obtained spherical elastomer particles was 5 μm.

The hardness of the elastomer (rubber) of the spherical elastomer particles 5 was measured using the same method as described above. The elastomer was a flat rubber with no stickiness (no tackiness), which exhibited a hardness of 78 as measured by an Asker A hardness meter.

[Preparation example 6: Spherical elastomer particles 6]

Spherical elastomer particles 6 were produced using the same procedure as in the preparation example 1 except that the acrylic-modified poly-ε-caprolactone/polyether copolymer 4 in the preparation example 1 was replaced with the acrylic-modified poly-ε-caprolactone/polyether copolymer 7. The volume average particle diameter of the obtained spherical elastomer particles was 5 μm.

The hardness of the elastomer (rubber) of the spherical elastomer particles 6 was measured using the same method as described above. The elastomer was a flat rubber with no stickiness (no tackiness), which exhibited a hardness of 22 as measured by an Asker A hardness meter.

[Comparative example 1: Cellulose powder]

Cellulose powder CELLULOBEADS D-5 (average particle diameter 5 μm) manufactured by DAITO KASEI KOGYO CO., LTD. was used.

[Comparative example 2: Spherical silica particles]

Spherical silica: SILICA MICRO BEAD P-1500 (average particle diameter 5 μm) manufactured by JGC Catalysts and Chemicals Ltd. was used.

[Preparation/production example 1: Elastomer composite particles 1]

A 500 mL glass flask equipped with a stirrer of a paddle-type stirring impeller was charged with 100 g of the dispersion of the spherical elastomer particles 1 obtained in the preparation example 1 and having a solid concentration of

20%, 184.9 g of pure water, and 0.2 g of a 30% aqueous lauryltrimethylammonium chloride solution. The temperature of the water dispersion was adjusted to 5 to 10°C, and 0.75 g of 5.0% ammonia water was added. Then, while the temperature of the water dispersion was maintained at 5 to 10°C, 11.2 g of tetramethoxysilane (this amount corresponds to 22.0 parts of silica based on 100 parts of the spherical elastomer particles after the hydrolysis-polycondensation reaction) was added dropwise over 15 to 30 minutes. The resulting mixture was stirred for 1 hour while the temperature of the mixture was maintained at 5 to 10°C.

Next, the resulting mixture was heated to 55 to 60°C and stirred and aged for 1 hour while the temperature was maintained to complete the hydrolysis-condensation reaction of tetramethoxysilane.

The dispersion obtained by subjecting the tetramethoxysilane in the dispersion of the spherical elastomer particles 1 to the hydrolysis-condensation reaction was subjected to dehydration using a pressure filtration apparatus to a water content of about 30%. Next, the dehydrated product was transferred to a 1 L glass flask equipped with a stirrer of an anchor-type stirring impeller, and 500 g of water was added. The resulting mixture was stirred for 30 minutes and dehydrated by pressure filtration. This washing/dehydration procedure was repeated three times, and the resulting dehydrated product was dried in a hot air fluidized bed dryer under the condition of 105°C, and the dried product was pulverized using a jet mill to thereby obtain flowable particles.

The obtained particles were observed by an electron microscope. Spherical silica was found to adhere to the spherical elastomer particles so as to cover the entire particle surfaces, and silica-coated spherical elastomer particles (elastomer composite particles 1) were found to be obtained. The aspect ratio of the silica-coated spherical elastomer particles (elastomer composite particles 1) thus obtained was 1.0.

The obtained silica-coated spherical elastomer particles (elastomer composite particles 1) were dispersed in water using a surfactant (polyoxyethylene lauryl ether). Their particle size distribution was measured by an electric resistance method using a particle size distribution analyzer (Multisizer 3 manufactured by Beckman Coulter, Inc.), and the volume average particle diameter was found to be about 5 μm, which is the same as that of the spherical elastomer particles 1 in the water dispersion described above.

[Preparation/production example 2: Elastomer composite particles 2]

Elastomer composite particles were produced using the same procedure as in the preparation/production example 1 except that the spherical elastomer particles 1 were replaced with the spherical elastomer particles 2 (preparation example 2). The volume average particle diameter of the obtained silica-coated spherical elastomer particles (elastomer composite particles 2) was about 2 μm.

[Preparation/production example 3: Elastomer composite particles 3]

Elastomer composite particles were produced using the same procedure as in the preparation/production example 1 except that the spherical elastomer particles 1 were replaced with the spherical elastomer particles 3 (preparation example 3). The volume average particle diameter of the obtained silica-coated spherical elastomer particles (elastomer composite particles 3) was about 11 μm.

[Preparation/production example 4: Elastomer composite particles 4]

Elastomer composite particles were produced using the same procedure as in the preparation/production example 1 except that the spherical elastomer particles 1 were replaced with the spherical elastomer particles 4 (preparation example 4). The volume average particle diameter of the obtained silica-coated spherical elastomer particles (elastomer composite particles 4) was about 20 μm.

[Preparation/production example 5: Elastomer composite particles 5]

Elastomer composite particles were produced using the same procedure as in the preparation/production example 1 except that the spherical elastomer particles 1 were replaced with the spherical elastomer particles 5 (preparation example 5). The volume average particle diameter of the obtained silica-coated spherical elastomer particles (elastomer composite particles 5) was about 5 μm.

[Preparation/production example 6: Elastomer composite particles 6]

Elastomer composite particles were produced using the same procedure as in the preparation/production example 1 except that the spherical elastomer particles 1 were replaced with the spherical elastomer particles 6 (preparation example 6). The volume average particle diameter of the obtained silica-coated spherical elastomer particles

(elastomer composite particles 6) was about 5 μm.

[Preparation/production example 7: Elastomer composite particles 7]

A 500 mL glass flask equipped with a stirrer of a paddle-type stirring impeller was charged with 100 g of the dispersion of the spherical elastomer particles 3 obtained in the preparation example 3 and having a solid concentration of 20%, 184.9 g of pure water, and 0.2 g of a 30% aqueous lauryltrimethylammonium chloride solution. The temperature of the water dispersion was adjusted to 5 to 10°C, and 0.84 g of 5.0% ammonia water was added. Then, while the temperature of the water dispersion was maintained at 5 to 10°C, 12.5 g of methyltrimethoxysilane (this amount corresponds to 22.0 parts of silane based on 100 parts of the spherical elastomer particles after the hydrolysis-polycondensation reaction) was added dropwise over 15 to 20 minutes. The resulting mixture was stirred for 1 hour while the temperature of the mixture was maintained at 5 to 10°C. The subsequent procedure followed that in the preparation/production example 1, and flowable particles were thereby obtained.

The obtained particles were observed by an electron microscope. Spherical polyorganosilsesquioxane was found to adhere to the spherical elastomer particles so as to cover the entire particle surfaces, and polyorganosilsesquioxane-coated spherical elastomer particles (elastomer composite particles 7) were found to be obtained. The aspect ratio of the obtained polyorganosilsesquioxane-coated spherical elastomer particles (elastomer composite particles 7) was 1.0.

The obtained polyorganosilsesquioxane-coated spherical elastomer particles (elastomer composite particles 7) were dispersed in water using a surfactant (polyoxyethylene lauryl ether). Their particle size distribution was measured by an electric resistance method using a particle size distribution analyzer (Multisizer 3 manufactured by Beckman Coulter, Inc.), and the volume average particle diameter was found to be about 11 μm.

[Preparation/production example 8: Elastomer composite particles 8]

Elastomer composite particles were produced using the same procedure as in the preparation/production example 1 except that the amount of the tetramethoxysilane added was changed from 11.2 g (this amount corresponds to 22.0 parts of silica based on 100 parts of the spherical elastomer particles after the hydrolysis-polycondensation reaction) to 5.6 g (this amount corresponds to 11.0 parts of silica based on 100 parts of the spherical elastomer particles after the hydrolysis-polycondensation reaction). The volume average particle diameter of the obtained silica-coated spherical elastomer particles (elastomer composite particles 8) was about 5 μm.

[Preparation/production example 9: Elastomer composite particles 9]

Elastomer composite particles were produced using the same procedure as in the preparation/production example 7 except that the spherical elastomer particles 3 were replaced with the spherical elastomer particles 4 and that the amount of the methyltrimethoxysilane added was changed from 12.5 g (this amount corresponds to 22.0 parts of silane based on 100 parts of the spherical elastomer particles after the hydrolysis-polycondensation reaction) to 0.51 g (this amount corresponds to 0.9 parts of silane based on 100 parts of the spherical elastomer particles after the hydrolysis-polycondensation reaction). The volume average particle diameter of the polyorganosilsesquioxane-coated spherical elastomer particles (elastomer composite particles 9) was about 20 μm.

[Comparative example 3: Silica-coated mica]

Silica-coated mica: VELVETVEIL 310 (composite powder prepared by coating mica to 10% with silica having an average particle diameter of 300 nm) manufactured by JGC Catalysts and Chemicals Ltd. was used.

The particle diameter of the spherical silica or polyorganosilsesquioxane adhering to spherical elastomer particles so as to cover their surfaces in each example was as shown in the following table.

[Table 1-1]

| Elastomer composite particles | 1 | 2 | 3 | 4 | 5 | 6 | 8 |
|---|---|---|---|---|---|---|---|
| Particle diameter of silica [ nm] | 26 | 21 | 34 | 42 | 66 | 17 | 13 |
| Elastomer composite particles | 7 | 9 | | | | | |
| Particle diameter of polyorganosilsesquioxane [ nm] | 40 | 22 | | | | | |

(1) Evaluation of user experiences

The particles in the preparation examples 1 to 6, the preparation/production examples 1 to 9, and the comparative example 1 to 3 were added to cosmetics, and their user experiences were evaluated.

Water-in-oil type creams prepared in formulation examples 1 to 15 and formulation comparative examples 1 to 3 were each evaluated for various items including user experiences during application (spreadability and uniform adhesion to the skin) and user experiences after application (transparency, softness, and the ability to conceal pores and wrinkles).

Powder foundations prepared in formulation examples 16 to 30 and formulation comparative examples 4 to 6 were each evaluated for various items including moldability, user experiences during application (ease of picking up, spreadability, and uniform adhesion to the skin), and user experiences after application (softness and the ability to conceal pores and wrinkles).

Water-in-oil sunscreen creams prepared in formulation examples 31 to 45 and formulation comparative examples 7 to 9 were evaluated for user experiences during application (spreadability and uniform adhesion to the skin) and user experiences after application (transparency, the ability to conceal pores and wrinkles, and absence of a greasy feel).

Ten expert panelists evaluated their user experiences for each item. The evaluation was performed according to evaluation criteria shown in Table 1, and the results of the ten panelists were evaluated based on the average values according to judgement criteria shown below.

The results for the water-in-oil type (emulsified liquid) creams are as shown in Table 2 below, and the results for the powder foundations are as shown in Table 3 below. The results for the water-in-oil type sunscreen creams are as shown in Table 4 below.

[Table 1]

| Item | User experience rating |
|---|---|
| 5 points | Good |
| 4 points | Fairly good |
| 3 points | Fair |
| 2 points | Fairly poor |
| 1 point | Poor |

(2) Judgement criteria for user experience

◎: The average is 4.0 or higher.
◦: The average is 3.0 or higher and lower than 4.0.
Δ: The average is 2.0 or higher and lower than 3.0.
×: The average is lower than 2.0.

[Table 2-1]

Formulation examples 1 to 15 and formulation comparative examples 1 to 3: Water-in-oil type creams

| Component | Mass (%) |
|---|---|
| 1. KSG-210 (*1) | 3.5 |
| 2. KSG-15 (*2) | 3.0 |
| 3. KF-6017 (*3) | 0.5 |
| 4. Dimethicone (6 cs) | 5.0 |
| 5. Cyclopentasiloxane | 6.5 |
| 6. One of preparation examples 1 to 6, preparation/production examples 1 to 9, and comparative examples 1 to 3 | 5.0 |
| 7. BG | 5.5 |
| 8. Na citrate | 0.2 |
| 9. Na chloride | 0.5 |

(continued)

| Formulation examples 1 to 15 and formulation comparative examples 1 to 3: Water-in-oil type creams | |
|---|---|
| Component | Mass (%) |
| 10. Water | 70.3 |
| Total | 100.0 |

(*1) Product of Shin-Etsu Chemical Co., Ltd.: Mixture of dimethicone 70 to 80% + (dimethicone/(PEG-10/15)) crosspolymer 20 to 30%
(*2) Product of Shin-Etsu Chemical Co., Ltd.: Mixture of cyclopentasiloxane 90 to 96% + (dimethicone/vinyl dimethicone) crosspolymer 4 to 10%
(*3) Product of Shin-Etsu Chemical Co., Ltd.: PEG-10 dimethicone

(Production method)

A: Components 1 to 6 were mixed uniformly.
B: Components 7 to 10 were mixed uniformly.
C: The mixture obtained in the step B was added to the mixture obtained in the step A, and the resulting mixture was stirred until uniform.
D: The resulting mixture obtained in the step C was defoamed and charged into a container, and a water-in-oil type cream was thereby obtained.

[Table 2]

| | User experiences during application | | User experiences after application | | |
|---|---|---|---|---|---|
| | Spreadability | Uniform adhesion to skin | Transparency | Softness | Ability to conceal pores and wrinkles (skin texture irregularity correction) |
| Formulation example 1 (preparation example 1 added) | ○ | ○ | ◎ | ◎ | ◎ |
| Formulation example 2 (preparation example 2 added) | Δ | Δ | ○ | ◎ | ◎ |
| Formulation example 3 (preparation example 3 added) | ○ | ○ | ◎ | ◎ | ◎ |
| Formulation example 4 (preparation example 4 added) | Δ | Δ | ○ | ◎ | ○ |
| Formulation example 5 (preparation example 5 added) | ◎ | ◎ | ○ | ○ | ○ |
| Formulation example 6 (preparation example 6 added) | Δ | ○ | ◎ | ◎ | ◎ |
| Formulation example 7 (preparation/production example 1 added) | ◎ | ◎ | ◎ | ◎ | ◎ |

(continued)

| | User experiences during application | | User experiences after application | | |
|---|---|---|---|---|---|
| | Spreadability | Uniform adhesion to skin | Transparency | Softness | Ability to conceal pores and wrinkles (skin texture irregularity correction) |
| Formulation example 8 (preparation/production example 2 added) | ○ | ○ | ○ | ◎ | ◎ |
| Formulation example 9 (preparation/production example 3 added) | ◎ | ◎ | ◎ | ◎ | ◎ |
| Formulation example 10 (preparation/production example 4 added) | ○ | ○ | ○ | ◎ | ○ |
| Formulation example 11 (preparation/production example 5 added) | ◎ | ◎ | ○ | ○ | ◎ |
| Formulation example 12 (preparation/production example 6 added) | ◎ | ○ | ◎ | ◎ | ◎ |
| Formulation example 13 (preparation/production example 7 added) | ◎ | ◎ | ◎ | ◎ | ◎ |
| Formulation example 14 (preparation/production example 8 added) | ◎ | ○ | ◎ | ◎ | ◎ |
| Formulation example 15 (preparation/production example 9 added) | ◎ | ○ | ◎ | ◎ | ◎ |
| Formulation comparative example 1 (cellulose powder added) | Δ | × | × | × | Δ |
| Formulation comparative example 2 (spherical silica added) | ○ | ○ | Δ | × | ○ |
| Formulation comparative example 3 (silica-coated mica added) | ○ | ○ | × | × | Δ |

As can be seen from the results in Table 2, the formulation examples 1 to 15 were effective in obtaining a transparent finish after application, in obtaining a soft coating film, and in skin texture correction. In particular, in the formulation examples 7 to 15, spreadability during application and the ability to adhere to the skin uniformly were particularly good.

However, in the formulation comparative example 1 to 3, the transparency after the application, the softness of the coating film, and the skin texture correction effect were poorer.

[Table 3-1]

| Formulation examples 16 to 30 and formulation comparative examples 4 to 6: Powder foundations | |
|---|---|
| Component | Mass (%) |
| 1. One of preparation examples 1 to 6, preparation/production examples 1 to 9, and comparative examples 1 to 3 | 7.0 |
| 2. Zinc stearate | 2.0 |
| 3. AES-3083 (*1)-treated mica | 30.0 |
| 4. AES-3083 (*1)-treated talc | 42.9 |
| 5. KTP-09W, R, Y, B (*2) | 9.6 |
| 6. Triethylhexanoin | 4.5 |
| 7. Dipentaerythrityl hexa(hydroxystearate/stearate/rosinate) | 0.5 |
| 8. KF-6038 (*3) | 0.5 |
| 9 .KF-56A (*4) | 1.0 |
| 10. Dimethicone (100 cs) | 2.0 |
| Total | 100.0 |

(*1) Product of Shin-Etsu Chemical Co., Ltd.: Triethoxycaprylylsilane
(*2) Product of Shin-Etsu Chemical Co., Ltd.: KF-9909-treated color inorganic pigment, W: white - R: red - Y: yellow - B: black
(*3) Product of Shin-Etsu Chemical Co., Ltd.: Lauryl PEG-9 polydimethylsiloxyethyl dimethicone
(*4) Product of Shin-Etsu Chemical Co., Ltd.: Diphenylsiloxy phenyl trimethicone

(Production method)

A: Components 1 to 5 were mixed uniformly using a Henschel mixer.
B: Components 6 to 10 were mixed uniformly.
C: The mixture obtained in the step B was added to the mixture obtained in the step A, and the resulting mixture was stirred until uniform using a Henschel mixer.
D: The mixture obtained in the step C was caused to pass through a sieve, and then the resulting mixture was pressed into a metal pan using a mold to thereby obtain a powder foundation.

[Table 3]

| | Moldability | User experiences during application | | | User experiences after application | |
|---|---|---|---|---|---|---|
| | | Ease of picking up | Spreadability | Uniform adhesion to skin | Softness | Ability to conceal pores and wrinkles (skin texture irregularity correction) |
| Formulation example 16 (preparation example 1 added) | ○ | ○ | ○ | ○ | ◎ | ◎ |
| Formulation example 17 (preparation example 2 added) | ○ | ○ | Δ | ○ | ◎ | ◎ |
| Formulation example 18 (preparation example 3 added) | ○ | ○ | ○ | ○ | ◎ | ◎ |

(continued)

| | Moldability | User experiences during application | | | User experiences after application | |
|---|---|---|---|---|---|---|
| | | Ease of picking up | Spreadability | Uniform adhesion to skin | Softness | Ability to conceal pores and wrinkles (skin texture irregularity correction) |
| Formulation example 19 (preparation example 4 added) | Δ | Δ | ○ | ○ | ◎ | ◎ |
| Formulation example 20 (preparation example 5 added) | ○ | ○ | ○ | ○ | ○ | ◎ |
| Formulation example 21 (preparation example 6 added) | ○ | Δ | Δ | Δ | ◎ | ◎ |
| Formulation example 22 (preparation/production example 1 added) | ◎ | ◎ | ◎ | ◎ | ◎ | ◎ |
| Formulation example 23 (preparation/production example 2 added) | ◎ | ◎ | ○ | ◎ | ◎ | ◎ |
| Formulation example 24 (preparation/production example 3 added) | ◎ | ◎ | ◎ | ◎ | ◎ | ◎ |
| Formulation example 25 (preparation/production example 4 added) | ○ | ○ | ◎ | ○ | ◎ | ○ |
| Formulation example 26 (preparation/production example 5 added) | ◎ | ◎ | ◎ | ◎ | ○ | ◎ |
| Formulation example 27 (preparation/production example 6 added) | ◎ | ○ | ○ | ○ | ◎ | ◎ |
| Formulation example 28 (preparation/production example 7 added) | ◎ | ◎ | ◎ | ◎ | ◎ | ◎ |
| Formulation example 29 (preparation/production example 8 added) | ○ | ○ | ◎ | ◎ | ◎ | ◎ |
| Formulation example 30 (preparation/production example 9 added) | ○ | ○ | ◎ | ◎ | ◎ | ◎ |
| Formulation comparative example 4 (cellulose powder added) | Δ | × | Δ | × | × | × |
| Formulation comparative example 5 (Spherical silica added) | ○ | Δ | ○ | Δ | × | Δ |

(continued)

| | Moldability | User experiences during application | | | User experiences after application | |
|---|---|---|---|---|---|---|
| | | Ease of picking up | Spreadability | Uniform adhesion to skin | Softness | Ability to conceal pores and wrinkles (skin texture irregularity correction) |
| Formulation comparative example 6 (Silica-coated mica added) | ○ | ○ | Δ | ○ | × | Δ |

As can be seen from the results in Table 3, the formulation examples 16 to 30 were effective in obtaining a soft coating film after application and in skin texture correction. In particular, in the formulation examples 22 to 30, the moldability, the spreadability during application, and the ability to adhere to the skin uniformly were particularly good.

However, in the formulation comparative examples 4 to 6, the softness of the coating film after application and the skin texture correction effect were poorer.

[Table 4-1]

Formulation examples 31 to 45 and formulation comparative examples 7 to 9:
Water-in-oil type sunscreen creams

| Component | Mass (%) |
|---|---|
| 1. KSG-240 (*1) | 2.0 |
| 2. KSG-18A (*2) | 2.0 |
| 3. KF-6048 (*3) | 1.5 |
| 4. Cyclopentasiloxane | 8.0 |
| 5. Isotridecyl isononanoate | 3.0 |
| 6. Disteardimonium hectorite | 0.8 |
| 7. Ethylhexyl methoxycinnamate | 7.0 |
| 8. Diethylamino hydroxybenzoyl hexyl benzoate | 2.0 |
| 9. KF-56A (*4) | 5.0 |
| 10. One of preparation examples 1 to 6, preparation/production examples 1 to 9, and comparative examples 1 to 3 | 3.0 |
| 11. SPD-T7 (*5) | 12.0 |
| 12. SPD-Z5 (*6) | 12.0 |
| 13. BG | 3.0 |
| 14. Ethanol | 5.0 |
| 15. Na citrate | 0.2 |
| 16. Na chloride | 0.5 |
| 17. Water | 34.0 |
| Total | 100.0 |

(*1) Product of Shin-Etsu Chemical Co., Ltd.: Mixture of cyclopentasiloxane 75 to 85% + (dimethicone/(-PEG-10/15)) crosspolymer 15 to 25%
(*2) Product of Shin-Etsu Chemical Co., Ltd.: Mixture of diphenylsiloxy phenyl trimethicone 80 to 90% + (dimethicone/phenyl vinyl dimethicone) crosspolymer 10 to 20%
(*3) Product of Shin-Etsu Chemical Co., Ltd.: Cetyl PEG/PPG-10/1 dimethicone
(*4) Product of Shin-Etsu Chemical Co., Ltd.: Diphenylsiloxy phenyl trimethicone
(*5) Product of Shin-Etsu Chemical Co., Ltd.: 45% dispersion of fine titanium oxide particles in cyclopentasiloxane solvent
(*6) Product of Shin-Etsu Chemical Co., Ltd.: 60% dispersion of fine zinc oxide particles in cyclopentasiloxane solvent

(Production method)

A: Components 1 to 10 were mixed uniformly.
B: Components 13 to 17 were mixed uniformly.
C: The mixture obtained in the step B was added to the mixture obtained in the step A, and the resulting mixture was stirred until uniform.
D: Components 11 to 12 were added to the mixture obtained in the step C, and the resulting mixture was stirred until uniform.
E: The mixture obtained in the step D was defoamed and then charged into a container to thereby obtain a water-in-oil type sunscreen cream.

[Table 4]

| | User experiences during application | | User experiences after application | | |
|---|---|---|---|---|---|
| | Spreadability | Uniform adhesion to skin | Transparency | Ability to conceal pores and wrinkles (skin texture irregularity correction) | Absence of greasy feel |
| Formulation example 31 (preparation example 1 added) | ○ | ○ | ◎ | ◎ | ◎ |
| Formulation example 32 (preparation example 2 added) | Δ | Δ | ○ | ◎ | ○ |
| Formulation example 33 (preparation example 3 added) | ○ | ○ | ◎ | ◎ | ◎ |
| Formulation example 34 (preparation example 4 added) | Δ | Δ | ○ | ◎ | ◎ |
| Formulation example 35 (preparation example 5 added) | ◎ | ◎ | ○ | ○ | ○ |
| Formulation example 36 (preparation example 6 added) | Δ | ○ | ◎ | ◎ | ◎ |
| Formulation example 37 (preparation/production example 1 added) | ◎ | ◎ | ◎ | ◎ | ◎ |
| Formulation example 38 (preparation/production example 2 added) | ○ | ○ | ○ | ◎ | ○ |
| Formulation example 39 (preparation/production example 3 added) | ◎ | ◎ | ◎ | ◎ | ◎ |
| Formulation example 40 (preparation/production example 4 added) | ○ | ○ | ○ | ◎ | ◎ |

(continued)

| | User experiences during application | | User experiences after application | | |
|---|---|---|---|---|---|
| | Spreadability | Uniform adhesion to skin | Transparency | Ability to conceal pores and wrinkles (skin texture irregularity correction) | Absence of greasy feel |
| Formulation example 41 (preparation/production example 5 added) | ◎ | ◎ | ○ | ○ | ○ |
| Formulation example 42 (preparation/production example 6 added) | ◎ | ○ | ◎ | ◎ | ◎ |
| Formulation example 43 (preparation/production example 7 added) | ◎ | ◎ | ◎ | ◎ | ◎ |
| Formulation example 44 (preparation/production example 8 added) | ◎ | ○ | ◎ | ◎ | ◎ |
| Formulation example 45 (preparation/production example 9 added) | ◎ | ○ | ◎ | ◎ | ◎ |
| Formulation comparative example 7 (cellulose powder added) | Δ | × | × | Δ | × |
| Formulation comparative example 8 (spherical silica added) | Δ | Δ | Δ | ○ | Δ |
| Formulation comparative example 9 (silica-coated mica added) | Δ | ○ | × | Δ | × |

As can be seen from the results in Table 4, the formulation examples 31 to 45 were effective in obtaining a transparent finish after application, in skin texture correction, and in preventing a greasy feel. In particular, in the formulation examples 37 to 45, spreadability during application and the ability to adhere to the skin uniformly were particularly good.

However, in the formulation comparative examples 7 to 9, the coating film did not exhibit a transparent finish after application, and the skin texture correction effect and the ability to prevent a greasy feel were poorer.

[Table 5]

| Formulation example 46: Water-based gel | |
|---|---|
| Component | Mass (%) |
| 1. Elastomer composite particles obtained in preparation/production example 1 | 5.0 |
| 2. KF-6100 (*1) | 0.5 |
| 3. Ethanol | 3.0 |
| 4. BG | 4.0 |
| 5. Glycerin | 2.0 |
| 6. (Ammonium acryloyldimethyltaurate/VP) copolymer | 0.2 |
| 7. Xanthan gum | 0.2 |
| 8. Arginine | 0.5 |
| 9. Preservative | Optimum amount |
| 10. Water | Remainder |

(continued)

| Formulation example 46: Water-based gel | |
|---|---|
| Component | Mass (%) |
| Total | 100.0 |
| (*1) Product of Shin-Etsu Chemical Co., Ltd.: Polyglyceryl-3 disiloxane dimethicone | |

(Production method)

A: Components 1 to 3 were mixed uniformly.
B: Components 4 to 10 were mixed uniformly.
C: The mixture obtained in the step A was added to the mixture obtained in the step B, and the resulting mixture was stirred until uniform.
D: The mixture obtained in the step C was defoamed and charged into a container to thereby obtain a water-based gel.

The water-based gel in the present invention obtained in the manner described above provided a highly moist texture upon application, spread easily with no stickiness, exhibited good adhesion, settled well, and provided a matte finish with reduced shine.

[Table 6]

| Formulation example 47: Oil-based gel | |
|---|---|
| Component | Mass (%) |
| 1. Spherical elastomer particles obtained in preparation example 1 | 10.0 |
| 2. KSG-19 (* 1) | 20.0 |
| 3. KSG-15 (*2) | 30.0 |
| 4. KF-56A (*3) | 5.0 |
| 5. Dimethicone (1.5 cs) | Remainder |
| Total | 100.0 |

(*1) Product of Shin-Etsu Chemical Co., Ltd.: Mixture of dimethicone 80 to 90% + (dimethicone/vinyl dimethicone) crosspolymer 10 to 20%)
(*2) Product of Shin-Etsu Chemical Co., Ltd.: Mixture of cyclopentasiloxane 90 to 96% + (dimethicone/vinyl dimethicone) crosspolymer 4 to 10%
(*3) Product of Shin-Etsu Chemical Co., Ltd.: Diphenylsiloxy phenyl trimethicone

(Production method)

A: Components 1 to 5 were mixed uniformly.
B: The mixture obtained in the step A was defoamed and then charged into a container to thereby obtain an oil-based gel.

The oil-based gel in the present invention obtained in the manner described above was very smooth during application, spread easily with no stickiness, exhibited good adhesion, settled well, and provided a matte finish with reduced shine.

[Table 7]

| Formulation example 48: Water-in-oil type cream | |
|---|---|
| Component | Mass (%) |
| 1. KSG-310 (*1) | 3.0 |
| 2. KSG-44 (*2) | 1.0 |
| 3. KF-6048 (*3) | 0.2 |

(continued)

| Formulation example 48: Water-in-oil type cream | |
| --- | --- |
| Component | Mass (%) |
| 4. Squalane | 10.8 |
| 5. Spherical elastomer particles obtained in preparation example 3 | 1.0 |
| 6. BG | 8.0 |
| 7. Ethanol | 5.0 |
| 8. Mg sulfate | 0.2 |
| 9. Na chloride | 0.5 |
| 10. Water | Remainder |
| Total | 100.0 |

(*1) Product of Shin-Etsu Chemical Co., Ltd.: Mixture of mineral oil 65 to 75% + (PEG-15/lauryl dimethicone) crosspolymer 25 to 35%
(*2) Product of Shin-Etsu Chemical Co., Ltd.: Mixture of squalane 65 to 75% + (vinyl dimethicone/lauryl dimethicone) crosspolymer 25 to 35%
(*3) Product of Shin-Etsu Chemical Co., Ltd.: Cetyl PEG/PPG-10/1 dimethicone

(Production method)

A: Components 1 to 5 were mixed uniformly.
B: Components 6 to 10 were mixed uniformly.
C: The mixture obtained in the step B was added to the mixture obtained in the step A, and the resulting mixture was stirred until uniform.
D: The mixture obtained in the step C was defoamed and then charged into a container to thereby obtain a water-in-oil type cream.

The water-in-oil type cream in the present invention obtained in the manner described above was very smooth during application, spread easily with no stickiness, exhibited good adhesion, settled well, and provided a natural finish with reduced oily shine.

[Table 8]

| Formulation example 49: Water-in-oil type cream | |
| --- | --- |
| Component | Mass (%) |
| 1. Dimethicone (6 cs) | 6.0 |
| 2. KF-54 (*1) | 4.0 |
| 3. Neopentyl glycol dioctanoate | 3.0 |
| 4. KF-6012 (*2) | 3.0 |
| 5. Elastomer composite particles obtained in preparation/production example 2 | 3.0 |
| 6. Glycerin | 10.0 |
| 7. Preservative | Optimum amount |
| 8. Essential oil | Optimum amount |
| 9. Water | Remainder |
| Total | 100.0 |

(*1) Product of Shin-Etsu Chemical Co., Ltd.: Diphenyl dimethicone
(*2) Product of Shin-Etsu Chemical Co., Ltd.: PEG/PPG-20/22 butyl ether dimethicone

(Production method)

A: Components 1 to 5 were mixed uniformly.
B: Components 6 to 7 and 9 were mixed until dissolution.
C: The mixture obtained in the step B was added to the mixture obtained in the step A, and the resulting mixture was stirred until uniform.
D: Component 8 was added to the mixture obtained in the step C, and the resulting mixture was stirred until uniform.
E: The mixture obtained in the step D was defoamed and then charged into a container to thereby obtain a water-in-oil type cream.

The water-in-oil type cream obtained in the manner described above was fine and smooth, spread easily with no stickiness and no greasy feel, and was found to be highly stable, showing no deterioration under varying temperatures and over time.

[Table 9]

| Formulation example 50: Water-in-oil type cream | |
|---|---|
| Component | Mass (%) |
| 1. KSG-340 (*1) | 6.0 |
| 2. Mineral oil | 8.5 |
| 3. Macadamia nut oil | 5.0 |
| 4. KF-6105 (*2) | 0.5 |
| 5. KSP-101 (*3) | 3.0 |
| 6. Elastomer composite particles obtained in preparation/production example 5 | 4.0 |
| 7. Na citrate | 0.2 |
| 8. PG | 8.0 |
| 9. Glycerin | 3.0 |
| 10. Preservative | Optimum amount |
| 11. Fragrance | Optimum amount |
| 12. Water | Remainder |
| Total | 100.0 |

(*1) Product of Shin-Etsu Chemical Co., Ltd.: Mixture of squalane 65 to 75% + (PEG-10/lauryl dimethicone) crosspolymer and (PEG-15/lauryl dimethicone) crosspolymer 25 to 35%
(*2) Product of Shin-Etsu Chemical Co., Ltd.: Lauryl polyglyceryl-3 polydimethylsiloxyethyl dimethicone
(*3) Product of Shin-Etsu Chemical Co., Ltd.: (Vinyl dimethicone/methicone silsesquioxane) crosspolymer

(Production method)

A: Components 1 to 6 were mixed.
B: Components 7 to 10 and 12 were mixed until dissolution.
C: The mixture obtained in the step B was added to the mixture obtained in the step A, and the resulting mixture was stirred until uniform.
D: Component 11 was added to the mixture obtained in the step C, and the resulting mixture was stirred until uniform.
E: The mixture obtained in the step D was defoamed and then charged into a container to thereby obtain a water-in-oil type cream.

The water-in-oil type cream obtained in the manner described above was fine and smooth, spread easily with no stickiness and no greasy feel, and was found to be highly stable, showing no deterioration under varying temperatures and over time.

[Table 10]

| Formulation example 51: Water-in-oil type cream | |
|---|---|
| Component | Mass (%) |
| 1. Cyclopentasiloxane | 10.5 |
| 2. Dimethicone (6 cs) | 4.0 |

(continued)

| Formulation example 51: Water-in-oil type cream | |
|---|---|
| Component | Mass (%) |
| 3. KF-6028 (*1) | 3.0 |
| 4. Octyldodeceth-5 | 1.0 |
| 5. Polysorbate 60 | 0.5 |
| 6. Elastomer composite particles obtained in preparation/production example 3 | 15.0 |
| 7. Mineral oil | 2.0 |
| 8. Macadamia nut oil | 1.0 |
| 9. Scutellaria baicalensis extract (*2) | 1.0 |
| 10. Gentiana lutea extract (*3) | 0.5 |
| 11. Ethanol | 3.0 |
| 12. BG | 4.0 |
| 13. Preservative | Optimum amount |
| 14. Water | Remainder |
| Total | 100.0 |

(*1) Product of Shin-Etsu Chemical Co., Ltd.: PEG-9 polydimethylsiloxyethyl dimethicone
(*2) Scutellaria baicalensis extract: Extracted with 50% BG water
(*3) Gentiana lutea extract: Extracted with 20% ethanol water

(Production method)

A: Components 1 to 8 were mixed.
B: Components 9 to 14 were mixed until dissolution.
C: The mixture obtained in the step B was added to the mixture obtained in the step A, and the resulting mixture was stirred until uniform.
D: The mixture obtained in the step C was defoamed and then charged into a container to thereby obtain a water-in-oil type cream.

The water-in-oil type cream obtained in the manner described above was fine and smooth, exhibited no stickiness, spread easily, exhibited good adhesion, had significant long-lasting makeup efficacy, and was found to be highly stable, showing no deterioration under varying temperatures and over time.

[Table 11]

| Formulation example 52: Oil-in-water type cream | |
|---|---|
| Component | Mass (%) |
| 1. KSG-43 (*1) | 5.0 |
| 2. Triethylhexanoin | 5.0 |
| 3. Elastomer composite particles obtained in preparation/production example 7 | 11.0 |
| 4. DPG | 7.0 |
| 5. Glycerin | 5.0 |
| 6. METOLOSE SM-4000 (*2) (2% aqueous solution) | 7.0 |
| 7. Polyacrylamide-based emulsifier (*3) | 2.0 |
| 8. Phenoxyethanol | Optimum amount |
| 9. Fragrance | Optimum amount |
| 10. Water | Remainder |

(continued)

| Formulation example 52: Oil-in-water type cream | |
|---|---|
| Component | Mass (%) |
| Total | 100.0 |
| (*1) Product of Shin-Etsu Chemical Co., Ltd.: Mixture of triethylhexanoin 65 to 75% + (vinyl dimethicone/lauryl dimethicone) crosspolymer 25 to 35%<br>(*2) Product of Shin-Etsu Chemical Co., Ltd.: Methyl cellulose<br>(*3) Product of SEPPIC: SEPIGEL 305 | |

(Production method)

A: Components 1 to 3 were mixed.
B: Components 4 to 10 were mixed.
C: The mixture obtained in the step B was added to the mixture obtained in the step A, and the resulting mixture was stirred until uniform.
D: The mixture obtained in the step C was defoamed and then charged into a container to thereby obtain an oil-in-water type cream.

The oil-in-water type cream obtained in the manner described above was fine and smooth, spread easily with no stickiness and no greasy feel, and was found to be highly stable, showing no deterioration under varying temperatures and over time.

[Table 12]

| Formulation example 53: Water-in-oil type sunscreen milky lotion | |
|---|---|
| Component | Mass (%) |
| 1. KSG-270 (*1) | 3.5 |
| 2. KSG-18A (*2) | 3.0 |
| 3. KF-6048 (*3) | 0.5 |
| 4. KF-56A (*4) | 5.0 |
| 5. KF-4418 (*5) | 7.5 |
| 6. KF-4422 (*6) | 6.5 |
| 7. Ethylhexyl triazone | 5.0 |
| 8. Ethylhexyl salicylate | 2.0 |
| 9. Octocrylene | 3.0 |
| 10. Bis-ethylhexyloxyphenol methoxyphenyl triazine | 1.0 |
| 11. Spherical elastomer particles obtained in preparation example 4 | 4.0 |
| 12. BG | 5.5 |
| 13. Na citrate | 0.2 |
| 14. Na chloride | 0.5 |
| 15. Water | Remainder |
| Total | 100.0 |
| (*1) Product of Shin-Etsu Chemical Co., Ltd.: Mixture of diphenylsiloxy phenyl trimethicone 75 to 85% + (dimethicone/(PEG-10/15)) crosspolymer 15 to 25%<br>(*2) Product of Shin-Etsu Chemical Co., Ltd.: Mixture of diphenylsiloxy phenyl trimethicone 80 to 90% + (dimethicone/phenyl vinyl dimethicone) crosspolymer 10 to 20%<br>(*3) Product of Shin-Etsu Chemical Co., Ltd.: Cetyl PEG/PPG-10/1 dimethicone<br>(*4) Product of Shin-Etsu Chemical Co., Ltd.: Diphenylsiloxy phenyl trimethicone<br>(*5) Product of Shin-Etsu Chemical Co., Ltd.: Caprylyl methicone<br>(*6) Product of Shin-Etsu Chemical Co., Ltd.: Ethyl Trimethicone | |

(Production method)

A: Components 1 to 11 were mixed uniformly.
B: Components 12 to 15 were mixed uniformly.
C: The mixture obtained in the step B was added to the mixture obtained in the step A, and the resulting mixture was stirred until uniform.
D: The mixture obtained in the step C was defoamed and then charged into a container to thereby obtain a water-in-oil type sunscreen milky lotion.

The sunscreen milky lotion in the present invention obtained in the manner described above was very smooth during application, provided a finish with reduced shine, had enhanced long-lasting makeup efficacy, and was found to be a very good sunscreen milky lotion.

[Table 13]

| Formulation example 54: Water-in-oil type sunscreen cream | |
|---|---|
| Component | Mass (%) |
| 1. KSG-270 (*1) | 5.0 |
| 2. KF-6105 (*2) | 2.5 |
| 3. Cyclopentasiloxane | 11.5 |
| 4. Ethylhexyl palmitate | 5.0 |
| 5. Ethylhexyl methoxycinnamate | 7.0 |
| 6. Homosalate | 3.0 |
| 7. KP-550 (*3) | 12.0 |
| 8. Tocopherol | 0.1 |
| 9. Spherical elastomer particles obtained in preparation example 6 | 8.0 |
| 10. AES-3083 (*4)-treated fine zinc oxide particles | 15.0 |
| 11. Na chloride | 0.5 |
| 12. BG | 2.0 |
| 13. Preservative | Optimum amount |
| 14. Fragrance | Optimum amount |
| 15. Water | Remainder |
| Total | 100.0 |

(*1) Product of Shin-Etsu Chemical Co., Ltd.: Mixture of diphenylsiloxy phenyl trimethicone 75 to 85% + (dimethicone/(PEG-10/15)) crosspolymer 15 to 25%
(*2) Product of Shin-Etsu Chemical Co., Ltd.: Lauryl polyglyceryl-3 polydimethylsiloxyethyl dimethicone
(*3) Product of Shin-Etsu Chemical Co., Ltd.: Dissolution product of isododecane 60% + (acrylates/dimethicone) copolymer 40%
(*4) Product of Shin-Etsu Chemical Co., Ltd.: Triethoxycaprylylsilane

(Production method)

A: Component 3 was added to part of component 2, and the mixture was stirred until uniform. Then component 10 was added and dispersed using a bead mill.
B: The rest of the component 2, component 1, and components 4 to 9 were mixed uniformly.
C: Components 11 to 13 and component 15 were mixed until dissolution.
D: The mixture obtained in the step C was added to the mixture obtained in the step B, and the resulting mixture was stirred until uniform.
E: The mixture obtained in the step A was added to the mixture obtained in the step D, and the resulting mixture was stirred until uniform.
F: Component 14 was added to the mixture obtained in the step E, and the resulting mixture was stirred until uniform.

G: The mixture obtained in the step F was defoamed and then charged into a container to thereby obtain a water-in-oil type sunscreen cream.

The sunscreen cream obtained in the manner described above was not sticky, spread easily, exhibited good adhesion, had a skin texture correcting effect, and had very enhanced long-lasting makeup efficacy. Moreover, the sunscreen cream was found to be highly stable, showing no deterioration under varying temperatures and over time.

[Table 14]

| Formulation example 55: Water-in-oil type sunscreen milky lotion | |
|---|---|
| Component | Mass (%) |
| 1. Cyclopentasiloxane | 20.0 |
| 2. KF-6038 (*1) | 0.5 |
| 3. KF-56A (*2) | 3.0 |
| 4. Sorbitan isostearate | 1.0 |
| 5. X-21-5250 (*3) | 1.0 |
| 6. Elastomer composite particles obtained in preparation/production example 8 | 6.0 |
| 7. KSP-105 (*4) | 2.0 |
| 8. Isostearic acid-treated fine titanium oxide particles | 10.0 |
| 9. Isostearic acid-treated fine zinc oxide particles | 10.0 |
| 10. Sorbitol | 2.0 |
| 11. Na chloride | 2.0 |
| 12. Preservative | Optimum amount |
| 13. Fragrance | Optimum amount |
| 14. Water | Remainder |
| Total | 100.0 |

(*1) Product of Shin-Etsu Chemical Co., Ltd.: Lauryl PEG-9 polydimethylsiloxyethyl dimethicone
(*2) Product of Shin-Etsu Chemical Co., Ltd.: Diphenylsiloxy phenyl trimethicone
(*3) Product of Shin-Etsu Chemical Co., Ltd.: Dissolution product of cyclopentasiloxane 50% + trimethylsiloxysilicate 50%
(*4) Product of Shin-Etsu Chemical Co., Ltd.: (Vinyl dimethicone/methicone silsesquioxane) crosspolymer

(Production method)

A: Component 2 was added to component 1, and the mixture was stirred until uniform. Then components 8 to 9 were added and dispersed using a bead mill.
B: Components 3 to 7 were mixed uniformly.
C: Components 10 to 12 and component 14 were mixed until dissolution.
D: The mixture obtained in the step C was added to the mixture obtained in the step B, and the resulting mixture was stirred until uniform.
E: The mixture obtained in the step A was added to the mixture obtained in the step D, and the resulting mixture was stirred until uniform.
F: Component 13 was added to the mixture obtained in the step E, and the resulting mixture was stirred until uniform.
G: The mixture obtained in the step F was defoamed and then charged into a container to thereby obtain a water-in-oil type sunscreen milky lotion.

The sunscreen milky lotion obtained in the manner described above was fine and smooth, spread easily, and was not sticky. Moreover, since the sunscreen milky lotion had enhanced long-lasting makeup efficacy, its ultraviolet protection effect lasted long. The sunscreen milky lotion was found to be very stable, showing no deterioration under varying temperatures and over time.

[Table 15]

| Formulation example 56: Water-in-oil type cream foundation | |
|---|---|
| Component | Mass (%) |
| 1. KSG-710 (*1) | 4.0 |
| 2. KSG-016F (*2) | 2.0 |
| 3 .KF-6104 (*3) | 3.0 |
| 4. Dimethicone (2 cs) | 12.0 |
| 5. Disteardimonium hectorite | 1.2 |
| 6. Spherical elastomer particles obtained in preparation example 5 | 2.0 |
| 7. KF-7312L (*4) | 5.0 |
| 8. Isotridecyl isononanoate | 2.0 |
| 9. KF-6106 (*5) | 0.5 |
| 10. KF-99P (*6)-treated inorganic color pigment | 10.0 |
| 11. Pentylene glycol | 5.0 |
| 12. Na citrate | 0.2 |
| 13. Na chloride | 0.5 |
| 14. Fragrance | Optimum amount |
| 15. Water | Remainder |
| Total | 100.0 |

(*1) Product of Shin-Etsu Chemical Co., Ltd.: Mixture of dimethicone 70 to 80% + (dimethicone/polyglycerin-3) crosspolymer 20 to 30%
(*2) Product of Shin-Etsu Chemical Co., Ltd.: Mixture of dimethicone 70 to 80% + (dimethicone/vinyl dimethicone) crosspolymer 20 to 30%
(*3) Product of Shin-Etsu Chemical Co., Ltd.: Polyglyceryl-3 polydimethylsiloxyethyl dimethicone
(*4) Product of Shin-Etsu Chemical Co., Ltd.: Dissolution product of dimethicone (2 cs) 50% + trimethylsiloxysilicate 50%
(*5) Product of Shin-Etsu Chemical Co., Ltd.: Polyglyceryl-3 polydimethylsiloxyethyl dimethicone
(*6) Product of Shin-Etsu Chemical Co., Ltd.: Methicone

(Production method)

A: Components 1 to 7 were mixed.
B: Components 8 to 10 were mixed and subjected to roll treatment.
C: The mixture obtained in the step B was added to the mixture obtained in the step A, and the resulting mixture was stirred until uniform.
D: Components 11 to 13 and component 15 were mixed until dissolution.
E: The mixture obtained in the step D was added to the mixture obtained in the step C, and the resulting mixture was stirred until uniform.
F: Component 14 was added to the mixture obtained in the step E, and the resulting mixture was stirred until uniform.
G: The mixture obtained in the step F was defoamed and then charged into a container to thereby obtain a water-in-oil type cream foundation.

The cream foundation obtained in the manner described above was excellent in smoothness during application and moisture retention, spread easily with no stickiness, exhibited good adhesion, settled well, and provided a natural finish with reduced shine.

[Table 16]

| Formulation example 57: Water-in-oil type cream foundation | |
|---|---|
| Component | Mass (%) |
| 1. Cyclopentasiloxane | 45.0 |
| 2. Dimethicone (6 cs) | 10.0 |

(continued)

| Formulation example 57: Water-in-oil type cream foundation | |
|---|---|
| Component | Mass (%) |
| 3. KF-6028P (*1) | 3.5 |
| 4. Disteardimonium hectorite | 1.5 |
| 5. Elastomer composite particles obtained in preparation/production example 4 | 14.5 |
| 6. Ethylhexyl palmitate | 5.0 |
| 7. KP-578 (*2) | 0.4 |
| 8. Mg stearate-treated inorganic color pigment | 8.6 |
| 9. DPG | 5.0 |
| 10. Preservative | Optimum amount |
| 11. Essential oil | Optimum amount |
| 12. Water | Remainder |
| Total | 100.0 |
| (*1) Product of Shin-Etsu Chemical Co., Ltd.: PEG-9 polydimethylsiloxyethyl dimethicone<br>(*2) Product of Shin-Etsu Chemical Co., Ltd.: (Acrylates/ethylhexyl acrylate/dimethicone methacrylate) copolymer | |

(Production method)

A: Components 1 to 5 were mixed.
B: Components 6 to 8 were mixed and subjected to roll treatment.
C: The mixture obtained in the step B was added to the mixture obtained in the step A, and the resulting mixture was stirred until uniform.
D: Components 9 to 10 and component 12 were mixed until dissolution.
E: The mixture obtained in the step D was added to the mixture obtained in the step C, and the resulting mixture was stirred until uniform.
F: Component 11 was added to the mixture obtained in the step E, and the resulting mixture was stirred until uniform.
G: The mixture obtained in the step F was defoamed and then charged into a container to thereby obtain a water-in-oil type cream foundation.

The cream foundation obtained in the manner described above was fine and smooth, spread easily with no stickiness and no greasy feel, had enhanced long-lasting makeup efficacy, and was found to be highly stable, showing no deterioration under varying temperatures and over time.

[Table 17]

| Formulation example 58: Water-in-oil type liquid foundation | |
|---|---|
| Component | Mass (%) |
| 1. Cyclopentasiloxane | 10.0 |
| 2. KF-56A (*1) | 4.5 |
| 3. Ethylhexyl methoxycinnamate | 5.0 |
| 4. KF-9021 (*2) | 1.0 |
| 5. KF-6105 (*3) | 1.0 |
| 6. Polyglyceryl-2 isostearate | 0.5 |
| 7. Elastomer composite particles obtained in preparation/production example 6 | 8.0 |
| 8. Neopentyl glycol dioctanoate | 3.0 |
| 9. Polyhydroxystearic acid | 1.0 |
| 10. Dimethicone-treated inorganic color pigment | 7.0 |
| 11. Glycerin | 3.0 |
| 12. Preservative | Optimum amount |
| 13. Water | Remainder |

(continued)

| Formulation example 58: Water-in-oil type liquid foundation | |
|---|---|
| Component | Mass (%) |
| Total | 100.0 |

(*1) Product of Shin-Etsu Chemical Co., Ltd.: Diphenylsiloxy phenyl trimethicone
(*2) Product of Shin-Etsu Chemical Co., Ltd.: Dissolution product of cyclopentasiloxane 50% + trimethylsiloxysilicate 50%
(*3) Product of Shin-Etsu Chemical Co., Ltd.: Lauryl polyglyceryl-3 polydimethylsiloxyethyl dimethicone

(Production method)

A: Components 1 to 7 were mixed.
B: Components 8 to 10 were mixed and subjected to roll treatment.
C: The mixture obtained in the step B was added to the mixture obtained in the step A, and the resulting mixture was stirred until uniform.
D: Components 11 to 13 were mixed until dissolution.
E: The mixture obtained in the step D was added to the mixture obtained in the step C, and the resulting mixture was stirred until uniform.
F: The mixture obtained in the step E was defoamed and then charged into a container to thereby obtain a water-in-oil type liquid foundation.

The liquid foundation obtained in the manner described above had a low viscosity, was fine and smooth, spread easily with no stickiness and no greasy feel, had a skin texture correcting effect, had enhanced long-lasting makeup efficacy, and was found to be highly stable, showing no deterioration under varying temperatures and over time.

[Table 18]

| Formulation example 59: Water-in-oil type liquid foundation | |
|---|---|
| Component | Mass (%) |
| 1. Cyclopentasiloxane | 15.0 |
| 2. KF-54 (*1) | 3.0 |
| 3. KF-6017 (*2) | 1.5 |
| 4. Polyglyceryl-2 diisostearate | 1.0 |
| 5. Elastomer composite particles obtained in preparation/production example 1 | 15.0 |
| 6. KMP-592 (*3) | 3.0 |
| 7. Triethylhexanoin | 10.0 |
| 8. KF-6115 (*4) | 1.0 |
| 9. Stearic acid-treated fine titanium oxide particles | 6.0 |
| 10. KF-9901 (*5)-treated inorganic color pigment | 9.0 |
| 11. BG | 7.0 |
| 12. Na chloride | 0.5 |
| 13. Preservative | Optimum amount |
| 14. Water | Remainder |
| Total | 100.0 |

(*1) Product of Shin-Etsu Chemical Co., Ltd.: Diphenyl dimethicone
(*2) Product of Shin-Etsu Chemical Co., Ltd.: PEG-10 dimethicone
(*3) Product of Shin-Etsu Chemical Co., Ltd.: (Methyl/phenyl) polysilsesquioxane
(*4) Product of Shin-Etsu Chemical Co., Ltd.: Lauryl polyglyceryl-3 polydimethylsiloxyethyl dimethicone
(*5) Product of Shin-Etsu Chemical Co., Ltd.: Hydrogen dimethicone

(Production method)

A: Components 1 to 7 were mixed.
B: Components 8 to 11 were mixed and subjected to roll treatment.
C: The mixture obtained in the step B was added to the mixture obtained in the step A, and the resulting mixture was stirred until uniform.
D: Components 12 to 14 were mixed until dissolution.
E: The mixture obtained in the step D was added to the mixture obtained in the step C, and the resulting mixture was stirred until uniform.
F:The mixture obtained in the step E was defoamed and then charged into a container to thereby obtain a water-in-oil type liquid foundation.

The liquid foundation obtained in the manner described above was not sticky, spread easily, exhibited good adhesion, and had significant long-lasting makeup efficacy. The liquid foundation was found to be highly stable, showing no deterioration under varying temperatures and over time.

[Table 19]

| Formulation example 60: Oil-in-water type base cream | |
|---|---|
| Component | Mass (%) |
| 1. Water | Remainder |
| 2. Glycerin | 3.0 |
| 3. Xanthan gum | 0.2 |
| 4. Pentylene glycol | 2.0 |
| 5. Sucrose cocoate | 0.2 |
| 6. Sorbitan stearate | 3.0 |
| 7. PEG-60 glyceryl isostearate | 0.5 |
| 8. Microcrystalline wax | 0.3 |
| 9. Behenyl alcohol | 0.5 |
| 10. Isononyl isononanoate | 6.0 |
| 11. Spherical elastomer particles obtained in preparation example 2 | 3.0 |
| 12. BG | 5.0 |
| 13. Silica-treated inorganic color pigment | 5.0 |
| 14. Polysorbate 60 | 0.3 |
| 15. (Hydroxyethyl acrylate/Na acryloyldimethyl taurate) copolymer | 0.6 |
| Total | 100.0 |

(Production method)

A: Components 1 to 7 were heated to 80°C and mixed uniformly.
B: Components 8 to 10 were heated to 80°C, and component 11 was added. Then the resulting mixture was stirred until uniform.
C: The heated mixture obtained in the step B was added to the heated mixture obtained in the step A, and the resulting mixture was stirred until uniform.
D: Components 12 to 13 were mixed and subjected to roll treatment.
E: The mixture obtained in the step C was cooled to room temperature. Then components 14 to 15 were added, and the resulting mixture was stirred until uniform.
F: The mixture obtained in the step D was added to the mixture obtained in the step E, and the resulting mixture was stirred until uniform.
G: The mixture obtained in the step F was defoamed and then charged into a container to thereby obtain an oil-in-water type base cream.

The base cream obtained in the manner described above provided a highly moist texture upon application, spread easily with no stickiness, exhibited good adhesion, settled well, and provided a matte finish with reduced shine.

[Table 20]

Formulation example 61: Oil-based cream foundation

| Component | Mass (%) |
|---|---|
| 1. Isododecane | Remainder |
| 2. KSG-42A (*1) | 10.0 |
| 3. KF-6104 (*2) | 4.0 |
| 4. TSPL-30-ID (*3) | 2.0 |
| 5. Dimethicone (6 cs) | 5.0 |
| 6. Ethanol | 8.0 |
| 7. Disteardimonium hectorite | 1.5 |
| 8. Silica silylate | 1.0 |
| 9. Spherical elastomer particles obtained in preparation example 1 | 6.0 |
| 10. Isotridecyl isononanoate | 10.0 |
| 11. KP-578 (*4) | 0.5 |
| 12. KF-9901 (*5)-treated fine titanium oxide particles | 8.0 |
| 13. KF-9901 (*5)-treated fine zinc oxide particles | 5.0 |
| 14. AES-3083 (*6)-treated inorganic color pigment | 7.5 |
| Total | 100.0 |

(*1) Product of Shin-Etsu Chemical Co., Ltd.: Mixture of isododecane 75 to 85% + (vinyl dimethicone/lauryl dimethicone) crosspolymer 15 to 25%
(*2) Product of Shin-Etsu Chemical Co., Ltd.: Polyglyceryl-3 polydimethylsiloxyethyl dimethicone
(*3) Product of Shin-Etsu Chemical Co., Ltd.: Dissolution product of tri(trimethylsiloxy)silylpropyl carbamoyl pullulan 30% + isododecane 70%
(*4) Product of Shin-Etsu Chemical Co., Ltd.: (Acrylates/ethylhexyl acrylate/dimethicone methacrylate) copolymer
(*5) Product of Shin-Etsu Chemical Co., Ltd.: Hydrogen dimethicone
(*6) Product of Shin-Etsu Chemical Co., Ltd.: Triethoxycaprylylsilane

(Production method)

A: Components 1 to 9 were mixed uniformly.
B: Components 10 to 14 were mixed uniformly and subjected to roll treatment.
C: The mixture obtained in the step B was added to the mixture obtained in the step A, and the resulting mixture was stirred until uniform.
D: The mixture obtained in the step C was defoamed and then charged into a container to thereby obtain an oil-based cream foundation.

The cream foundation obtained in the manner described above spread easily, settled well on the skin, provided a moist finish, formed a cosmetic film with reduced shine and enhanced tautness, had enhanced long-lasting makeup efficacy, and was a very good cream foundation.

[Table 21]

Formulation example 62: Oil-based mousse foundation

| Component | Mass (%) |
|---|---|
| 1. Dimethicone (6 cs) | Remainder |
| 2. KF-7312L (*1) | 10.0 |
| 3. KSG-048Z (*2) | 30.0 |
| 4. Squalane | 1.0 |
| 5. Jojoba oil | 1.0 |

(continued)

| Formulation example 62: Oil-based mousse foundation | |
|---|---|
| Component | Mass (%) |
| 6. KF-56A (*3) | 1.0 |
| 7. Elastomer composite particles obtained in preparation/production example 7 | 18.0 |
| 8. Methyl methacrylate crosspolymer | 1.0 |
| 9. Nylon-12 | 1.0 |
| 10. KTP-09W, R, Y, B (*4) | 10.0 |
| 11. Stearic acid-treated fine titanium oxide particles | 10.0 |
| 12. Tocopherol | Optimum amount |
| 13. Dimethicone-treated talc | 3.0 |
| 14. Dimethicone-treated mica | 5.0 |
| Total | 100.0 |

(*1) Product of Shin-Etsu Chemical Co., Ltd.: Dissolution product of dimethicone (2 cs) 50% + trimethylsiloxysilicate 50%
(*2) Product of Shin-Etsu Chemical Co., Ltd.: Mixture of dimethicone 75 to 85% + (lauryl polydimethylsiloxyethyl dimethicone/bis-vinyldimethicone) crosspolymer 15 to 25%
(*3) Product of Shin-Etsu Chemical Co., Ltd.: Diphenylsiloxy phenyl trimethicone
(*4) Product of Shin-Etsu Chemical Co., Ltd.: KF-9909-treated color inorganic pigment, W: white - R: red - Y: yellow - B: black

(Production method)

A: Part of component 1 and components 2 to 9 were mixed uniformly.
B: The rest of the component 1 was mixed with components 10 to 14, and the mixture was subjected to roll treatment.
C: The mixture obtained in the step B was added to the mixture obtained in the step A, and the resulting mixture was stirred until uniform.
D: The mixture obtained in the step C was defoamed and then charged into a container to thereby obtain an oil-based mousse foundation.

The mousse foundation obtained in the manner described above was in the form of a souffle, was easy to pick up, spread easily, and had a non-oily and non-powdery texture upon application. The water resistance, water repellency, and perspiration resistance of the mousse foundation were good, and the mousse foundation lasted long, was resistant to makeup smearing, and was found to be highly stable, showing no deterioration under varying temperatures and over time.

[Table 22]

| Formulation example 63: Oil-based solid foundation | |
|---|---|
| Component | Mass (%) |
| 1. Synthetic wax | 4.0 |
| 2. Carnauba wax | 2.0 |
| 3. Shea butter | 0.5 |
| 4. Caprylic/capric triglyceride | 3.0 |
| 5. KF-56A (*1) | 5.0 |
| 6. Ethylhexyl methoxycinnamate | 7.0 |
| 7. Bis-ethylhexyloxyphenol methoxyphenyl triazine | 0.5 |
| 8. Dimethicone (2 cs) | 5.0 |
| 9. Isotridecyl isononanoate | Remainder |
| 10. Spherical elastomer particles obtained in preparation/production example 6 | 4.0 |
| 11. KMP-591 (*2) | 1.0 |
| 12. Cetyl ethylhexanoate | 9.0 |
| 13. KF-6115 (*3) | 1.0 |

(continued)

| Formulation example 63: Oil-based solid foundation | |
|---|---|
| Component | Mass (%) |
| 14. KF-9901 (*4)-treated fine titanium oxide particles | 7.0 |
| 15. KTP-09W, R, Y, B (*5) | 11.0 |
| Total | 100.0 |

(*1) Product of Shin-Etsu Chemical Co., Ltd.: Diphenylsiloxy phenyl trimethicone
(*2) Product of Shin-Etsu Chemical Co., Ltd.: Polymethylsilsesquioxane
(*3) Product of Shin-Etsu Chemical Co., Ltd.: Lauryl polyglyceryl-3 polydimethylsiloxyethyl dimethicone
(*4) Product of Shin-Etsu Chemical Co., Ltd.: Hydrogen dimethicone
(*5) Product of Shin-Etsu Chemical Co., Ltd.: KF-9909-treated color inorganic pigment, W: white - R: red - Y: yellow - B: black

(Production method)

A: Components 1 to 9 were heated until dissolution.
B: Components 12 to 15 were mixed uniformly and subjected to roll treatment.
C: The mixture obtained in the step B and components 10 to 11 were added to the heated mixture obtained in the step A, and the resulting mixture was stirred until uniform.
D: The mixture obtained in the step C was defoamed under heating, then charged into a container, and cooled to room temperature to thereby obtain an oil-based solid foundation.

The solid foundation obtained in the manner described above spread easily, settled well on the skin, provided a moist finish, formed a cosmetic film with reduced shine and enhanced tautness, had enhanced long-lasting makeup efficacy, and was a very good foundation.

[Table 23]

| Formulation example 64: Powder foundation | |
|---|---|
| Component | Mass (%) |
| 1. Elastomer composite particles obtained in preparation/production example 5 | 9.0 |
| 2. KSP-411 (*1) | 1.0 |
| 3. Polyethylene | 1.5 |
| 4. Ba sulfate | 5.0 |
| 5. KF-9909 (*2)-treated mica | 40.0 |
| 6. KF-9909 (*2)-treated talc | Remainder |
| 7. KTP-09 W, R, Y, B (*3) | 15.0 |
| 8. Mineral oil | 2.0 |
| 9. Squalane | 2.0 |
| 10. KF-4418 (*4) | 4.0 |
| 11. Dimethicone (50 cs) | 1.0 |
| Total | 100.0 |

(*1) Product of Shin-Etsu Chemical Co., Ltd.: Polysilicone-1 crosspolymer
(*2) Product of Shin-Etsu Chemical Co., Ltd.: Triethoxysilylethyl polydimethylsiloxyethyl hexyl dimethicone
(*3) Product of Shin-Etsu Chemical Co., Ltd.: KF-9909-treated color inorganic pigment, W: white - R: red - Y: yellow - B: black
(*4) Product of Shin-Etsu Chemical Co., Ltd.: Caprylyl methicone

(Production method)

A: Components 1 to 7 were mixed uniformly using a Henschel mixer.
B: Components 8 to 11 were mixed uniformly.
C: The mixture obtained in the step B was added to the mixture obtained in the step A, and the resulting mixture was stirred until uniform using a Henschel mixer.
D: The mixture obtained in the step C was caused to pass through a sieve, and then the resulting mixture was pressed into a metal pan using a mold to thereby obtain a powder foundation.

The powder foundation obtained in the manner described above was very smooth during application, spread easily with no stickiness, exhibited good adhesion, settled well, provided a finish with reduced shine, did not smear, had enhanced long-lasting makeup efficacy, and was found to be a very good foundation.

[Table 24]

| Formulation example 65: Loose powder | |
|---|---|
| Component | Mass (%) |
| 1. Elastomer composite particles obtained in preparation/production example 2 | 20.0 |
| 2. KSP-100 (*1) | 10.0 |
| 3. KSP-300 (*2) | 5.0 |
| 4. Lauroyl lysine | 5.0 |
| 5. Boron nitride | 3.0 |
| 6. Pearl pigment | 3.0 |
| 7. KF-9901 (*3)-treated synthetic fluorphlogopite | 15.0 |
| 8. KF-9901 (*3)-treated talc | Remainder |
| 9. Disodium N-stearoyl L-glutamate-treated inorganic color pigment | 4.0 |
| 10. Isononyl isononanoate | 2.0 |
| 11. Ethylhexylglycerin | 0.5 |
| Total | 100.0 |

(*1) Product of Shin-Etsu Chemical Co., Ltd.: (Vinyl dimethicone/methicone silsesquioxane) crosspolymer
(*2) Product of Shin-Etsu Chemical Co., Ltd.: (Diphenyl dimethicone/vinyl diphenyl dimethicone/silsesquioxane) crosspolymer
(*3) Product of Shin-Etsu Chemical Co., Ltd.: Hydrogen dimethicone

(Production method)

A: Components 1 to 9 were mixed uniformly using a Henschel mixer.
B: Components 10 to 11 were mixed uniformly until dissolution.
C: The mixture obtained in the step B was added to the mixture obtained in the step A, and the resulting mixture was stirred until uniform using a Henschel mixer.
D: The mixture obtained in the step C was caused to pass through a sieve and then charged into a container to thereby obtain a loose powder.

The loose powder obtained in the manner described above was very smooth during application, provided a finish with reduced shine, had enhanced long-lasting makeup efficacy, and was found to be a very good loose powder.

[Table 25]

| Formulation example 66: Oil-based blush | |
|---|---|
| Component | Mass (%) |
| 1. KSG-16 (*1) | 28.0 |
| 2. Cyclopentasiloxane | Remainder |
| 3. Neopentyl glycol dicaprate | 9.0 |
| 4. Stearoyl inulin | 10.0 |
| 5. Antioxidant | Optimum amount |
| 6. Spherical elastomer particles obtained in preparation example 6 | 12.0 |

(continued)

| Formulation example 66: Oil-based blush | |
|---|---|
| Component | Mass (%) |
| 7. Red No. 202 | 0.2 |
| 8. KTP-09W, R, Y, B (*2) | 5.0 |
| 9. KF-9909-treated pearl pigment (*3) | 5.0 |
| 10. KF-9909-treated mica (*3) | 11.5 |
| Total | 100.0 |

(*1) Product of Shin-Etsu Chemical Co., Ltd.: Mixture of dimethicone 70 to 80% + (dimethicone/vinyl dimethicone) crosspolymer 20 to 30%
(*2) Product of Shin-Etsu Chemical Co., Ltd.: KF-9909-treated color inorganic pigment, W: white - R: red - Y: yellow - B: black
(*3) Product of Shin-Etsu Chemical Co., Ltd.: Triethoxysilylethyl polydimethylsiloxyethyl hexyl dimethicone

(Production method)

A: Components 1 to 5 were mixed and heated to 80°C, and the resulting mixture was stirred until uniform.
B: Components 6 to 10 were mixed uniformly using a Henschel mixer.
C: The mixture obtained in the step B was added to the mixture obtained in the step A, and the resulting mixture was stirred until uniform at 80°C.
D: The mixture obtained in the step C was defoamed under heating, then charged into a container, and cooled to room temperature to thereby obtain an oil-based blush.

The blush obtained in the manner described above was in the form of sponge, was easy to pick up, spread easily, and had a non-oily and non-powdery texture upon application. The water resistance, water repellency, and perspiration resistance of the blush were good, and the blush lasted long, was resistant to makeup smearing, and was found to be highly stable, showing no deterioration under varying temperatures and over time.

[Table 26]

| Formulation example 67: Powder blush | |
|---|---|
| Component | Mass (%) |
| 1. Elastomer composite particles obtained in preparation/production example 6 | 7.0 |
| 2. KSP-441 (*1) | 2.0 |
| 3. Zinc laurate | 1.5 |
| 4. Isostearyl sebacate-treated mica | 10.0 |
| 5. Isostearyl sebacate-treated talc | Remainder |
| 6. Dimethicone-treated pearl pigment | 3.0 |
| 7. Red No. 202 | 0.3 |
| 8. Yellow No. 4 | 0.5 |
| 9. Isostearyl sebacate-treated inorganic color pigment | 3.5 |
| 10. Triethylhexanoin | 2.0 |
| 11. Diisostearyl malate | 1.5 |
| 12. KF-6038 (*2) | 1.0 |
| 13. Dimethicone (20 cs) | 3.0 |
| Total | 100.0 |

(*1) Product of Shin-Etsu Chemical Co., Ltd.: Polysilicone-22
(*2) Product of Shin-Etsu Chemical Co., Ltd.: Lauryl PEG-9 polydimethylsiloxyethyl dimethicone

(Production method)

A: Components 1 to 9 were mixed uniformly using a Henschel mixer.
B: Components 10 to 13 were mixed uniformly.
C: The mixture obtained in the step B was added to the mixture obtained in the step A, and the resulting mixture was stirred until uniform using a Henschel mixer.
D: The mixture obtained in the step C was caused to pass through a sieve and then pressed into a metal pan using a mold to thereby obtain a powder blush.

The powder blush obtained in the manner described above was smooth during application, spread easily with no stickiness, exhibited good adhesion, settled well, provided a finish with reduced shine, did not smear, had enhanced long-lasting makeup efficacy, and was found to be a very good powder blush.

[Table 27]

| Formulation example 68: Water-in-oil type cream eyeshadow | |
|---|---|
| Component | Mass (%) |
| 1. Cyclopentasiloxane | 15.0 |
| 2. Dimethicone (6 cs) | 10.0 |
| 3. KF-6028 (*1) | 2.0 |
| 4. Pentaerythrityl tetraethylhexanoate | 5.0 |
| 5. Spherical elastomer particles obtained in preparation example 4 | 16.0 |
| 6. Al dimyristate-treated inorganic color pigment | 4.5 |
| 7. Na chloride | 2.0 |
| 8. PG | 8.0 |
| 9. Preservative | Optimum amount |
| 10. Fragrance | Optimum amount |
| 11. Water | Remainder |
| Total | 100.0 |
| (*1) Product of Shin-Etsu Chemical Co., Ltd.: PEG-9 polydimethylsiloxyethyl dimethicone | |

(Production method)

A: Components 1 to 4 were mixed uniformly.
B: Components 5 to 6 were mixed uniformly using a Henschel mixer.
C: Components 7 to 9 and component 11 were mixed uniformly until dissolution.
D: The mixture obtained in the step C was added to the mixture obtained in the step A, and the resulting mixture was stirred until uniform.
E: Component 10 was added to the mixture obtained in the step D, and the resulting mixture was stirred until uniform.
F: The mixture obtained in the step E was defoamed and then charged into a container to thereby obtain a water-in-oil type cream eyeshadow.

The cream eyeshadow obtained in the manner described above spread easily and had a non-oily and non-powdery texture upon application. The water resistance, water repellency, and perspiration resistance of the cream eyeshadow were good, and the cream eyeshadow lasted long, was resistant to makeup smearing, and was found to be highly stable, showing no deterioration under varying temperatures and over time.

[Table 28]

| Formulation example 69: Oil-based eyecolor | |
|---|---|
| Component | Mass (%) |
| 1. Isotridecyl isononanoate | Remainder |

(continued)

| Formulation example 69: Oil-based eyecolor | |
|---|---|
| Component | Mass (%) |
| 2. Squalane | 20.0 |
| 3. Dextrin palmitate | 10.0 |
| 4. KSG-16 (*1) | 12.0 |
| 5. Spherical elastomer particles obtained in preparation example 2 | 6.0 |
| 6. Ba sulfate | 5.0 |
| 7. (PET/Al) laminate | 4.5 |
| 8. Dimethicone-treated pearl pigment | 13.5 |
| 9. Antioxidant | Optimum amount |
| 10. Titanium oxide-coated borosilicate (Ca/Al) | 1.5 |
| 11. Iron oxide-coated borosilicate (Ca/Al) | 7.5 |
| Total | 100.0 |
| (*1) Product of Shin-Etsu Chemical Co., Ltd.: Mixture of dimethicone 70 to 80% + (dimethicone/vinyl dimethicone) crosspolymer 20 to 30% | |

(Production method)

A: Components 1 to 5 were mixed and heated to 90°C, and the resulting mixture was stirred until uniform.
B: Components 6 to 11 were added to the mixture obtained in the step A, and the resulting mixture was stirred until uniform at 90°C.
C: The mixture obtained in the step B was defoamed under heating, then charged into a container, and cooled to room temperature to thereby obtain an oil-based eyecolor.

The eyecolor obtained in the manner described above was in the form of jelly, was easy to pick up, spread easily, and had a non-oily and non-powdery texture upon application. The water resistance, water repellency, and perspiration resistance of the eyecolor were good, and the eyecolor lasted long, was resistant to makeup smearing, and was found to be highly stable, showing no deterioration under varying temperatures and over time.

[Table 29]

| Formulation example 70: Powder eyeshadow | |
|---|---|
| Component | Mass (%) |
| 1. Elastomer composite particles obtained in preparation/production example 8 | 8.0 |
| 2. Zinc myristate | 2.0 |
| 3. Boron nitride | 3.0 |
| 4. Ba sulfate | 5.0 |
| 5. KF-9901 (*1)-treated synthetic fluorphlogopite | 15.0 |
| 6. KF-9901 (*1)-treated mica | Remainder |
| 7. KF-9901 (*1)-treated pearl pigment | 10.0 |
| 8. Red No. 201 | 0.2 |
| 9. Yellow No. 4 | 0.3 |
| 10. Lauroyl lysine-treated inorganic color pigment | 6.0 |
| 11. Isotridecyl isononanoate | 2.0 |
| 12. KP-561P (*2) | 1.0 |
| 13. KF-56A (*3) | 4.0 |
| Total | 100.0 |
| (*1) Product of Shin-Etsu Chemical Co., Ltd.: Hydrogen dimethicone<br>(*2) Product of Shin-Etsu Chemical Co., Ltd.: (Acrylates/stearyl acrylate/dimethicone methacrylate) copolymer<br>(*3) Product of Shin-Etsu Chemical Co., Ltd.: Diphenylsiloxy phenyl trimethicone | |

(Production method)

A: Components 1 to 10 were mixed uniformly using a Henschel mixer.
B: Components 11 to 13 were mixed uniformly.
C: The mixture obtained in the step B was added to the mixture obtained in the step A, and the resulting mixture was stirred until uniform using a Henschel mixer.
D: The mixture obtained in the step C was caused to pass through a sieve and pressed into a metal pan using a mold to thereby obtain a powder eyeshadow.

The powder eyeshadow obtained in the manner described above was very smooth during application, spread easily with no stickiness, exhibited good adhesion, settled well, provided a finish with reduced shine, did not smear, had enhanced long-lasting makeup efficacy, and was found to be a very good powder eyeshadow.

[Table 30]

| Formulation example 71: Stick-type lipstick | |
|---|---|
| Component | Mass (%) |
| 1. Candelilla wax | 8.0 |
| 2. Polyethylene | 8.0 |
| 3. KP-561P (*1) | 12.0 |
| 4. KF-54HV (*2) | 3.0 |
| 5. Isotridecyl isononanoate | 15.5 |
| 6. Glyceryl isostearate | 16.0 |
| 7. Polyglyceryl-2 triisostearate | Remainder |
| 8. AES-3083 (*3)-treated organic color pigment | 2.0 |
| 9. AES-3083 (*3)-treated inorganic color pigment | 4.0 |
| 10. Spherical elastomer particles obtained in preparation example 6 | 6.0 |
| 11. Fragrance | 0.1 |
| Total | 100.0 |

(*1) Product of Shin-Etsu Chemical Co., Ltd.: (Acrylates/stearyl acrylate/dimethicone methacrylate) copolymer
(*2) Product of Shin-Etsu Chemical Co., Ltd.: Diphenyl dimethicone
(*3) Product of Shin-Etsu Chemical Co., Ltd.: Triethoxycaprylylsilane

(Production method)

A: Components 1 to 6 and part of component 7 were mixed and heated to 90°C, and the resulting mixture was stirred until uniform.
B: Components 8 to 9 and the rest of the component 7 were mixed and subjected to roll treatment.
C: The mixture obtained in the step B was added to the mixture obtained in the step A, and the resulting mixture was stirred until uniform at 90°C.
D: Components 10 to 11 were added to the mixture obtained in the step C, and the resulting mixture was stirred until uniform at 90°C.
E: The mixture obtained in the step D was defoamed under heating, then charged into a container, and cooled to room temperature to thereby obtain a stick-type lipstick.

The stick-type lipstick obtained in the manner described above easily spread, had a non-oily and non-powdery texture, was excellent in water resistance and water repellency, lasted long, and was highly stable.

[Table 31]

| Formulation example 72: Water-in-oil type cream lipstick | |
|---|---|
| Component | Mass (%) |
| 1. KSG-43 (*1) | 8.0 |
| 2. KF-6105 (*2) | 2.0 |
| 3. Dextrin (palmitate/ethylhexanoate) | 9.0 |
| 4. X-21-5249 (*3) | 5.0 |
| 5. Elastomer composite particles obtained in preparation/production example 3 | 8.0 |
| 6. Cyclopentasiloxane | Remainder |
| 7. Triethylhexanoin | 3.0 |
| 8. KF-6115 (*4) | 0.3 |
| 9. Red No. 202 | 0.3 |
| 10. Yellow No. 4 | 0.5 |
| 11. BG | 5.0 |
| 12. Water | 10.0 |
| Total | 100.0 |

(*1) Product of Shin-Etsu Chemical Co., Ltd.: Mixture of triethylhexanoin 65 to 75% + (vinyl dimethicone/lauryl dimethicone) crosspolymer 25 to 35%
(*2) Product of Shin-Etsu Chemical Co., Ltd.: Lauryl polyglyceryl-3 polydimethylsiloxyethyl dimethicone
(*3) Product of Shin-Etsu Chemical Co., Ltd.: Dissolution product of cyclopentasiloxane 50% + trimethylsiloxysilicate 50%
(*4) Product of Shin-Etsu Chemical Co., Ltd.: Lauryl polyglyceryl-3 polydimethylsiloxyethyl dimethicone

(Production method)

A: Components 1 to 7 were mixed and heated to 90°C, and the resulting mixture was stirred until uniform.
B: Components 8 to 10 were mixed and subjected to roll treatment.
C: Components 11 to 12 were mixed uniformly until dissolution.
D: The mixture obtained in the step B was added to the mixture obtained in the step A, and the resulting mixture was stirred until uniform at 90°C.
E: The mixture obtained in the step C was added to the mixture obtained in the step D, and the resulting mixture was stirred until uniform at 90°C.
F: The mixture obtained in the step E was cooled to room temperature, defoamed, and then charged into a container to thereby obtain a water-in-oil type cream lipstick.

The obtained cream lipstick was excellent in spreadability and smoothness during application, was not sticky, exhibited good adhesion, settled well, provided a beautiful finish with less unnatural shine, resisted smearing, color migration, and fading, had enhanced long-lasting makeup efficacy, was helpful in preventing dryness and roughness of the lips, and was found to have good characteristics.

[Table 32]

| Formulation example 73: Lip gloss | |
|---|---|
| Component | Mass (%) |
| 1. Glyceryl tri(behenate/isostearate/eicosandioate) | 8.0 |
| 2. Hydrogenated isobutene | 30.0 |
| 3. KSG-44 (*1) | 6.0 |
| 4. KP-545L (*2) | 3.0 |
| 5. Isotridecyl isononanoate | Remainder |
| 6. Elastomer composite particles obtained in preparation/production example 7 | 5.0 |
| 7. Ethylhexyl palmitate | 3.0 |
| 8. KF-6015 (*3) | 0.5 |

(continued)

| Formulation example 73: Lip gloss | |
|---|---|
| Component | Mass (%) |
| 9. Red No. 202 | 0.1 |
| 10. Yellow No. 4 | 0.2 |
| 11. KF-99P (*4)-treated inorganic color pigment | 1.0 |
| 12. Pearl pigment | Optimum amount |
| Total | 100.0 |

(*1) Product of Shin-Etsu Chemical Co., Ltd.: Mixture of squalane 65 to 75% + (vinyl dimethicone/lauryl dimethicone) crosspolymer 25 to 35%
(*2) Product of Shin-Etsu Chemical Co., Ltd.: Dissolution product of dimethicone (2 cs) 60% + (acrylates/dimethicone) copolymer 40%
(*3) Product of Shin-Etsu Chemical Co., Ltd.: PEG-3 dimethicone
(*4) Product of Shin-Etsu Chemical Co., Ltd.: Methicone

(Production method)

A: Components 1 to 6 were mixed uniformly.
B: Components 7 to 11 were mixed uniformly and subjected to roll treatment.
C: The mixture obtained in the step B was added to the mixture obtained in the step A, and the resulting mixture was stirred until uniform.
D: Component 12 was added to the mixture obtained in the step C, and the resulting mixture was stirred until uniform.
E: The mixture obtained in the step E was defoamed and then charged into a container to thereby obtain a lip gloss.

The lip gloss obtained in the manner described above was excellent in spreadability and smoothness during application, was not sticky, exhibited good adhesion, settled well, provided a beautiful finish with less unnatural shine, resisted smearing, color migration, and fading, had enhanced long-lasting makeup efficacy, was helpful in preventing dryness and roughness of the lips, and was found to have good characteristics.

[Table 33]

| Formulation example 74: Oil-based mascara | |
|---|---|
| Component | Mass (%) |
| 1. Paraffine | 20.0 |
| 2. Microcrystalline wax | 8.0 |
| 3. Polyethylene | 3.0 |
| 4. Stearoyl inulin | 1.0 |
| 5. Disteardimonium hectorite | 2.0 |
| 6. KF-6028 (*1) | 1.0 |
| 7. NBN-30-ID (*2) | 3.0 |
| 8. Hydrogenated isobutene | Remainder |
| 9. Spherical elastomer particles obtained in preparation example 4 | 2.0 |
| 10. Neopentyl glycol dicaprate | 5.0 |
| 11. KF-6115 (*3) | 1.0 |
| 12. KTP-09W, B (*4) | 6.5 |
| Total | 100.0 |

(*1) Product of Shin-Etsu Chemical Co., Ltd.: PEG-9 polydimethylsiloxyethyl dimethicone
(*2) Product of Shin-Etsu Chemical Co., Ltd.: Dissolution product of isododecane 70% + norbornene/tris(trimethylsiloxy)silylnorbornene) copolymer 30%
(*3) Product of Shin-Etsu Chemical Co., Ltd.: Lauryl polyglyceryl-3 polydimethylsiloxyethyl dimethicone
(*4) Product of Shin-Etsu Chemical Co., Ltd.: KF-9909-treated color inorganic pigment, W: white - B: black

(Production method)

A: Components 1 to 8 were heated until dissolution.
B: Components 10 to 12 were mixed uniformly and subjected to roll treatment.
C: The mixture obtained in the step B and component 9 were added to the heated mixture obtained in the step A, and the resulting mixture was stirred until uniform.
D: The mixture obtained in the step C was defoamed, cooled to room temperature, and then charged into a container to thereby obtain an oil-based mascara.

The oil-based mascara obtained in the manner described above was smooth during application, spread easily with no stickiness, exhibited good adhesion, settled well, provided a finish with reduced shine, did not smear, had enhanced long-lasting makeup efficacy, and was found to be very good mascara.

[Table 34]

Formulation example 75: Water-in-oil type eyeliner

| Component | Mass (%) |
|---|---|
| 1. Cyclopentasiloxane | Remainder |
| 2. KF-6017P (*1) | 3.0 |
| 3. KF-7312J (*2) | 15.0 |
| 4. Disteardimonium hectorite | 2.0 |
| 5. KF-9901 (*3)-treated inorganic color pigment | 8.0 |
| 6. Spherical elastomer particles obtained in preparation example 2 | 12.0 |
| 7. BG | 5.0 |
| 8. Preservative | Optimum amount |
| 9. Water | 15.0 |
| Total | 100.0 |

(*1) Product of Shin-Etsu Chemical Co., Ltd.: PEG-10 dimethicone
(*2) Product of Shin-Etsu Chemical Co., Ltd.: Dissolution product of cyclopentasiloxane 50% + trimethylsiloxysilicate 50%
(*3) Product of Shin-Etsu Chemical Co., Ltd.: Hydrogen dimethicone

(Production method)

A: Components 1 to 4 were mixed, and components 5 and 6 were added. Then the resulting mixture was stirred until uniform.
B: Components 7 to 9 were mixed uniformly until dissolution.
C: The mixture obtained in the step B was added to the mixture obtained in the step A, and the resulting mixture was stirred until uniform.
D: The mixture obtained in the step C was defoamed and then charged into a container to thereby obtain a water-in-oil type eyeliner.

The eyeliner obtained in the manner described above spread easily, was easy to apply, provided a refresh feel and a non-greasy finish, provided a non-sticky texture upon application, and was highly useful and stable, showing no deterioration under varying temperatures and over time. The eyeliner was excellent in water resistance and perspiration resistance, and its staying power was found to be very good.

[Table 35]

Formulation example 76: Water-in-oil type eyeliner

| Component | Mass (%) |
|---|---|
| 1. Cyclopentasiloxane | Remainder |

(continued)

| Formulation example 76: Water-in-oil type eyeliner | |
|---|---|
| Component | Mass (%) |
| 2. Dimethicone (6 cs) | 5.0 |
| 3. Jojoba oil | 2.0 |
| 4. KF-6017 (*1) | 1.0 |
| 5. KF-6038 (*2) | 1.0 |
| 6. KP-545 (*3) | 15.0 |
| 7. Elastomer composite particles obtained in preparation/production example 5 | 10.0 |
| 8. KTP-09B (*4) | 18.0 |
| 9. Ethanol | 5.0 |
| 10. Preservative | Optimum amount |
| 11. Water | 35.0 |
| Total | 100.0 |

(*1) Product of Shin-Etsu Chemical Co., Ltd.: PEG-10 dimethicone
(*2) Product of Shin-Etsu Chemical Co., Ltd.: Lauryl PEG-9 polydimethylsiloxyethyl dimethicone
(*3) Product of Shin-Etsu Chemical Co., Ltd.: Dissolution product of cyclopentasiloxane 70% + (acrylates/dimethicone) copolymer 30%
(*4) Product of Shin-Etsu Chemical Co., Ltd.: KF-9909-treated color inorganic pigment, B: black

(Production method)

A: Components 1 to 6 were mixed, and components 7 and 8 were added. Then the resulting mixture was stirred until uniform.
B: Components 9 to 11 were mixed uniformly until dissolution.
C: The mixture obtained in the step B was added to the mixture obtained in the step A, and the resulting mixture was stirred until uniform.
D: The mixture obtained in the step C was defoamed and then charged into a container to thereby obtain a water-in-oil type eyeliner.

The eyeliner obtained in the manner described above was easy to spread, had a non-oily and non-powdery texture, was excellent in water resistance, water repellency, and perspiration resistance, lasted long, and was resistant to makeup smearing. The eyeliner was found to be highly stable, showing no deterioration under varying temperatures and over time.

[Table 36]

| Formulation example 77: Water-in-oil type eyeliner | |
|---|---|
| Component | Mass (%) |
| 1. Cyclopentasiloxane | Remainder |
| 2. Dimethicone (6 cs) | 2.0 |
| 3. AES-3083 (*1)-treated inorganic color pigment | 20.0 |
| 4. Elastomer composite particles obtained in preparation/production example 4 | 4.0 |
| 5. X-21-5595 (*2) | 10.0 |
| 6. Jojoba oil | 2.0 |
| 7. Bentonite | 3.0 |
| 8. KF-6017 (*3) | 2.0 |
| 9. Antioxidant | Optimum amount |
| 10. Ethanol | 3.0 |
| 11. Pentylene glycol | 5.0 |
| 12. Preservative | Optimum amount |
| 13. Water | 20.0 |

(continued)

| Formulation example 77: Water-in-oil type eyeliner | |
|---|---|
| Component | Mass (%) |
| Total | 100.0 |
| (*1) Product of Shin-Etsu Chemical Co., Ltd.: Triethoxycaprylylsilane<br>(*2) Product of Shin-Etsu Chemical Co., Ltd.: Dissolution product of isododecane 40% + trimethylsiloxysilicate 60%<br>(*3) Product of Shin-Etsu Chemical Co., Ltd.: PEG-10 dimethicone | |

(Production method)

A: Components 1, 2, 5 to 9 were mixed, and components 3 and 4 were added. Then the resulting mixture was stirred until uniform.
B: Components 10 to 13 were mixed.
C: The mixture obtained in the step B was added to the mixture obtained in the step A, and the resulting mixture was stirred until uniform.
D: The mixture obtained in the step C was defoamed and then charged into a container to thereby obtain a water-in-oil type eyeliner.

The eyeliner obtained in the manner described above spread easily, was easy to apply, provided a refresh feel and a non-greasy finish, provided a non-sticky texture upon application, was excellent in water resistance and perspiration resistance, and was found to have significant long-lasting makeup efficacy. Moreover, the eyeliner was highly stable, showing no deterioration under varying temperatures and over time.

[Table 37]

| Formulation example 78: Water-in-oil type antiperspirant cream | |
|---|---|
| Component | Mass (%) |
| 1. KSG-210 (*1) | 7.0 |
| 2. Cyclopentasiloxane | 10.0 |
| 3. Neopentyl glycol diethylhexanoate | 7.0 |
| 4. Elastomer composite particles obtained in preparation/production example 6 | 10.0 |
| 5. KSP-102 (*2) | 5.0 |
| 6. DPG | 5.0 |
| 7. Na citrate | 0.2 |
| 8. (Al/zirconium) trichlorohydrex glycine | 18.0 |
| 9. Fragrance | Optimum amount |
| 10. Water | Remainder |
| Total | 100.0 |
| (*1) Product of Shin-Etsu Chemical Co., Ltd.: Mixture of dimethicone 70 to 80% + (dimethicone/(PEG-10/15)) crosspolymer 20 to 30%<br>(*2) Product of Shin-Etsu Chemical Co., Ltd.: (Vinyl dimethicone/methicone silsesquioxane) crosspolymer | |

(Production method)

A: Components 1 to 5 were mixed uniformly.
B: Components 6 to 10 were mixed uniformly until dissolution.
C: The mixture obtained in the step B was added to the mixture obtained in the step A, and the resulting mixture was stirred until uniform.
D: The mixture obtained in the step C was defoamed and then charged into a container to thereby obtain a water-in-oil type antiperspirant cream.

The antiperspirant cream obtained in the manner described above spread easily, had a non-sticky and non-oily texture, and was very highly usable and stable, showing no deterioration under varying temperatures and over time.

[Table 38]

| Formulation example 79: Roll-on type antiperspirant cosmetic | |
|---|---|
| Component | Mass (%) |
| 1. KSG-210 (*1) | 20.0 |
| 2. Dimethicone (6 cs) | 10.0 |
| 3. KSG-15 (*2) | 15.0 |
| 4. Cyclopentasiloxane | Remainder |
| 5. (Al/zirconium) trichlorohydrex glycine | 20.0 |
| 6. Elastomer composite particles obtained in preparation/production example 1 | 20.0 |
| 7. Fragrance | Optimum amount |
| Total | 100.0 |

(*1) Product of Shin-Etsu Chemical Co., Ltd.: Mixture of dimethicone 70 to 80% + (dimethicone/(PEG-10/15)) Crosspolymer 20 to 30%
(*2) Product of Shin-Etsu Chemical Co., Ltd.: Mixture of cyclopentasiloxane 90 to 96% + (dimethicone/vinyl dimethicone) crosspolymer 4 to 10%

(Production method)

A: Components 1 to 4 were mixed uniformly.
B: Components 5 to 7 were added to the mixture obtained in the step A, and the resulting mixture was stirred until uniform.
C: The mixture obtained in the step B was defoamed and then charged into a container to thereby obtain a roll-on type antiperspirant cosmetic.

The roll-on type antiperspirant cosmetic obtained in the manner described above spread easily, had a non-sticky and non-oily texture, and was very highly usable and stable, showing no deterioration under varying temperatures and over time.

[Table 39]

| Formulation example 80: Oil-based antiperspirant cream | |
|---|---|
| Component | Mass (%) |
| 1. KF-4422 (*1) | 30.0 |
| 2. KSG-15 (*2) | 21.5 |
| 3. Neopentyl glycol dioctanoate | Remainder |
| 4. Polyethylene | 3.0 |
| 5. Ceresin | 6.0 |
| 6. Antioxidant | Optimum amount |
| 7. (Al/zirconium) trichlorohydrex glycine | 19.0 |
| 8. Spherical elastomer particles obtained in preparation example 6 | 10.0 |
| 9. Silica dimethyl silylate | 0.5 |
| 10. Fragrance | Optimum amount |
| Total | 100.0 |

(*1) Product of Shin-Etsu Chemical Co., Ltd.: Ethyl trimethicone
(*2) Product of Shin-Etsu Chemical Co., Ltd.: Mixture of (cyclopentasiloxane 90 to 96% + (dimethicone/vinyl dimethicone) crosspolymer 4 to 10%)

(Production method)

A: Components 1 to 6 were heated and mixed uniformly.
B: Components 7 to 9 were added to the mixture obtained in the step A, and the resulting mixture was stirred until uniform.
C: Component 10 was added to the mixture obtained in the step B, and the resulting mixture was stirred until uniform.
D: The mixture obtained in the step C was defoamed and then charged into a container to thereby obtain an oil-based antiperspirant cream.

The antiperspirant cream obtained in the manner described above could be applied vary smoothly, had good spreadability, provided a non-overly dry feel and a non-sticky feel, and maintained the deodorizing effect for a long time.

[Table 40]

| Formulation example 81: Nail enamel | |
|---|---|
| Component | Mass (%) |
| 1. KP-549 (*1) | 45.0 |
| 2. TMF-1.5 (*2) | 5.0 |
| 3. Nitrocellulose | 3.0 |
| 4. Camphorquinone | 0.5 |
| 5. Acetyl tributyl citrate | 1.0 |
| 6. Dimethyldistearyl ammonium hectorite | 0.5 |
| 7. Butyl acetate | 30.0 |
| 8. Ethyl acetate | 10.0 |
| 9. Isopropyl alcohol | 5.0 |
| 10. Elastomer composite particles obtained in preparation/production example 9 | 1.0 |
| Total | 100.0 |

(*1) Product of Shin-Etsu Chemical Co., Ltd.: Dissolution product of methyl trimethicone 60% + (acrylates/dimethicone) copolymer 40%
(*2) Product of Shin-Etsu Chemical Co., Ltd.: Methyl trimethicone

(Production method)

A: Components 7 to 9 were mixed, and components 4 to 6 were added to the mixture. Then the resulting mixture was stirred until uniform.
B: Components 1 to 3 were added to the mixture obtained in the step A, and the resulting mixture was stirred until uniform.
C: Component 10 was added to the mixture obtained in the step B, and the resulting mixture was stirred until uniform.
D: The mixture obtained in the step C was defoamed and then charged into a container to thereby obtain a nail enamel.

The nail enamel obtained in the manner described above spread easily, imparted a smooth visual appearance, had water resistance and oil resistance, lasted long, did not cause stress on the nails and yellowing of the nails, formed a cosmetic film resistant to deterioration under varying temperatures and over time, and was found to be very highly stable.

Industrial Applicability

The spherical elastomer particles and elastomer composite particles (polyorganosilsesquioxane- or silica-coated spherical elastomer particles) in the present invention are expected to be very useful particularly for cosmetics because of their unique structural composition.
The degradable polyester structure (particularly poly-ε-caprolactone) and the polyether structure present in the powder/particle skeleton are expected to exhibit/impart high biodegradability.
Therefore, the cosmetic of the present invention containing the spherical elastomer particles and/or the

elastomer composite particles is expected to be used/utilized as an environmental load-reducing material.

## Claims

1. A cosmetic comprising at least one type of particles (i) and (ii) defined as:

   (i) spherical elastomer particles that have a volume average particle diameter of 1.0 to 100 μm and are crosslinked particles of a copolymer having a polyester structure and a polyether structure; and
   (ii) elastomer composite particles containing

   the spherical elastomer particles (i) and
   polyorganosilsesquioxane or silica present on surfaces of the spherical elastomer particles (i).

2. The cosmetic according to claim 1, wherein the copolymer is a polyester-polyether copolymer having at least two radical-polymerizable unsaturated groups per molecule.

3. The cosmetic according to claim 2, wherein the copolymer is a polyester-polyether copolymer represented by general formula (1) or (2) defined as:

[Chemical formula 1]

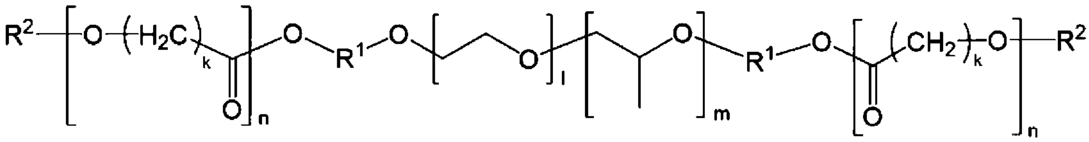
- (1)

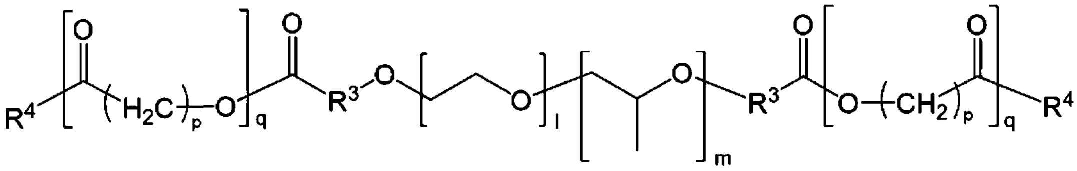
- (2)

wherein, in the general formula (1), each $R^1$ independently represents a divalent hydrocarbon group having 1 to 10 carbon atoms; each $R^2$ independently represents a radical-polymerizable functional group-containing organic group represented by general formula (3a), (3b), or (3c) as defined below; each k is independently a number satisfying $1 \leq k \leq 10$; 1 is a number satisfying $1 \leq 1 \leq 1{,}000$; m is a number satisfying $1 \leq m \leq 1{,}000$; and each n is independently a number satisfying $1 \leq n \leq 100$, and
wherein, in the general formula (2), each $R^3$ independently represents a divalent hydrocarbon group having 1 to 10 carbon atoms; each $R^4$ independently represents a radical-polymerizable functional group-containing organic group represented by general formula (4a) or (4b) as defined below; each p is independently a number satisfying $1 \leq p \leq 10$; 1 is a number satisfying $1 \leq 1 \leq 1{,}000$; m is a number satisfying $1 \leq m \leq 1{,}000$; and each q is independently a number satisfying $1 \leq q \leq 100$,

[Chemical formula 2]

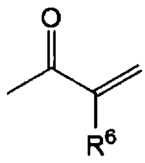

- (3a)

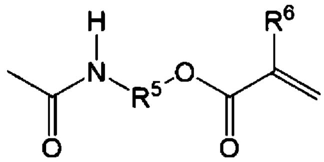

- (3b)

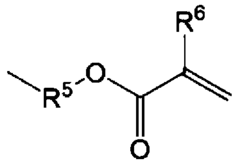

- (3c)

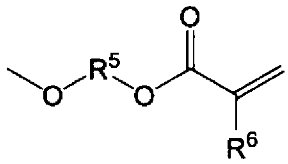

- (4a)

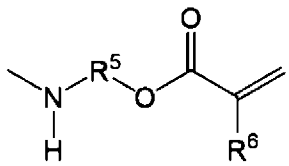

- (4b)

wherein, in the general formulas (3a), (3b), (3c), (4a), and (4b), each $R^5$ independently represents a divalent hydrocarbon group having 1 to 8 carbon atoms; and each $R^6$ independently represents a hydrogen atom or a hydrocarbon group having 1 to 3 carbon atoms.

**4.** The cosmetic according to claim 2, wherein the copolymer is a polyester-polyether copolymer represented by general formula (5) defined as:

[Chemical formula 3]

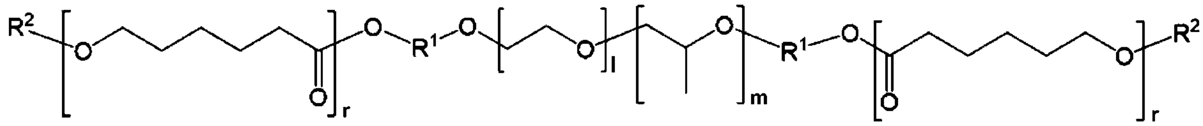

- (5)

wherein, in the general formula (5), each $R^1$ independently represents a divalent hydrocarbon group having 1 to 10 carbon atoms; 1 is a number satisfying $1 \leq 1 \leq 1{,}000$; m is a number satisfying $1 \leq m \leq 1{,}000$; each r is independently a number satisfying $1 \leq r \leq 100$, and each $R^2$ independently represents a radical-polymerizable functional group-containing organic group represented by general formula (3a), (3b), or (3c) defined as

[Chemical formula 4]

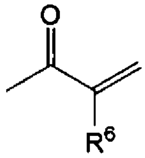

- (3a)

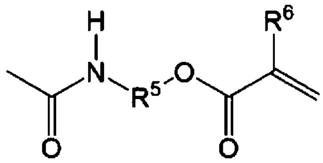

- (3b)

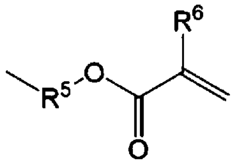

- (3c)

wherein, in the general formulas (3a), (3b), and (3c), each $R^5$ independently represents a divalent hydrocarbon group having 1 to 8 carbon atoms, and each $R^6$ independently represents a hydrogen atom or a hydrocarbon group having 1 to 3 carbon atoms.

**5.** The cosmetic according to claim 1, wherein the spherical elastomer particles have a rubber hardness of 10 to 80 as measured by a type A durometer specified in JIS K 6253.

**6.** The cosmetic according to claim 1, wherein the cosmetic is a skincare cosmetic.

**7.** The cosmetic according to claim 1, wherein the cosmetic is a makeup cosmetic.

**8.** The cosmetic according to claim 1, wherein the cosmetic is a suncare cosmetic.

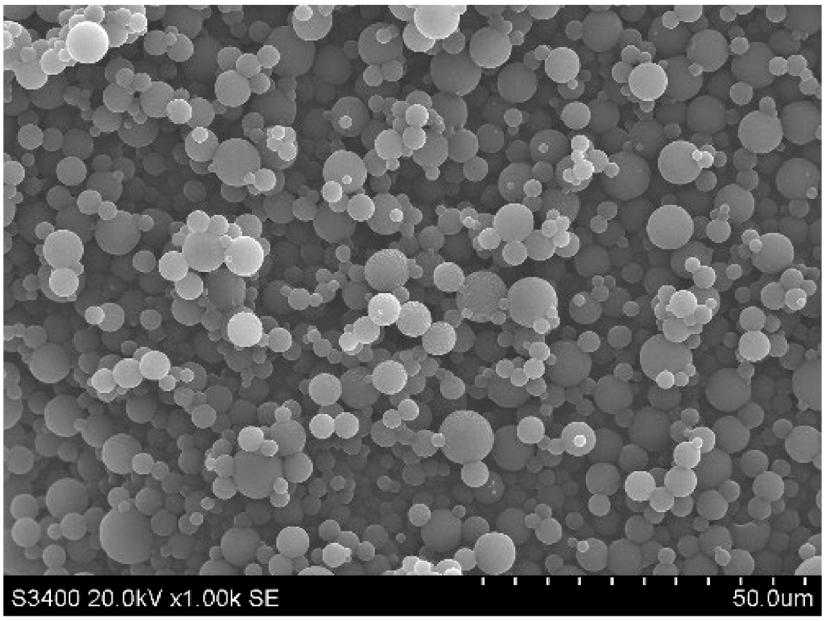


FIG.1

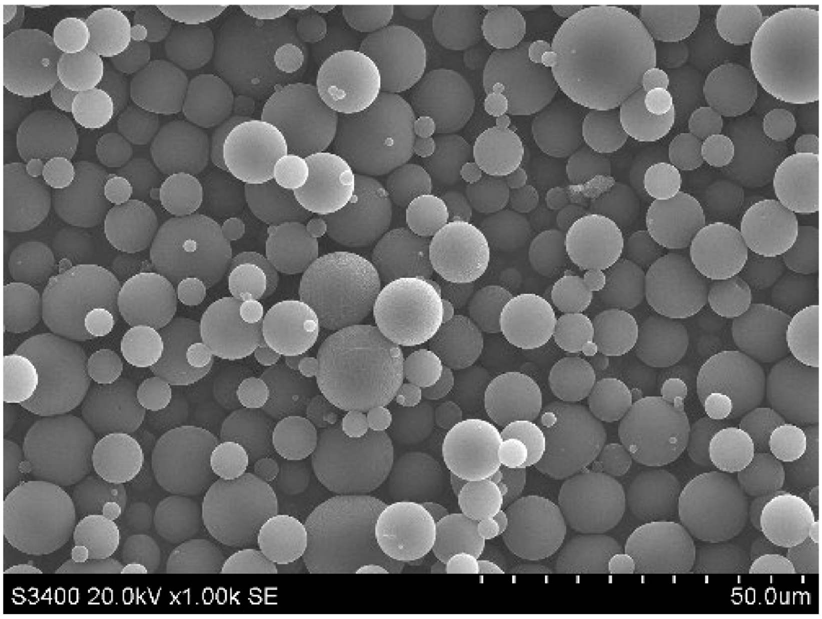


FIG.2

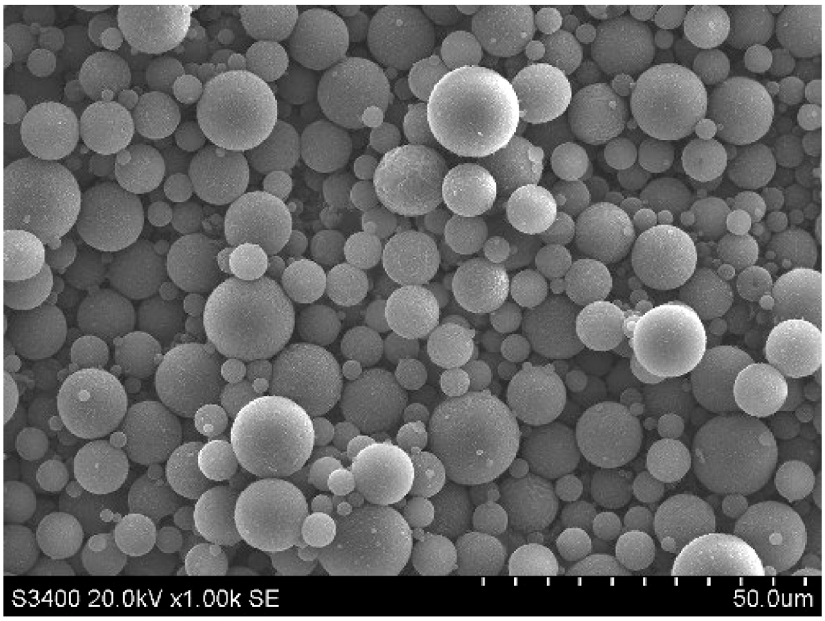


FIG.3

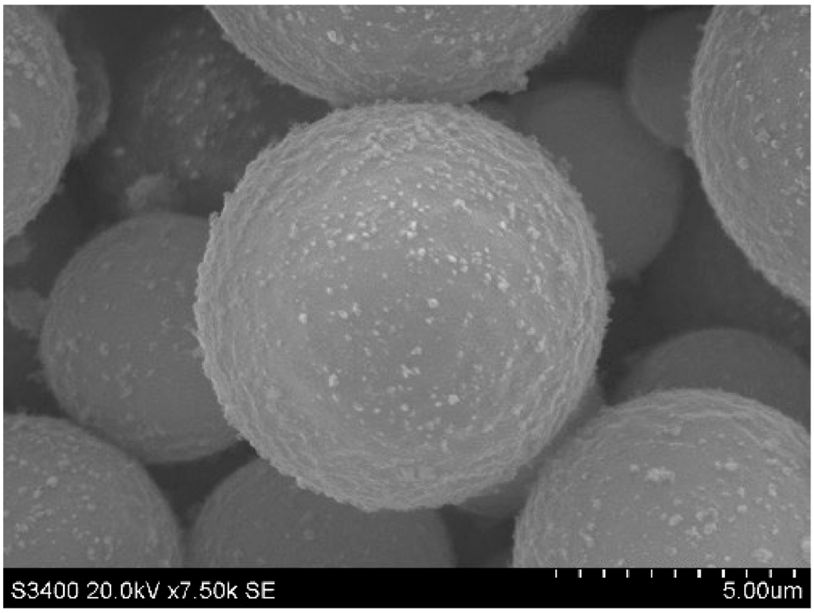


FIG.4

## INTERNATIONAL SEARCH REPORT

International application No.
**PCT/JP2024/028740**

**A. CLASSIFICATION OF SUBJECT MATTER**

***A61K 8/25***(2006.01)i; ***A61K 8/89***(2006.01)i; ***A61Q 1/00***(2006.01)i; ***A61Q 1/02***(2006.01)i; ***A61Q 1/04***(2006.01)i; ***A61Q 1/06***(2006.01)i; ***A61Q 1/10***(2006.01)i; ***A61Q 3/02***(2006.01)i; ***A61Q 15/00***(2006.01)i; ***A61Q 17/04***(2006.01)i; ***A61Q 19/00***(2006.01)i; ***C08F 299/04***(2006.01)i; ***C08G 63/91***(2006.01)i; ***C08G 77/442***(2006.01)i; ***C08L 101/16***(2006.01)i
FI: A61K8/25; A61Q1/00; A61Q17/04; A61K8/89; A61Q1/02; A61Q1/10; A61Q1/06; A61Q1/04; A61Q15/00; A61Q3/02; C08G63/91; C08F299/04; C08G77/442; C08L101/16; A61Q19/00 ZBP

According to International Patent Classification (IPC) or to both national classification and IPC

**B. FIELDS SEARCHED**

Minimum documentation searched (classification system followed by classification symbols)

A61K8/25; A61K8/89; A61Q1/00; A61Q1/02; A61Q1/04; A61Q1/06; A61Q1/10; A61Q3/02; A61Q15/00; A61Q17/04; A61Q19/00; C08F299/04; C08G63/91; C08G77/442; C08L101/16

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2012-92327 A (SEKISUI PLASTICS CO., LTD.) 17 May 2012 (2012-05-17) claims 1, 3, 9, paragraphs [0046], [0062], [0074]-[0078], [0086]-[0097], examples 1-5, 8-11 | 1-2, 5-8 |
| Y | paragraph [0062], examples | 6-8 |
| A | entire text | 3-4 |
| X | JP 2008-239638 A (DAICEL CHEM. IND. LTD.) 09 October 2008 (2008-10-09) claim 1, paragraphs [0019], [0033], [0070]-[0083], tables 1-3, examples 4-7, 12-13 | 1, 5 |
| Y | claim 1, paragraphs [0019], [0033], [0070]-[0083], tables 1-3, examples 4-7, 12-13 | 6-8 |
| A | entire text | 2-4 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 October 2024** | **05 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/028740

C. DOCUMENTS CONSIDERED TO BE RELEVANT

| Category* | Citation of document, with indication, where appropriate, of the relevant passages | Relevant to claim No. |
|---|---|---|
| X | JP 2019-178117 A (KOSE CORPORATION) 17 October 2019 (2019-10-17) paragraphs [0078]-[0098], tables 1-3, examples 1-10, 22-29 | 1, 5-7 |
| Y | paragraphs [0078]-[0098], tables 1-3, examples 1-10, 22-29 | 8 |
| A | entire text, all drawings | 2-4 |
| X | JP 2019-178124 A (KOSE CORPORATION) 17 October 2019 (2019-10-17) paragraphs [0040], [0042]-[0052], tables 1-2, examples 1-6, 9-12 | 1, 5-7 |
| Y | paragraphs [0040], [0042]-[0052], tables 1-2, examples 1-6, 9-12 | 8 |
| A | entire text | 2-4 |
| P, X | WO 2023/238840 A1 (SHIN-ETSU CHEMICAL CO., LTD.) 14 December 2023 (2023-12-14) claims, paragraphs [0012]-[0015], [0018], examples | 1-8 |

Form PCT/ISA/210 (second sheet) (July 2022)

**INTERNATIONAL SEARCH REPORT**
**Information on patent family members**

International application No.
**PCT/JP2024/028740**

| Patent document cited in search report | Publication date (day/month/year) | Patent family member(s) | Publication date (day/month/year) |
|---|---|---|---|
| JP 2012-92327 A | 17 May 2012 | (Family: none) | |
| JP 2008-239638 A | 09 October 2008 | (Family: none) | |
| JP 2019-178117 A | 17 October 2019 | (Family: none) | |
| JP 2019-178124 A | 17 October 2019 | (Family: none) | |
| WO 2023/238840 A1 | 14 December 2023 | TW 202402877 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H06128122 A
- WO 9203119 A
- JP H08188723 A
- JP H0920609 A
- JP 2002146238 A
- JP 2003040737 A
- JP 3702072 B
- JP 3963635 B
- JP H083451 A
- JP H03294357 A
- JP H02232263 A
- JP H03281536 A
- JP 2011001332 A
- JP S63297313 A
- JP H0812524 A
- JP H0920631 A